# EUROPEAN PATENT SPECIFICATION

(11) **EP 4 026 881 B1**
(45) Date of publication and mention of the grant of the patent: **30.07.2025**
(21) Application number: 20860625.1
(22) Date of filing: 04.09.2020
(51) Int. Cl.: H10K 85/60, H10K 30/85, C09K 11/06, H10K 50/16, H10K 50/17, H10K 85/30

(54) **ORGANIC THIN FILM AND METHOD FOR PRODUCING ORGANIC THIN FILM**
ORGANISCHER DÜNNFILM UND VERFAHREN ZUR HERSTELLUNG VON ORGANISCHEM DÜNNFILM
FILM MINCE ORGANIQUE ET PROCÉDÉ DE FABRICATION DE FILM MINCE ORGANIQUE

(30) Priority: 06.09.2019 JP 2019163279; 17.06.2020 JP 2020104737
(43) Date of publication of application: 13.07.2022
(73) Proprietor: Nippon Hoso Kyokai, Tokyo 150-8001 (JP); Nippon Shokubai Co., Ltd., Osaka-shi, Osaka 541-0043 (JP)
(72) Inventor: FUKAGAWA, Hirohiko, Tokyo 1578510 (JP); SHIMIZU, Takahisa, Tokyo 1578510 (JP); SASAKI, Tsubasa, Tokyo 1578510 (JP); OONO, Taku, Tokyo 1578510 (JP); HASEGAWA, Munehiro, Suita-shi, Osaka 5640034 (JP); MORII, Katsuyuki, Suita-shi, Osaka 5640034 (JP); FUKUDOME, Hiroki, Suita-shi, Osaka 5640034 (JP)
(74) Representative: Cabinet Beau de Loménie
(86) International application number: PCT/JP2020/033542
(87) International publication number: WO 2021/045178

(56) References cited:
- WO-A1-2016/181705
- JP-A- 2013 258 144
- US-A1- 2015 076 456
- SCHNEIDER SEVERIN ET AL: "Efficient n-Doping and Hole Blocking in Single-Walled Carbon Nanotube Transistors with 1,2,4,5-Tetrakis(tetramethylguanidino)ben-zene", ACS NANO, vol. 12, no. 6, 22 May 2018 (2018-05-22), US, pages 5895 - 5902, XP055911633, ISSN: 1936-0851, DOI: 10.1021/acsnano.8b02061
- SCHWAMM RYAN J., VIANELLO ROBERT, MARŠAVELSKI ALEKSANDRA, GARCÍA M. ÁNGELES, CLARAMUNT ROSA M., ALKORTA IBON, SAAME JAAN, LEITO IV: "15N NMR spectroscopy, x-ray and neutron diffraction, quantum-chemical calculations, and UV/vis-spectrophotometric titrations as complementary techniques for the analysis of pyridine-supported bicyclic guanidine superbases", THE JOURNAL OF ORGANIC CHEMISTRY, vol. 81, no. 17, 5 August 2016 (2016-08-05), pages 7612 - 7625, XP055799352, DOI: 10.1021/acs.joc.6b01330
- ALSARRAF, J. ET AL.: "Cyclic guanidines as efficient organocatalysts for the synthesis of polyurethanes", MACROMOLECULES, vol. 45, 21 February 2012 (2012-02-21), pages 2249 - 2256, XP055440014, DOI: 10.1021/ma2026258

## Description

### TECHNICAL FIELD

The present invention relates an organic thin film and a method for producing an organic thin film, an organic electroluminescence (hereinafter, electroluminescence is also referred to as "EL") device, a display device, a lighting system, an organic thin film solar cell, a thin film transistor, a photoelectric transducer, a coating composition, and an organic electroluminescence device material.

### BACKGROUND ART

Organic EL devices are thin, soft, and flexible. Display devices including organic EL devices are capable of providing higher brightness, higher definition display than currently dominant liquid crystal display devices and plasma display devices. Further, display devices including organic EL devices have a wider viewing angle than liquid crystal display devices. Thus, organic EL display devices are expected to be widely used, for example, as displays of TVs and mobiles.

In addition, organic EL devices are also expected to be used as lighting systems.

An organic EL device is a laminate of a cathode, an emitting layer, and an anode. In organic EL devices, the energy difference between the work function of the anode and the highest occupied molecular orbital (HOMO) of the emitting layer is smaller than the energy difference between the work function of the cathode and the lowest unoccupied molecular orbital (LUMO) of the emitting layer. It is therefore more difficult to inject electrons into the emitting layer from the cathode than to inject holes into the emitting layer from the anode. For this reason, in traditional organic EL devices, an electron injection layer is placed between the cathode and the emitting layer to facilitate electron injection from the cathode to the emitting layer. In addition, the electron injection property and the electron transport property are improved by doping dopants in a layer between the cathode and the emitting layer (see, for example, Non-Patent Literature 1 and Non-Patent Literature 2).

An example of the electron injection layer of an organic EL device is an inorganic oxide layer (see, for example, Non-Patent Literature 3). However, the inorganic oxide layer is poor in electron injection property.

The electron injection property of an organic EL device can be improved by forming an additional electron injection layer on an inorganic oxide layer. For example, Non-Patent Literature 4 discloses an organic EL device that includes an electron injection layer made of polyethyleneimine. Further, Non-Patent Literature 5 discloses that amines effectively improve the injection rate of electrons. Non-Patent Literatures 6, 7, and 8 disclose the effects of an amino group on electron injection at an interface between an electrode and an organic layer.

Severin Schneider et al in ACS Nano, 12(6), 2018, 5895-5902 disclose a tetraguanidine compound as an electron injecting compound. US 2019/0081239 A1 discloses a methyl substituted cyclic guanidine compound (MTBD) as electron injecting material.

### CITATION LIST

### - Non-Patent Literature

Non-Patent Literature 1: Karsten Walzer and three others, "Chemical Review", Vol. 107, 2007, pp. 1233-1271
Non-Patent Literature 2: Peng Wei and three others, "Journal of the American Chemical Society", Vol. 132, 2010, p. 8852
Non-Patent Literature 3: Jiangshan Chen and six others, "Journal Of Materials Chemistry", Vol. 22, 2012, pp. 5164-5170
Non-Patent Literature 4: Hyosung Choi and eight others, "Advanced Materials", Vol. 23, 2011, p. 2759
Non-Patent Literature 5: Yinhua Zho and 21 others, "Science", Vol. 336, 2012, p. 327
Non-Patent Literature 6: Young-Hoon Kim and five others, "Advanced Functional Materials", 2014, DOI: 10.1002/adfm.201304163
Non-Patent Literature 7: Stefan Hofle and four others, "Advanced Materials", 2014, DOI: 10.1002/adma.201304666
Non-Patent Literature 8: Stefan Hofle and five others, "Advanced Materials", Vol. 26, 2014, DOI: 10.1002/adma.201400332
Non-Patent Literature 9: Peng Wei and three others, "Journal of the American Chemical Society", Vol. 132, 2010, p. 8852

### SUMMARY OF INVENTION

### - Technical Problem

Organic EL devices including an existing electron injection layer however require a further improved electron injection property and a further improved electron transport property.

The present invention has been made in view of the above-mentioned circumstances, and aims to provide an organic thin film that imparts an excellent electron injection property and an excellent electron transport property when it is used as an electron injection layer of an organic EL device, a coating composition suitable for producing the organic thin film, and an organic EL device material for the organic thin film and the coating composition.

The present invention also aims to provide an organic EL device including the organic thin film of the present invention, a display device and a lighting system each including the organic EL device, and an organic thin film solar cell, a photoelectric transducer, and an organic thin film transistor each including the organic thin film of the present invention.

### - Solution to Problem

The present inventors focused on and examined basic organic materials as materials to be used for an electron injection layer of an organic EL device. As a result of the examination, the inventors found that an organic thin film containing a hexahydropyrimidopyrimidine compound having a predetermined structure, which is an organic material having an acid dissociation constant pKa of 1 or greater, and a material which transports electrons may be used as an electron injection layer of an organic EL device.

That is, the organic material having a pKa of 1 or greater is capable of extracting a proton (H⁺) from other materials. Thus, it is presumable that, in an organic EL device having an electron injection layer which is such an organic thin film, the organic material having a pKa of 1 or greater extracts a proton (H⁺) from the electron transport material, so that a negative charge is generated, leading to enhancement of the electron injection property.

The present invention has been accomplished based on the above findings, and by the embodiments defined in the appended claims.

### - Advantageous Effects of Invention

The organic thin film of the present invention at least contains a first material which is a specific organic material having an acid dissociation constant pKa of 1 or greater and a second material which transports electrons. Thus, when the organic thin film of the present invention is used as, for example, an electron injection layer of an organic EL device, the organic EL device can have an excellent electron injection property and an excellent electron transport property.

Since the organic EL device of the present invention includes the organic thin film of the present invention between the cathode and the emitting layer, the organic EL device can have an excellent electron injection property and an excellent electron transport property owing to the organic thin film.

The organic thin film of the present invention containing a first material which is a specific hexahydropyrimidopyrimidine compound having an acid dissociation constant pKa of 1 or greater and a second material which transports electrons can be formed by either application or evaporation. Thus, an organic EL device including the organic thin film of the present invention can be produced with little restriction on the production process, and such an organic thin film can be easily used as materials of layers constituting the organic EL device. The method for producing an organic thin film of the present invention is a method for producing such an organic thin film of the present invention.

The coating composition of the present invention contains a first material which is a specific organic material having an acid dissociation constant pKa of 1 or greater and a second material which transports electrons. Accordingly, an organic thin film suitable for an electron injection layer of an organic EL device can be obtained by applying the coating composition of the present invention to a surface on which the organic thin film is to be formed.

The organic EL device material of the present invention is useful for the organic thin film and the coating composition of the present invention to be used for producing an organic EL device or the like. The organic EL device material is also useful in that it can be used alone as an electron injection layer or an electron transport layer.

The display device and the lighting system of the present invention each include the organic EL device of the present invention, and are thus driven with a low voltage and have excellent properties.

In addition, the organic thin film solar cell, the photoelectric transducer, and the organic thin film transistor of the present invention each include the organic thin film of the present invention, and thus have excellent properties.

### BRIEF DESCRIPTION OF DRAWINGS

FIG. 1 is an explanatory schematic cross-sectional view illustrating an exemplary organic EL device of the present invention.
FIG. 2 is an explanatory schematic cross-sectional view illustrating an exemplary organic EL device of the present invention.
FIG. 3 is a schematic cross-sectional view illustrating another exemplary laminate structure of the organic EL device of the present invention.
FIG. 4 is a schematic cross-sectional view illustrating another exemplary laminate structure of the organic EL device of the present invention.
FIG. 5-1 is a schematic cross-sectional view illustrating an exemplary organic thin film of the present invention.
FIG. 5-2 is a schematic cross-sectional view illustrating an exemplary organic thin film of the present invention.
FIG. 6-1 is a schematic cross-sectional view illustrating an exemplary laminate structure of the organic thin film of the present invention.
FIG. 6-2 is a schematic cross-sectional view illustrating an exemplary laminate structure of the organic thin film of the present invention.
FIG. 7-1 is a schematic cross-sectional view illustrating an exemplary laminate structure of the organic thin film of the present invention.
FIG. 7-2 is a schematic cross-sectional view illustrating an exemplary laminate structure of the organic thin film of the present invention.
FIG. 8-1 is a schematic cross-sectional view illustrating an exemplary laminate structure of the organic thin film of the present invention.
FIG. 8-2 is a schematic cross-sectional view illustrating an exemplary laminate structure of the organic thin film of the present invention.
FIG. 9-1 is a schematic cross-sectional view illustrating an exemplary laminate structure of the organic thin film of the present invention.
FIG. 9-2 is a schematic cross-sectional view illustrating an exemplary laminate structure of the organic thin film of the present invention.
FIG. 10-1 is a schematic cross-sectional view illustrating an exemplary laminate structure of the organic thin film of the present invention.
FIG. 10-2 is a schematic cross-sectional view illustrating an exemplary laminate structure of the organic thin film of the present invention.
FIG. 11-1 is a schematic cross-sectional view illustrating an exemplary laminate structure of the organic thin film of the present invention.
FIG. 11-2 is a schematic cross-sectional view illustrating an exemplary laminate structure of the organic thin film of the present invention.
FIG. 12 is a graph of the relationship between the applied voltage and the luminance of organic EL devices produced in Examples 1 and 2 and Comparative Example 1.
FIG. 13 is a graph of the relationship between the applied voltage and the luminance of organic EL devices produced in Examples 3 and 4 and Comparative Examples 1 and 2.
FIG. 14 illustrates emissions of the organic EL devices produced in Examples 3 and 4 and Comparative Examples 1 and 2.
FIG. 15 is a graph of the relationship between the applied voltage and the luminance of organic EL devices produced in Example 5 and Comparative Example 3.
FIG. 16 is a graph of the relationship between the applied voltage and the luminance of organic EL devices produced in Examples 5 and 6.
FIG. 17 is a graph of the relationship between the applied voltage and the luminance of organic EL devices produced in Examples 7 and 8 and Comparative Example 4.
FIG. 18 is a graph of the relationship between the applied voltage and the luminance of organic EL devices produced in Examples 9 and 10.
FIG. 19 is a graph of the temporal change of the luminance of the organic EL devices produced in Examples 9 and 10.
FIG. 20 is a cross-sectional view illustrating a structure of an organic EL device produced in Example 11.
FIG. 21 is a graph of the relationship between the applied voltage and the luminance of the organic EL devices produced in Examples 8 and 11.
FIG. 22 is a graph of the relationship between the applied voltage and the luminance of organic EL devices produced in Examples 12 to 14 and Comparative Examples 5 and 6.
FIG. 23 is a graph of the temporal change of the luminance of the organic EL devices produced in Example 13 and Comparative Example 6.
FIG. 24 is a graph of the temporal change of the luminance of the organic EL devices produced in Examples 12 and 14.
FIG. 25 is a cross-sectional view illustrating a structure of an organic EL device produced in Example 15.
FIG. 26 is a graph of the relationship between the applied voltage and the luminance of the organic EL devices produced in Example 15 and Comparative Example 7.
FIG. 27 is a graph of the relationship between the applied voltage and the luminance of organic EL devices produced in Example 16 and Comparative Examples 8 and 9.
FIG. 28 is a graph of the relationship between the applied voltage and the luminance of organic EL devices produced in Examples 17 and Comparative Examples 10 and 11.
FIG. 29 is a graph of the relationship between the applied voltage and the luminance of organic EL devices produced in Examples 18 and Comparative Examples 12 and 13.
FIG. 30 is a graph of the relationship between the applied voltage and the luminance of organic EL devices produced in Example 19 and Comparative Example 15.
FIG. 31 is a graph of the relationship between the applied voltage and the luminance of organic EL devices produced in Example 20 and Comparative Example 14.
FIG. 32 is a graph of the temporal change of the luminance of the organic EL devices produced in Example 20 and Comparative Example 14 at room temperature.
FIG. 33 is a graph of the temporal change of the luminance of the organic EL devices produced in Example 20 and Comparative Example 14 at high temperature (85°C).
FIG. 34 is a graph of the relationship between the applied voltage and the luminance of organic EL devices produced in Examples 21 and 22 and Comparative Example 16.
FIG. 35 is a graph of the relationship between the applied voltage and the luminance of organic EL devices produced in Example 23 and Comparative Example 17.
FIG. 36 is a graph of the relationship between the applied voltage and the luminance of organic EL devices produced in Example 24 and Comparative Example 18.
FIG. 37 is a graph of the relationship between the applied voltage and the luminance of organic EL devices produced in Examples 25 and 26 and Comparative Example 19.

### DESCRIPTION OF EMBODIMENTS

The present invention is described in detail below. "Organic thin film and Organic EL device material"

The organic thin film of the present invention contains a first material which is a hexahydropyrimidopyrimidine compound having a structure of the following formula (1) and a second material which transports electrons. The organic thin film of the present invention may be a film of a single layer containing the first material and the second material or may be a laminate film including a layer containing the first material and a layer containing the second material.

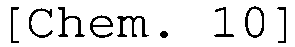

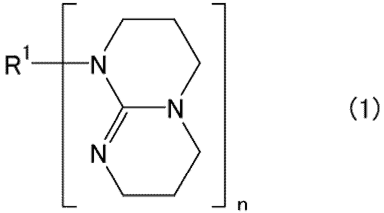

In the formula (1), R¹ is an optionally substituted aromatic hydrocarbon group, an optionally substituted aromatic heterocyclic group, an optionally substituted arylalkylene group, an optionally substituted divalent to tetravalent acyclic or cyclic hydrocarbon group, a group of a combination of two or more of these groups, or a group of a combination of one or more of these groups and a nitrogen atom; and n is an integer of 1 to 4 wherein the aromatic heterocyclic group is a compound consisting of one aromatic heterocyclic ring selected from thiophene, furan, pyrrole, oxazole, oxadiazole, thiazole, thiadiazole, imidazole, pyrimidine, pyrazine, or triazine; a compound in which any two or more of the compounds each consisting of one aromatic heterocyclic ring are directly bound to each other via a carbon-carbon bond, and bipyridine; and a group prepared by removing 1 to 4 hydrogen atoms from any of the aromatic heterocyclic rings of a condensed cyclic heteroaromatic hydrocarbon compound, selected from quinoline, quinoxaline, benzothiophene, benzothiazole, benzimidazole, benzoxazole, indole, carbazole, dibenzofuran, dibenzothiophene, acridine, or phenanthroline.

Since the first material constituting the organic thin film of the present invention is the organic material having a pKa of 1 or greater, it can extract a proton (H⁺) from the second material. The first material preferably has a pKa of 5 or greater, more preferably 11 or greater. The higher the pKa of the first material, the higher the ability of the first material to extract a proton from the second material. Thus, an organic EL device including the organic thin film used as, for example, an electron injection layer can achieve an excellent electron injection property and an excellent electron transport property. Further, it is confirmed that the first material is suitably placed at a defective portion of an inorganic compound to prevent reaction at an interface with oxygen or water entering from the outside, leading to an increase in atmospheric stability of the device. Furthermore, the first material can interact with an inorganic compound to reduce the work function of the inorganic compound. As a result, the electron injection property of the metal oxide layer can be improved.

Thus, the organic thin film of the present invention can be used not only in a device consisting only of organic compounds, but also in, in particular, a device including organic compounds and inorganic compounds, and can enhance the electron injection property and the atmospheric stability. The first material capable of reducing the work function of an inorganic compound is also capable of improving the efficiency of electron extraction from an organic compound used in an active layer that generates electrons by absorption of light, in an organic thin film solar cell or a photoelectric transducer.

The organic thin film of the present invention contains a hexahydropyrimidopyrimidine compound having a structure of the formula (1) as the first material. The organic thin film of the present invention, when used as an electron injection layer, can impart an excellent electron injection property and an excellent electron transport property. An organic EL device material containing a hexahydropyrimidopyrimidine compound having a structure of the formula (1) imparting such excellent effects is also one aspect of the present invention. An electron injection layer or an electron transport layer consisting of the organic EL device material of the present invention can impart an excellent electron injection property and an excellent electron transport property.

In the present invention, the "pKa" usually means the "acid dissociation constant in water". When the pKa cannot be measured in water, it means the "acid dissociation constant in dimethyl sulfoxide (DMSO)", and when the pKa cannot be measured even in DMSO, it means the "acid dissociation constant in acetonitrile". The pKa preferably means the "acid dissociation constant in water".

In the formula (1), R¹ is an optionally substituted aromatic hydrocarbon group, an optionally substituted aromatic heterocyclic group, an optionally substituted arylalkylene group, an optionally substituted divalent to tetravalent acyclic or cyclic hydrocarbon group, a group of a combination of two or more of these groups, or a group of a combination of one or more of these groups and a nitrogen atom.

The aromatic hydrocarbon group and the aromatic heterocyclic group each preferably have a carbon number of 3 to 30, more preferably 4 to 24, still more preferably 5 to 20.

Examples of the aromatic hydrocarbon group include a compound consisting of one aromatic ring, such as benzene; a compound in which multiple aromatic rings are directly bound to each other via a carbon-carbon bond, such as biphenyl or diphenylbenzene; and a group prepared by removing 1 to 4 hydrogen atoms from any of aromatic rings of a condensed cyclic aromatic hydrocarbon compound, such as naphthalene, anthracene, phenanthrene, or pyrene.

Examples of the arylalkylene group include a group of a combination of the aromatic hydrocarbon group and a C1-C3 alkylene group.

The divalent to tetravalent acyclic or cyclic hydrocarbon group preferably has a carbon number of 1 to 12, more preferably 1 to 6, still more preferably 1 to 4. The acyclic hydrocarbon group may be linear or branched.

R¹ may be a group of a combination of two or more groups selected from the aromatic hydrocarbon group, the aromatic heterocyclic group, the arylalkylene group, and the divalent to tetravalent acyclic hydrocarbon group.

R¹ may also be a group of a combination of one or more groups selected from the aromatic hydrocarbon group, the aromatic heterocyclic group, the arylalkylene group, and the divalent to tetravalent acyclic hydrocarbon group and a nitrogen atom. Examples of these groups include groups prepared by removing 1 to 4 hydrogen atoms from a trialkylamine such as trimethylamine or triphenylamine.

The aromatic hydrocarbon group, the aromatic heterocyclic group, and the arylalkylene group may contain one or more monovalent substituents.

Examples of the monovalent substituent include a fluorine atom; haloalkyl groups such as fluoromethyl, difluoromethyl, and trifluoromethyl groups; C1-C20 linear or branched acyclic alkyl groups such as methyl, ethyl, propyl, isopropyl, butyl, isobutyl, and tert-butyl groups; C5-C7 cyclic alkyl groups such as cyclopentyl, cyclohexyl, and cycloheptyl groups; C1-C20 linear or branched acyclic alkoxy groups such as methoxy, ethoxy, propoxy, isopropoxy, butoxy, isobutoxy, tert-butoxy, pentyloxy, hexyloxy, heptyloxy, and octyloxy groups; a nitro group; a cyano group; C1-C10 alkyl group-containing alkylamino groups such as methylamino, ethylamino, dimethylamino, and diethylamino groups; cyclic amino groups such as pyrrolidino, piperidino, and morpholino group; diarylamino groups such as diphenylamino and carbazolyl groups; acyl groups such as acetyl, propionyl, and butyryl groups; C2-C30 alkenyl groups such as a styryl group; a C5-C20 aryl group optionally substituted with a halogen atom such as a fluorine atom, a C1-C20 alkyl group, a C1-C20 alkoxy group, a C1-C20 amino group, or the like (specific examples of the aryl group include the above-described examples of the aromatic hydrocarbon group); a C4-C20 heterocyclic group containing at least one of a nitrogen atom, a sulfur atom, or an oxygen atom, optionally substituted with a halogen atom such as a fluorine atom, a C1-C20 alkyl group, a C1-C20 alkoxy group, an amino group, or the like (the heterocyclic group may consist of one ring or may be a compound in which multiple compounds each consisting of one aromatic heterocyclic ring are directly bound to each other via a carbon-carbon bond, or a condensed heterocyclic group, and specific examples of the heterocyclic group include the above-described specific examples of the aromatic heterocyclic group); and ester groups and thioether groups. These groups may be substituted with a halogen atom, a heteroatom, an alkyl group, or an aromatic ring, for example.

The letter n in the formula (1) is an integer of 1 to 4, preferably 2 or 3.

Specific examples of the hexahydropyrimidopyrimidine compound having a structure of the formula (1) include compounds of any of the following formulas 2-1, 2-4, 2-6 - 2-10, 2-12 - 2-22 and 2-25 - 2-34. The other compounds are not according to the invention.

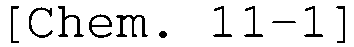

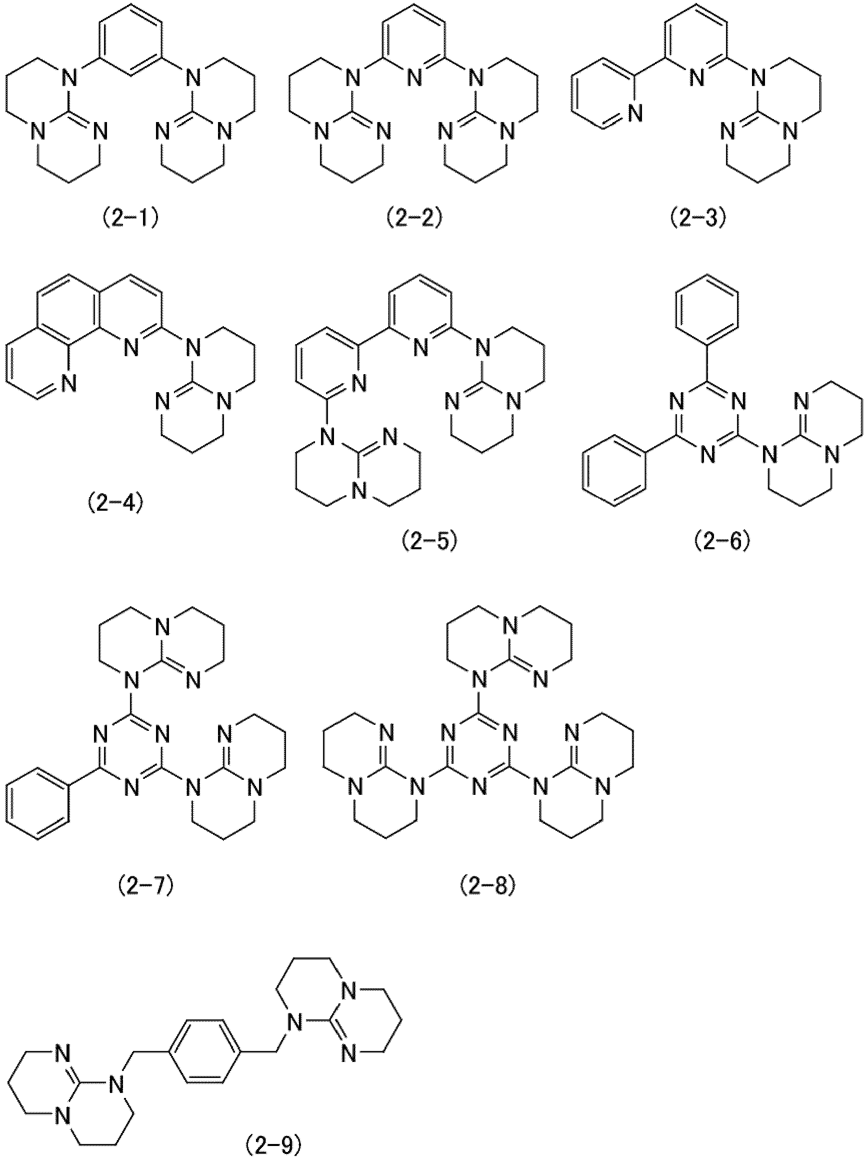

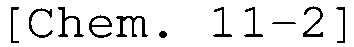

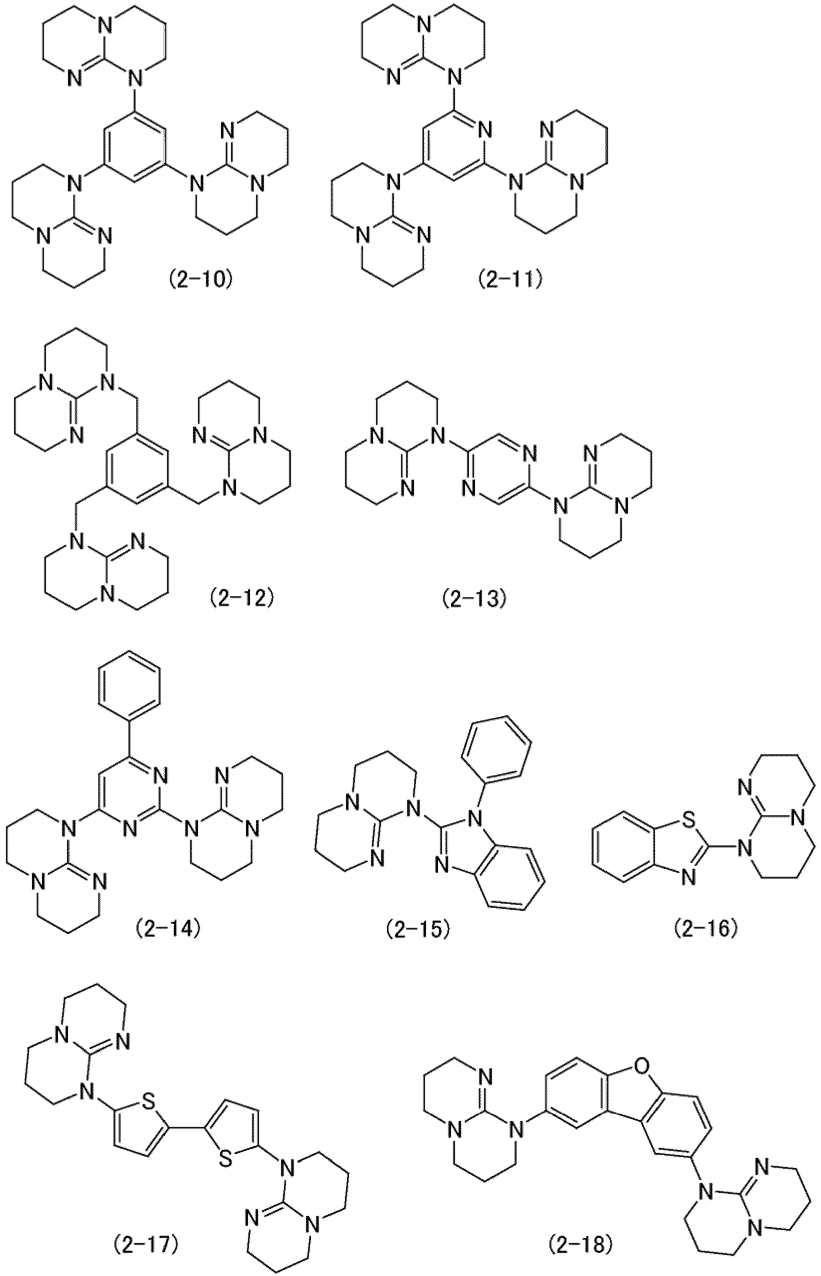

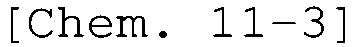

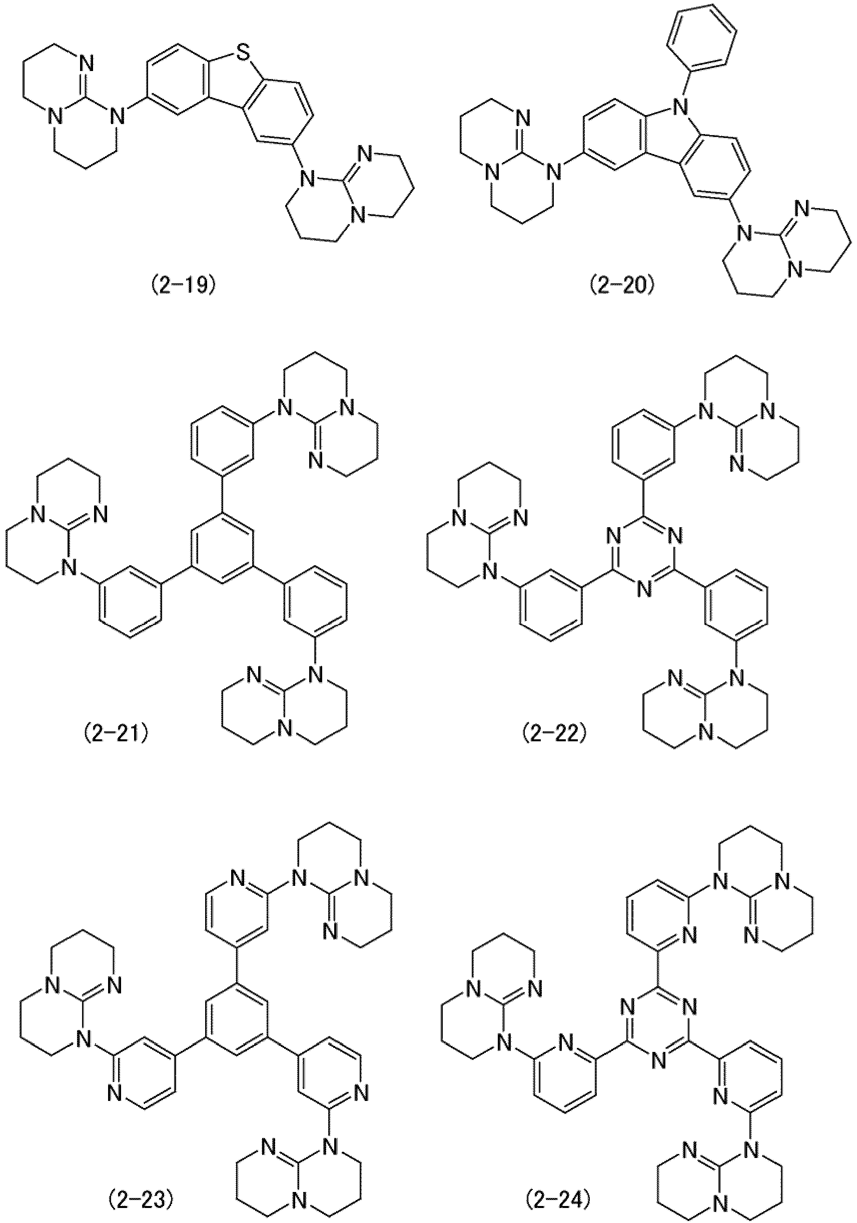

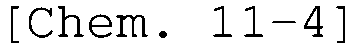

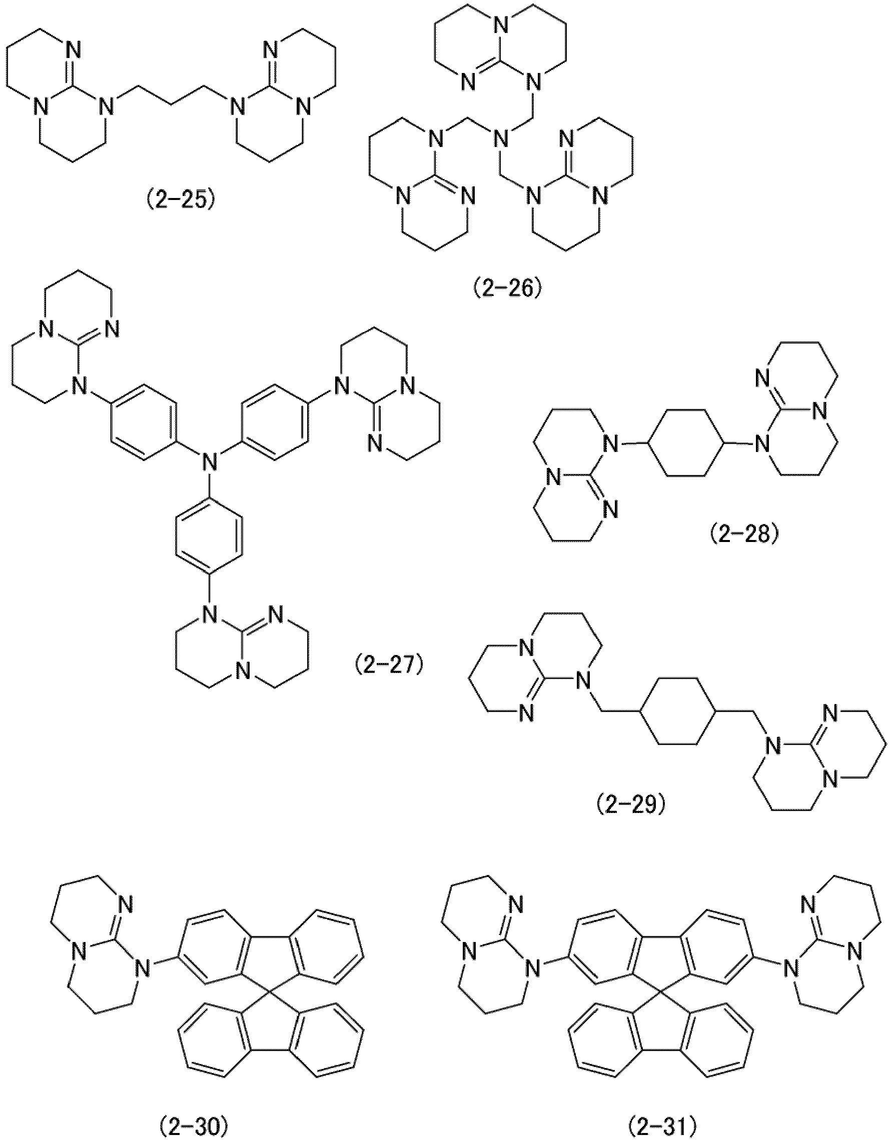

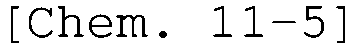

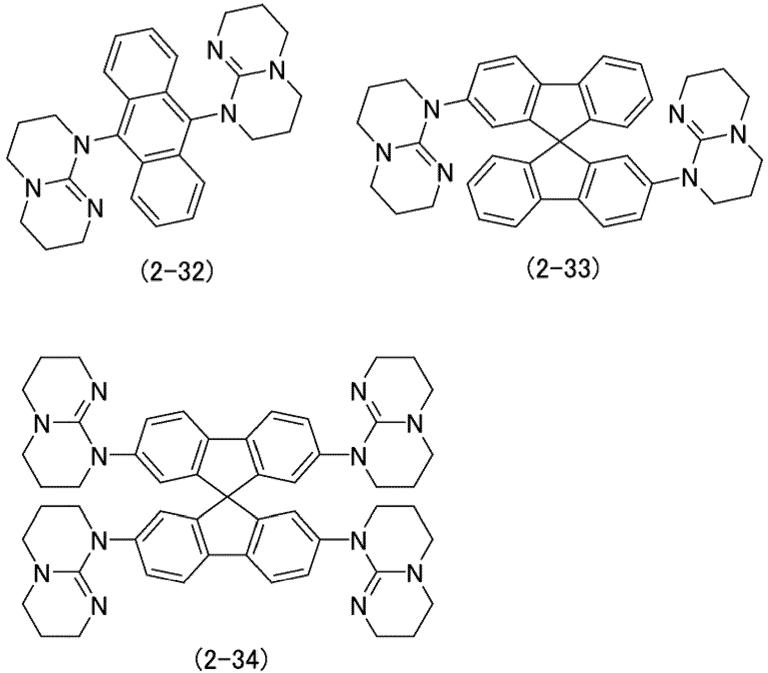

The compound of the formula (1) can be synthesized from an iodine-, bromine-, chlorine-, or fluorine-containing halogen compound and hexahydropyrimidopyrimidine as starting materials by the Ullmann coupling reaction, the Buchwald-Hartwig amination reaction, or the nucleophilic substitution reaction as shown in the following scheme (3).

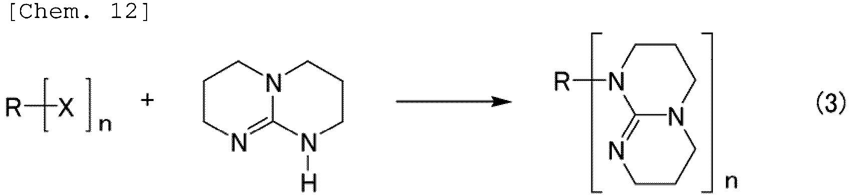

The second material has only to be a material which transports electrons, and is preferably an organic material, more preferably an organic material having a lowest unoccupied molecular orbital (LUMO) level of 2.0 eV to 4.0 eV. Particularly preferred is an n-type organic semiconductor material having a LUMO level of 2.5 eV to 3.5 eV. For example, any of the below-described conventionally known materials may be used as a material of an electron transport layer of the organic EL device. In particular, a material satisfying the requirements of the LUMO level is preferred.

Specific examples of the second material include phosphine oxide derivatives such as phenyl-dipyrenylphosphine oxide (POPy₂); pyridine derivatives such as tris-1,3,5-(3'-(pyridin-3"-yl)phenyl)benzene (TmPhPyB), 1,3,5-tris(6-(3-(pyridin-3-yl)phenyl)pyridine-2-yl)benzene, and 8,9-diphenyl-7,10-(3-(pyridin-3-yl))fluoranthene; quinoline derivatives such as (2-(3-(9-carbazolyl)phenyl)quinoline (mCQ)); pyrimidine derivatives such as 2-phenyl-4,6-bis(3,5-dipyridylphenyl)pyrimidine (BPyPPM), 2-methyl-4,6-bis(3,5-dipyridylphenyl)pyrimidine, and 9-(4-(4,6-diphenylpyrimidine-2-yl)phenyl)-9H-carbazole; pyrazine derivatives; phenanthroline derivatives such as bathophenanthroline (BPhen) and 2,9-dimethyl-4,7-diphenyl-1,10-phenanthroline (BCP); triazine derivatives such as 2,4-bis(4-biphenyl)-6-(4'-(2-pyridinyl)-4-biphenyl)-(1,3,5)triazine (MPT), tris-1,3,5-(3'-(pyridin-3"-yl)phenyl)triazine (TmPhPyTz), tris-1,3,5-([1,1'-biphenyl)-3-yl)triazine, 2-(3-(4,6-di(pyridin-3-yl)-1,3,5-triazin-2-yl)phenyl)-1-phenyl-1H-benzo[d]imidazole, 9-(4-(4,6-diphenyl-1,3,5-triazin-2-yl)phenyl)-9H-3,9'-bicarbazole, 9-(4-(4,6-diphenyl-1,3,5-triazin-2-yl)phenyl)-9H-carbazole, 11-(4,6-diphenyl-1,3,5-triazin-2-yl)-12-phenyl-11,12-dihydroindolo(2,3-a)carbazole, 12-(2-(4, 6-diphenyl-1,3,5-triazin-2-yl)phenyl)-12H-benzofuro[2,3,a]-carbazole, and 9-((4-(4,6-dipyridin-3-yl)-1,3,5-triazin-2-yl)phenyl)-9H-carbazole; triazole derivatives such as 3-phenyl-4-(1'-naphthyl)-5-phenyl-1,2,4-triazole (TAZ); oxazole derivatives; oxadiazole derivatives such as 2-(4-biphenyl)-5-(4-tert-butylphenyl-1,3,4-oxadiazole) (PBD); imidazole derivatives such as 2,2',2"-(1,3,5-benzenetriyl)-tris(1-phenyl-1-H-benzimidazole) (TPBI); aromatic tetracarboxylic anhydrides such as naphthalene and perylene; a compound containing a carbonyl-containing heterocyclic ring such as a compound of the below-described formula (24); various metal complexes typified by bis(2-(2-hydroxyphenyl)benzothiazolato) zinc (Zn(BTZ)₂) and tris(8-hydroxyquinolinato) aluminum (Alq₃); organic silane derivatives typified by silole derivatives such as 2,5-bis(6'-(2',2"-bipyridyl))-1,1-dimethyl-3,4-diphenylsilole (PyPySPyPy); and boron-containing compounds disclosed in Japanese Patent Application No. 2012-228460, Japanese Patent Application No. 2015-503053, Japanese Patent Application No. 2015-053872, Japanese Patent Application No. 2015-081108, and Japanese Patent Application No. 2015-081109. One or two or more of these may be used.

The second material may also be the below-described material of an emitting layer.

Of these, the second material is more preferably a phosphine oxide derivative such as POPy₂, a boron-containing compound of any of the following formulas (4) to (7), a metal complex such as Alq₃, a pyridine derivative such as TmPhPyB, or a triazine derivative such as TmPhPyTz. Of these, the second material is particularly preferably a boron-containing compound or a triazine derivative. The letter n¹ in the formula (6) is an integer of 1 or more.

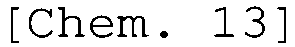

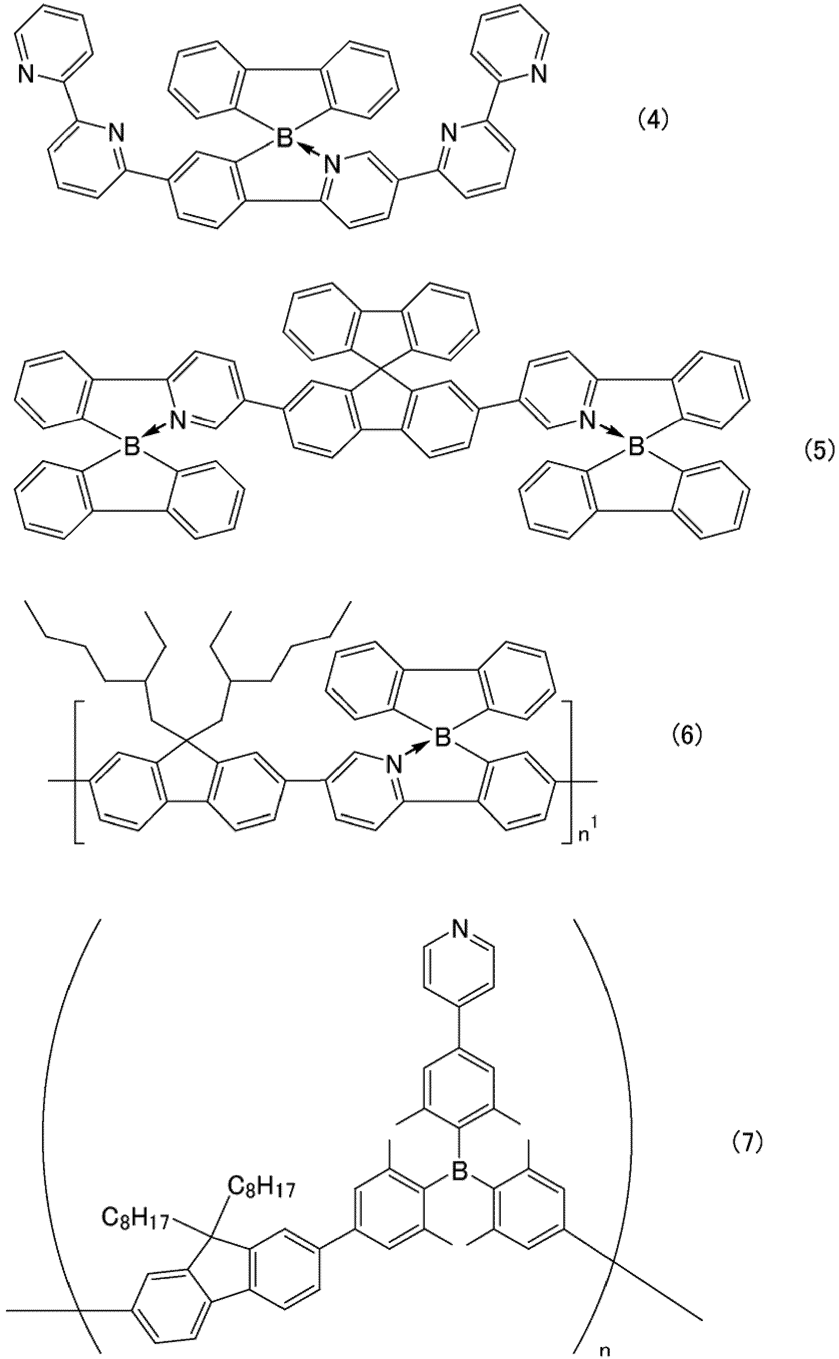

When a boron-containing compound is used as the second material having an electron transport property, a uniform organic thin film is easily obtained by application of a coating composition containing the first material and the second material. The coating composition containing the first material of the formula (1) and the second material which transports electrons is another aspect of the present invention. Furthermore, since the hexahydropyrimidopyrimidine compound which serves as the first material of the formula (1) has an excellent electron injection property, a coating composition containing the first material, free from the second material is also useful. The coating composition containing a hexahydropyrimidopyrimidine compound which serves as the first material of the formula (1) is another aspect of the present invention.

A boron-containing compound and a triazine derivative are suitable for a material of an electron injection layer of an organic EL device owing to their deep unoccupied molecular orbital (LUMO) levels. Thus, an organic thin film containing a boron-containing compound as the second material is suitable particularly as an electron injection layer of an organic EL device.

The first material and the second material may be present at any ratio in the organic thin film of the present invention, and the ratio may be appropriately determined depending on the types of compounds used as the first material and the second material. The ratio (mass ratio) between the first material and the second material (first material:second material) is preferably 0.1:99.9 to 20:1. The ratio is more preferably 0.5:99 to 10:1. For example, when the first material is a compound of the formula (2-2) and the second material is a boron-containing compound of the formula (4), the ratio between the first material and the second material is preferably within the range of the mass ratio indicated above. With a ratio within the mass ratio indicated above, the electron transport property and the electron injection property are remarkably improved owing to the presence of the first material and the second material in the organic thin film.

The organic thin film of the present invention may be a single film containing the first material and the second material or a laminate film including a film containing at least the first material and a film containing at least the second material. The laminate film may be a laminate film including a film containing only the first material and a film containing only the second material or may be a laminate film including a film containing the first material and the second material and a film containing either the first material or the second material. In the laminate film including a film containing the first material and the second material and a film containing either the first material or the second material, the film containing either the first material or the second material may contain either the first material or the second material, and preferably contains the second material.

When such an organic thin film is used in an organic electroluminescence device as a constituent layer, either the film containing the first material and the second material or the film containing either the first material or the second material may be located on a cathode side, and preferably, the film containing either the first material or the second material is located on the cathode side.

When the organic thin film of the present invention is a laminate film including a film containing the first material and the second material and a film containing only the first material, the first material is present in the both two films of the laminate. The first material may be the same or different between the two films.

When the organic thin film of the present invention is a laminate film including a film containing the first material and the second material and a film containing only the second material, the second material is present in the both two films of the laminate. The second material may be the same or different between the two films.

### "Method for producing organic thin film"

The following describes a method for producing the organic thin film of the present invention with examples.

The organic thin film of the present invention contains a first material which is a hexahydropyrimidopyrimidine compound having an acid dissociation constant pKa of 1 or greater and having a structure of the formula (1) and a second material which transports electrons. Due to the relatively large molecular weights of the first material and the second material, the organic thin film of the present invention can be formed not only by application but also by evaporation. Thus, an organic EL device including the organic thin film of the present invention can be produced with little restriction on the production process, and the organic thin film can be easily used as materials of layers constituting the organic EL device.

When the organic thin film is produced by evaporation, it may be produced by the same evaporation as in the production of the other layers constituting the organic EL device. The first material and the second material may be evaporated simultaneously or sequentially. When the first material and the second material are evaporated sequentially, either of them may be evaporated first. Alternatively, either the first material or the second material may be evaporated, followed by co-evaporation of both of them, or both the first material and the second material may be co-evaporated, followed by evaporation of either of them. In a preferred embodiment of the method for producing an organic thin film of the present invention, the method for producing an organic thin film includes simultaneous evaporation of the first material which is a hexahydropyrimidopyrimidine compound having a structure of the formula (1) and the second material which transports electrons, on a surface on which the organic thin film is to be formed. In another preferred embodiment of the method for producing an organic thin film of the present invention, the method for producing an organic thin film includes evaporation of one of the first material or the second material on a surface on which the organic thin film is to be formed, followed by evaporation of the other material or both of the materials, or the method for producing an organic thin film includes simultaneous evaporation of the first material and the second material on a surface on which the organic thin film is to be formed, followed by evaporation of either the first material or the second material.

The organic thin film of the present invention can also be produced by application. In this case, the organic thin film can be produced by a method including preparing a coating composition containing the first material and the second material which transports electrons and applying the coating composition or a method including preparing a coating composition containing the first material and a coating composition containing the second material and sequentially applying them. When the coating composition containing the first material and the coating composition containing the second material are applied sequentially, either of them may be applied first. Alternatively, the coating composition containing either of the materials may be applied, followed by application of the coating composition containing both of the materials, or the coating composition containing both of the materials may be applied, followed by application of the coating composition containing either of the materials. In a preferred embodiment of the method for producing an organic thin film of the present invention, the method for producing an organic thin film includes application of a coating composition containing the first material which is a hexahydropyrimidopyrimidine compound having a structure of the formula (1) and the second material which transports electrons on a surface on which the organic thin film is to be formed. In another preferred embodiment of the method for producing an organic thin film of the present invention, the method for producing an organic thin film includes application of one of a coating composition containing only the first material or a coating composition containing only the second material to a surface on which the organic thin film is to be formed to form a coat, followed by application of the other coating composition or a coating composition containing both of the materials to the coat, or the method for producing an organic thin film includes application of the coating composition containing both of the first material and the second material to form a coat, followed by application of the coating composition containing either the first material or the second material to the coat.

The following describes the method for producing an organic thin film including preparing a coating composition containing the first material which is a hexahydropyrimidopyrimidine compound having a pKa of 1 or greater and having a structure of the specific formula (1) and the second material which transports electrons and applying the coating composition.

The coating composition may be obtained by adding predetermined amounts of the first material and the second material to a solvent in a vessel or adding a solvent to the first material and the second material in a vessel and stirring the contents for dissolution, for example. Examples of the solvent to dissolve the first material and the second material include inorganic solvents, organic solvents, and solvent mixtures containing any of these solvents.

Examples of the inorganic solvents include nitric acid, sulfuric acid, ammonia, hydrogen peroxide, water, phosphoric acid, and hydrochloric acid.

Examples of the organic solvents include ketone-based solvents such as methyl ethyl ketone (MEK), acetone, diethyl ketone, methyl isobutyl ketone (MIBK), methyl isopropyl ketone (MIPK), diisobutyl ketone, 3,5,5-trimethylcyclohexanone, diacetone alcohol, cyclopentanone, and cyclohexanone; alcohol-based solvents such as methanol, ethanol, isopropanol, ethylene glycol, diethylene glycol (DEG), and glycerine; ether-based solvents such as diethyl ether, diisopropyl ether, 1,2-dimethoxy ethane (DME), 1,4-dioxane, tetrahydrofuran (THF), tetrahydropyran (THP), anisole, diethylene glycol dimethyl ether (diglyme), and diethylene glycol ethyl ether (carbitol); cellosolve-based solvents such as methyl cellosolve, ethyl cellosolve, and phenyl cellosolve; aliphatic hydrocarbon-based solvents such as hexane, pentane, heptane, and cyclohexane; aromatic hydrocarbon-based solvents such as toluene, xylene, and benzene; aromatic heterocyclic compound-based solvents such as pyridine, pyrazine, furan, pyrrole, thiophene, and methylpyrrolidone; amide-based solvents such as N,N-dimethylformamide (DMF) and N,N-dimethylacetamide (DMA); halogen compound-based solvents such as chlorobenzene, dichloromethane, chloroform, and 1,2-dichloroethane; ester-based solvents such as ethyl acetate, methyl acetate, and ethyl formate; sulfur compound-based solvents such as dimethyl sulfoxide (DMSO) and sulfolane; nitrile-based solvents such as acetonitrile, propionitrile, and acrylonitrile; organic acid-based solvents such as formic acid, acetic acid, trichloroacetic acid, and trifluoroacetic acid; organic amine-based solvents such as triethylamine and pyridine; carbonate-based solvents such as diethyl carbonate, ethyl methyl carbonate, ethylene carbonate, and propylene carbonate. Preferred among these are ketone-based solvents such as methyl ethyl ketone (MEK), acetone, diethyl ketone, methyl isobutyl ketone (MIBK), methyl isopropyl ketone (MIPK), diisobutyl ketone, 3,5,5-trimethylcyclohexanone, diacetone alcohol, and cyclopentanone.

Various methods can be used to apply the coating composition containing the first material and the second material, and examples thereof include a spin coating method, a casting method, a micro gravure coating method, a gravure coating method, a bar coating method, a roll coating method, a wire bar coating method, a dip coating method, a spray coating method, a screen printing method, a flexographic printing method, an offset printing method, and an inkjet printing method.

After the application of the coating composition by such a method, the coating composition is preferably annealed at 70°C to 200°C for 0.1 to 5 hours in a nitrogen atmosphere or in the air atmosphere. Such annealing can vaporise solvents, forming an organic thin film.

The hexahydropyrimidopyrimidine compound which serves as the first material of the formula (1) is excellent in electron injection property. Thus, an organic thin film containing the first material, but free from the second material is also useful as a material of organic EL devices and the like. A method for producing an organic thin film including forming a film containing such a hexahydropyrimidopyrimidine compound having a structure of the formula (1) on a surface to be coated is another aspect of the present invention, and in a preferred embodiment of the method for producing an organic thin film, the method includes forming a film containing such a hexahydropyrimidopyrimidine compound having a structure of the formula (1) on a surface to be coated containing the second material.

### "Organic EL device"

The present invention also relates to an organic electroluminescence (EL) device including a cathode, an anode, a light emitting layer therebetween, and a layer of the organic thin film of the present invention, a layer of a laminate film including the layer of the organic thin film and a metal oxide layer, or a layer containing the organic electroluminescence device material of the present invention.

The layer of the organic thin film, the layer of a laminate film including the layer of the organic thin film and a metal oxide layer, or the layer containing the organic electroluminescence device material of the present invention may be present between the cathode and the emitting layer or between the anode and the emitting layer in the organic EL device of the present invention, preferably between the cathode and the emitting layer.

The layer containing the organic electroluminescence device material of the present invention may contain a different component as long as it contains a hexahydropyrimidopyrimidine compound having a structure of the formula (1). Preferably, it consists of a hexahydropyrimidopyrimidine compound.

The present invention also relates to an organic electroluminescence (EL) device including a cathode, an anode, an emitting layer therebetween, and a layer of the organic thin film of the present invention or a layer of a laminate film including the layer of the organic thin film and a metal oxide layer between the cathode and the anode, with the light emitting layer containing the second material. The present invention also relates to an organic electroluminescence (EL) device including a layer of the organic thin film of the present invention or a layer of a laminate film including the layer of the organic thin film and a metal oxide layer between the cathode and the anode. The organic thin film or the laminate film is adjacent to the emitting layer and contains a material to be used for an emitting layer as the second material, or the organic thin film or the laminate film contains the second material and serves as an emitting layer.

The hexahydropyrimidopyrimidine compound which serves as the first material in the present invention is remarkably excellent in electron injection property and thus can directly inject electrons into an emitting material and a host material to be used for an emitting layer. Thus, use of the first material in the present invention for the electron injection layer enables reduction in the number of materials to be used and enables reduction in the number of layers to be laminated, leading to simplification of the structure of the organic EL device.

In other words, even in a device including the organic thin film or the laminate film of the present invention in which a layer containing the first material, a layer containing the second material, and an emitting layer are adjacent to each other, and an emitting material or a host material for an emitting layer is used as a material of the layer containing the second material, electrons can be injected from the layer containing the first material, and the layer containing the second material and the emitting layer can be formed using the same material, whereby the number of materials to be used can be reduced. In this case, the second material may be either an emitting material or a host material for an emitting layer. When a layer containing the second material formed from an emitting material or a host material for an emitting layer is used as an emitting layer, formation of an additional emitting layer can be eliminated, whereby the number of layers to be laminated can be reduced and an organic EL device having a simpler structure can be provided. Further, when the second material is used for a layer between the emitting layer and the anode and adjacent to the emitting layer, the number of layers to be laminated can be further reduced and an organic EL device having a simpler structure can be provided. Thus, in a preferred embodiment of the organic electroluminescence (EL) device of the present invention, the emitting layer contains the second material, and a layer containing the second material is present between the anode and the emitting layer.

The following specifically describes the organic EL device of the present invention with examples.

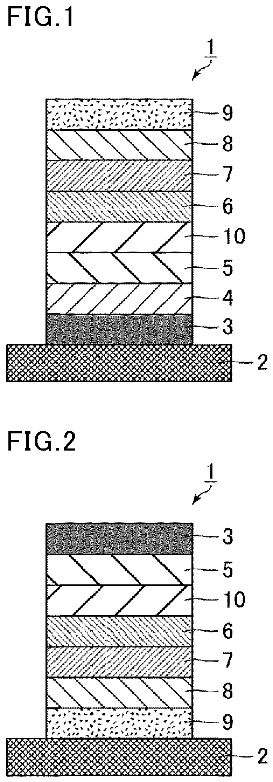

FIG. 1 is an explanatory schematic cross-sectional view illustrating an exemplary organic EL device of the present invention. An organic EL device 1 of the present invention illustrated in FIG. 1 includes a cathode 3, an anode 9, and an emitting layer 6 therebetween. The organic EL device 1 illustrated in FIG. 1 includes an electron injection layer 5 made of the organic thin film of the present invention or the organic electroluminescence device material of the present invention between the cathode 3 and the emitting layer 6. An oxide layer 4 is present between the cathode 3 and the organic thin film of the present invention or the electron injection layer 5 made of the organic electroluminescence device material of the present invention, and the oxide layer 4 is adjacent to the electron injection layer 5. These features are included in preferred embodiments of the organic EL device of the present invention.

The organic EL device 1 of the present invention has a laminate structure in which the following layers are formed on a substrate 2 in the stated order: the cathode 3, the inorganic oxide layer 4, the electron injection layer 5, an electron transport layer 10, the emitting layer 6, a hole transport layer 7, a hole injection layer 8, and the anode 9. In a preferred embodiment of the organic EL device of the present invention, an inorganic oxide layer is present between the cathode and a layer of the organic thin film or a layer of the organic electroluminescence device material of the present invention.

The organic EL device 1 illustrated in FIG. 1 is an inverted organic EL device including the cathode 3 between the substrate 2 and the emitting layer 6. Also, the organic EL device 1 illustrated in FIG. 1 is an organic-inorganic hybrid organic electroluminescence device (HOILED device) in which one or more of the layers that constitute the organic EL device (at least the inorganic oxide layer 4) is formed from an inorganic compound.

The organic EL device 1 illustrated in FIG. 1 may be a top emission device in which light is extracted from the side opposite to the substrate 2, or may be a bottom emission device in which light is extracted from a substrate 2 side.

FIG. 2 illustrates an exemplary device structure of the organic EL device of the present invention, which is a conventional organic EL device, in which the emitting layer 6 is present between the substrate 2 and the cathode 3.

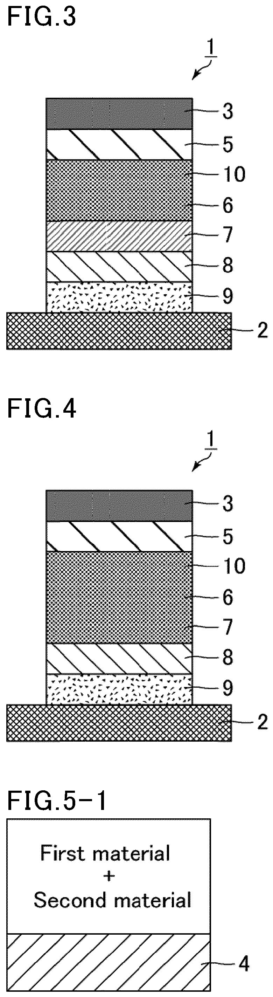

As described above, the hexahydropyrimidopyrimidine compound in the present invention is remarkably excellent in electron injection property and thus can directly inject electrons into a material to be used for an emitting layer. Thus, when an organic EL device material containing the hexahydropyrimidopyrimidine compound in the present invention is used and the hexahydropyrimidopyrimidine compound is used for the electron injection layer 5, even an organic EL device having a simple structure in which the electron transport layer 10 is made of a material used for an emitting layer and the emitting layer 6 and the electron transport layer 10 are made of the same material, as illustrated in FIG. 3, can be driven with a low voltage. Such a device can be produced using one or more fewer materials than a typical organic EL device. Here, when the electron transport layer 10 contains the second material in the present invention, the organic EL device can be said to include the organic thin film of the present invention.

Further, the hole injection layer 8 can relatively easily inject holes into a material used for an emitting layer. Thus, even a device in which a material used for an emitting layer is used for a hole transport layer 7, as illustrate in FIG. 4, can be driven with a low voltage when the organic EL device material containing the hexahydropyrimidopyrimidine compound of the present invention is used for the electron injection layer 5. Such a device can be produced using two or more fewer materials than a typical organic EL device. When the electron transport layer 10 contains the second material in the present invention, the organic EL device can be said to include the organic thin film of the present invention.

The above describes a conventional organic EL device with reference to figures. The organic EL device of the present invention in which the emitting layer contains the second material is not limited to a conventional organic EL device, but may be an inverted organic EL device.

The organic EL device will be described with an inverted organic EL device as an example in the present embodiment described below. The organic EL device of the present invention may be a conventional organic EL device in which the anode is present between the substrate and the emitting layer. When the organic EL device of the present invention is a conventional organic EL device, it also has the organic thin film between the cathode and the emitting layer like an inverted organic EL device. In an inverted organic EL device, the electron injection layer may also be referred to as an organic buffer layer. All of the contents described below shall be applied to conventional organic EL devices.

### "Substrate"

The substrate 2 is made of, for example, a resin material or a glass material.

Examples of the resin material of the substrate 2 include polyethylene terephthalate, polyethylene naphthalate, polypropylene, cycloolefin polymers, polyamides, polyethersulfone, polymethylmethacrylate, polycarbonate, and polyarylate. The substrate 2 made of a resin material is preferred because it makes the organic EL device 1 to be highly flexible.

Examples of the glass material of the substrate 2 include silica glass and soda glass.

When the organic EL device 1 is a bottom emission device, a transparent substrate may be used as the material of the substrate 2.

When the organic EL device 1 is a top emission device, not only a transparent substrate but also an opaque substrate may be used as the material of the substrate 2. Examples of the opaque substrate include a substrate made of a ceramic material such as alumina, a substrate in which an oxide film (insulating film) is formed on the surface of a metal plate such as a stainless steel plate, and a substrate made of a resin material.

The average thickness of the substrate 2 may be determined according to, for example, the material of the substrate 2, and is preferably 0.1 to 30 mm, more preferably 0.1 to 10 mm. The average thickness of the substrate 2 can be measured with a digital multimeter or a caliper.

### "Cathode"

The cathode 3 is formed in direct contact with the substrate 2.

Examples of a material of the cathode 3 include an oxide such as indium tin oxide (ITO), indium zinc oxide (IZO), fluorine tin oxide (FTO), In₃O₃, SnO₂, Sb-containing SnO₂, or Al-containing ZnO; Al, Au, Pt, Ag, and Cu; and an alloy containing any of these, which is a conductive material. Preferred among these materials of the cathode 3 are ITO, IZO, and FTO.

The average thickness of the cathode 3 is not limited, and is preferably 10 to 500 nm, more preferably 100 to 200 nm.

The average thickness of the cathode 3 can be measured with a stylus profiler, a spectroscopic ellipsometer, or a quartz crystal thickness monitor.

### "Oxide layer"

The inorganic oxide layer 4 functions as an electron injection layer and/or a cathode. The oxide layer 4 is a layer including a thin semiconductor film and/or a thin insulating film. Specifically, the oxide layer 4 may be a layer consisting of a single metal oxide; a laminate of layers each consisting of a combination of two or more different metal oxides, a laminate of layers each consisting of a single metal oxide, or a laminate of a layer(s) each consisting of a combination of two or more different metal oxides and a layer(s) consisting of a single metal oxide; or a layer consisting of a combination of two or more different metal oxides.

Examples of a metal element of the metal oxide that forms the oxide layer 4 include magnesium, calcium, strontium, barium, titanium, zirconium, hafnium, vanadium, niobium, tantalum, chromium, molybdenum, tungsten, manganese, indium, gallium, iron, cobalt, nickel, copper, zinc, cadmium, aluminum, and silicon.

When the oxide layer 4 includes a layer consisting of a combination of two or more different metal oxides, at least one of the metal elements of the metal oxides is preferably magnesium, aluminum, calcium, zirconium, hafnium, silicon, titanium, or zinc.

When the oxide layer 4 is a layer consisting of a single metal oxide, the metal oxide is preferably selected from the group consisting of magnesium oxide, aluminum oxide, zirconium oxide, hafnium oxide, silicon oxide, titanium oxide, and zinc oxide.

When the oxide layer 4 is a laminate of layers each consisting of a combination of two or more different metal oxides, a laminate of layers each consisting of a single metal oxide, a laminate of a layer(s) each consisting of a combination of two or more different metal oxides and a layer(s) consisting of a single metal oxide, or a layer consisting of a combination of two or more different metal oxides, two different metal oxides may be laminated and/or combined selected as a combination from, for example, titanium oxide/zinc oxide, titanium oxide/magnesium oxide, titanium oxide/zirconium oxide, titanium oxide/aluminum oxide, titanium oxide/hafnium oxide, titanium oxide/silicon oxide, zinc oxide/magnesium oxide, zinc oxide/zirconium oxide, zinc oxide/hafnium oxide, zinc oxide/silicon oxide, and calcium oxide/aluminum oxide; and three different metal oxides may be laminated and/or combined selected as a combination from, for example, titanium oxide/zinc oxide/magnesium oxide, titanium oxide/zinc oxide/zirconium oxide, titanium oxide/zinc oxide/aluminum oxide, titanium oxide/zinc oxide/hafnium oxide, titanium oxide/zinc oxide/silicon oxide, and indium oxide/gallium oxide/zinc oxide.

The oxide layer 4 may optionally contain indium gallium zinc oxide (IGZO) which is an oxide semiconductor with a particular composition and good characteristics and/or a 12CaO·7Al₂O₃ electride.

The average thickness of the oxide layer 4 is not limited, and is preferably 1 to 1000 nm, more preferably 2 to 100 nm.

The average thickness of the oxide layer 4 can be measured with a stylus profiler or a spectroscopic ellipsometer.

### "Electron injection layer"

The electron injection layer 5 improves the injection rate of electrons from the cathode to the emitting layer 6 and the electron transport property. The electron injection layer 5 includes the organic thin film.

The average thickness of the electron injection layer 5 is preferably 0.5 to 100 nm, more preferably 1 to 100 nm, still more preferably 5 to 100 nm, particularly preferably 10 to 50 nm. In the case of forming an electron injection layer 5 having an average thickness of 0.5 nm or greater, the electron injection layer 5 may be formed by applying a coating composition containing the first material and the second material, by sequentially applying a coating composition containing the first material and a coating composition containing the second material, or by forming separately the first material and the second material into layers and laminating the layers. In this case, the resulting electron injection layer 5 has a smooth surface and sufficiently prevents leakage caused in the production of the organic EL device 1. In the case of forming an electron injection layer 5 having an average thickness of 100 nm or smaller, an increase in the voltage for driving the organic EL device 1 due to the formation of the electron injection layer 5 can be sufficiently prevented.

The electron injection layer 5 may be formed by vapor evaporation. Specifically, it may be formed by simultaneous evaporation of the first material and the second material or by vapor evaporation of one of the first material and the second material, followed by vapor evaporation of the other material.

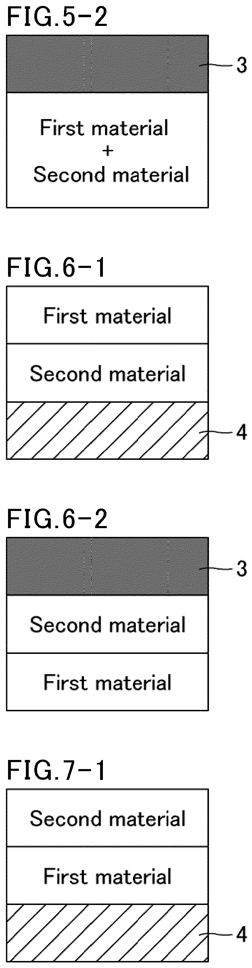

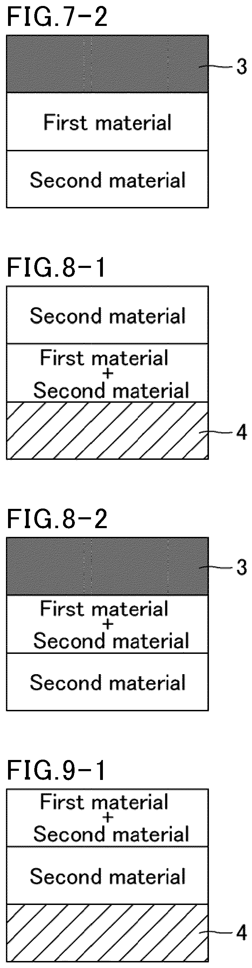

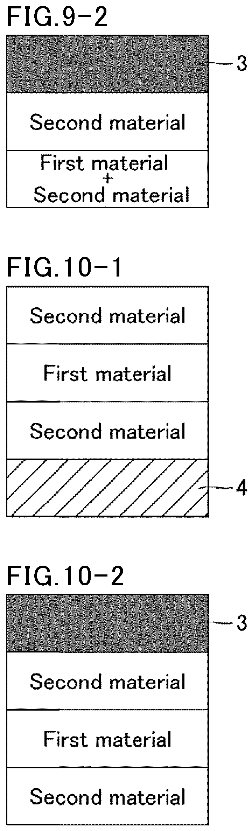

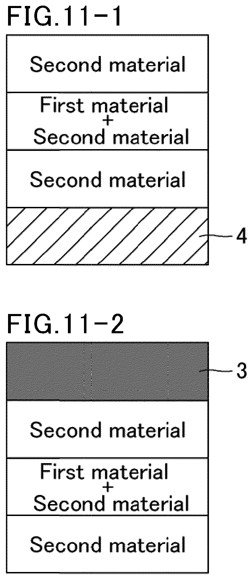

A film containing the first material and the second material may have any of the structures illustrated in FIGs. 5-1 to 11-1 (FIGs. 5-2 to 11-2 in the case of a conventional structure). For example, a single layer of the film containing the first material and the second material as a whole may constitute an electron injection layer (FIGs. 5-1 and 5-2), or one of a film consisting of the first material or a film consisting of the second material may be formed adjacent to the cathode or the oxide, and the other film may be formed adjacent to the film (FIGs. 6-1, 6-2, 7-1, and 7-2). A film containing the first material and the second material may be formed adjacent to the cathode or the oxide, and a film consisting of the second material may be formed adjacent to the film (FIGs. 8-1 and 8-2), or a film consisting of the second material, free from the first material, may be formed adjacent to the cathode or the oxide, and a film containing the first material and the second material may be formed adjacent to the film (FIGs. 9-1 and 9-2). Furthermore, a film consisting of the first material or a film containing the first material and the second material may be present between films each consisting of the second material to have a three-layer structure (FIGs. 10-1, 10-2, 11-1, and 11-2). The films of the present invention encompass the films having the structures illustrated in FIGs. 5-1 to 11-1 and FIGs. 5-2 to 11-2. When the second material is contained in both two adjacent films or in two or more films of a three-layer structure as illustrated in FIGs. 8-1 to 11-1 and FIGs. 8-2 to 11-2, the second material may be the same or different between these two or more films.

Of these structures, in the structures including a layer consisting of the first material as illustrated in FIGs. 6-1, 6-2, 7-1, 7-2, 10-1, and 10-2, the layer consisting of the first material may be regarded as a layer made of the organic thin film of the present invention (organic thin film consisting of the first material, free from the second material).

The average thickness of the electron injection layer 5 can be measured, for example, with a stylus profiler or a spectroscopic ellipsometer.

As described above, the first material which is an organic material having a pKa of 1 or greater is capable of extracting a proton (H⁺) from other materials. Thus, when the organic thin film of the present invention is formed adjacent to the oxide layer 4, a larger amount of the first material is preferably present on an oxide layer 4 side in order to sufficiently facilitate electron injection from the oxide layer 4. Thus, the electron injection layer 5 preferably has a concentration distribution in which the concentration of the first material diminishes from the oxide layer 4 side to an electron transport layer 10 side.

In an inverted organic electroluminescence device, an electron injection layer having such a concentration distribution may be formed, for example, by applying a solution containing the first material to the oxide layer 4 to form a coat and applying a solution containing the second material to the coat of the first material, but may be formed by any other method that can provide an electron injection layer having the above concentration distribution.

Also, when the organic thin film of the present invention is formed adjacent to the cathode 3, a larger amount of the first material is preferably present on a cathode 3 side in order to sufficiently facilitate electron injection from the cathode 3. Thus, the electron injection layer 5 preferably has a concentration distribution in which the concentration of the first material diminishes from the cathode 3 side to the electron transport layer 10 side.

In a conventional organic EL device, an electron injection layer having such a concentration distribution may be formed, for example, by applying a solution containing the second material to the electron transport layer 10 to form a coat and applying a solution containing the first material to the coat of the second material, but may be formed by any other method that can provide an electron injection layer having the above concentration distribution.

The concentration distribution may be measured by time-of-flight secondary ion mass spectrometry (TOF-SIMS), for example.

The inverted organic EL device may further include a layer of the first material on the oxide layer 4, and a layer containing the first material and the second material thereon in addition to the electron injection layer having the above concentration distribution. The conventional organic EL device may further include a layer of the second material on the electron transport layer 10, and a layer containing the first material and the second material thereon.

These layers can be formed by either application or evaporation.

As described above, the first material, which is an organic material having a pKa of 1 or greater, is capable of extracting protons (H⁺) from other materials, and suitably coordinates to the defective portion of the inorganic compound (oxide) to prevent reaction at the interface with oxygen or water entering from the outside. The organic thin film of the present invention can exhibit the effects without being formed adjacent to the cathode or oxide layer. In order to sufficiently obtain the effects of the organic thin film of the present invention, the organic thin film of the present invention is preferably formed adjacent to the cathode or oxide layer. The thus obtained films having a laminate structure, that is, a laminate film including an oxide layer and a layer of the organic thin film of the present invention formed adjacent to the oxide layer and a laminate film including a cathode and the layer of the organic thin film of the present invention formed adjacent to the cathode are also another aspect of the present invention.

When the organic EL device has a laminate structure including an oxide layer and a layer of the organic thin film of the present invention formed adjacent to the oxide layer between a cathode and an emitting layer or a laminate structure including a cathode and a layer of the organic thin film of the present invention formed adjacent to the cathode, the organic EL device can be said to include the organic thin film of the present invention or can be said to include the laminate film of the present invention. Such an organic EL device including the organic thin film or laminate film of the present invention between the cathode and the emitting layer is also another aspect of the present invention. The organic EL device including the organic thin film of the present invention or a laminate film including a cathode and a layer of the organic thin film of the present invention formed adjacent to the cathode is also another aspect of the present invention.

The organic EL device of the present invention may include a laminate film as illustrated in FIG. 8-1, 8-2, 9-1, 9-2, 11-1, or 11-2 as an electron injection layer. In other words, in a preferred embodiment of the organic EL device of the present invention, a laminate film including a film containing the first material and the second material and a film containing the second material is present between the cathode and the emitting layer.

In such a preferred embodiment of the organic EL device of the present invention, the organic EL device includes a layer containing the second material between the emitting layer and the film containing the first material and the second material, or the organic EL device includes a layer containing the second material between the cathode and the film containing the first material and the second material.

### "Electron transport material"

The material of the electron transport layer 10 can be any material that can be commonly used as a material of an electron transport layer.

Specific examples of the material of the electron transport layer 10 include phosphine oxide derivatives such as phenyl-dipyrenylphosphine oxide (POPy₂); pyridine derivatives such as tris-1,3,5-(3'-(pyridin-3"-yl)phenyl)benzene (TmPhPyB); quinoline derivatives such as (2-(3-(9-carbazolyl)phenyl)quinoline (mCQ)); pyrimidine derivatives such as 2-phenyl-4,6-bis(3,5-dipyridylphenyl)pyrimidine (BPyPPM); pyrazine derivatives; phenanthroline derivatives such as bathophenanthroline (BPhen); triazine derivatives such as 2,4-bis(4-biphenyl)-6-(4'-(2-pyridinyl)-4-biphenyl)-[1,3,5]triazine (MPT); triazole derivatives such as 3-phenyl-4-(1'-naphthyl)-5-phenyl-1,2,4-triazole (TAZ); oxazole derivatives; oxadiazole derivatives such as 2-(4-biphenyl)-5-(4-tert-butylphenyl-1,3,4-oxadiazole) (PBD); imidazole derivatives such as 2,2',2"-(1,3,5-benzenetriyl)-tris(1-phenyl-1-H-benzimidazole) (TPBI); aromatic tetracarboxylic anhydrides such as naphthalene and perylene; various metal complexes typified by bis[2-(2-hydroxyphenyl)benzothiazolato]zinc (Zn(BTZ)₂) and tris(8-hydroxyquinolinato)aluminum (Alq₃); organic silane derivatives typified by silole derivatives such as 2,5-bis(6'-(2',2"-bipyridyl))-1,1-dimethyl-3,4-diphenylsilole (PyPySPyPy); and boron-containing compounds disclosed in Japanese Patent Application No. 2012-228460, Japanese Patent Application No. 2015-503053, Japanese Patent Application No. 2015-053872, Japanese Patent Application No. 2015-081108, and Japanese Patent Application No. 2015-081109. One or two or more of these may be used.

Particularly preferred among the materials of the electron transport layer 10 are phosphine oxide derivatives such as POPy₂, metal complexes such as Alq₃, and pyridine derivatives such as TmPhPyB.

The organic EL device of the present invention may not include an electron transport layer containing the electron transport material as described above in the laminate structure, as long as the organic EL device functions like an organic EL device including a layer containing the second material formed from an emitting material or a host material to be used in an emitting layer.

The average thickness of the electron transport layer 10 is not limited, and is preferably 10 to 150 nm, more preferably 20 to 100 nm.

The average thickness of the electron transport layer 10 can be measured with a stylus profiler or a spectroscopic ellipsometer.

### "Emitting layer"

The emitting layer 6 may be made of any material that can be commonly used for the emitting layer 6 or may be made of a mixture of these materials. Specifically, for example, the emitting layer 6 may contain bis[2-(2-benzothiazolyl)phenolato]zinc(II) (Zn(BTZ)₂) and tris[1-phenylisoquinoline]iridium(III) (Ir(piq)₃).

The material of the emitting layer 6 may be a low-molecular compound or a high-molecular compound. The term "low-molecular material" as used herein refers to a material that is not a high-molecular material (polymer), and does not necessarily refer to a low molecular weight organic compound.

Examples of the high-molecular material of the emitting layer 6 include polyacetylene-based compounds such as trans-polyacetylene, cis-polyacetylene, poly(diphenylacetylene) (PDPA), and poly(alkylphenylacetylene) (PAPA); polyparaphenylenevinylene-based compounds such as poly(para-phenylenevinylene) (PPV), poly(2,5-dialkoxy-para-phenylenevinylene) (RO-PPV), cyano-substituted-poly(para-phenylenevinylene) (CN-PPV), poly(2-dimethyloctylsilyl-para-phenylenevinylene) (DMOS-PPV), and poly(2-methoxy-5-(2'-ethylhexoxy)-para-phenylenevinylene) (MEH-PPV); polythiophene-based compounds such as poly(3-alkylthiophene) (PAT) and poly(oxypropylene)triol (POPT); polyfluorene-based compounds such as poly(9,9-dialkylfluorene) (PDAF), poly(dioctylfluorene-alt-benzothiadiazole) (F8BT), α,ω-bis[N,N'-di(methylphenyl)aminophenyl]-poly[9,9-bis(2-ethylhexyl)fluorene-2,7-diyl] (PF2/6am4), and poly(9,9-dioctyl-2,7-divinylenefluorenyl-ortho-co(anthracene-9,10-diyl); polyparaphenylene-based compounds such as poly(para-phenylene) (PPP) and poly(1,5-dialkoxy-para-phenylene) (RO-PPP); polycarbazole-based compounds such as poly(N-vinylcarbazole) (PVK); polysilane-based compounds such as poly(methylphenylsilane) (PMPS), poly(naphthylphenylsilane) (PNPS), and poly(biphenylylphenylsilane) (PBPS); and boron compound-based high-molecular materials disclosed in Japanese Patent Application No. 2010-230995 and Japanese Patent Application No. 2011-6457.

Examples of the low-molecular material of the emitting layer 6 include various metal complexes such as a tridentate iridium complex having 2,2'-bipyridine-4,4'-dicarboxylic acid as a ligand, fac-tris(2-phenylpyridine)iridium (Ir(ppy)₃), fac-tris(3-methyl-2-phenylpyridinato-N,C2'-)iridium(III) (Ir(mppy)₃), 8-hydroxyquinoline aluminum (Alq₃), tris(4-methyl-8-quinolinolato)aluminum(III) (Almq₃), 8-hydroxyquinoline zinc (Znq₂), (1,10-phenanthroline)-tris-(4,4,4-trifluoro-1-(2-thienyl)-butane-1,3-dionate)europium(III) (Eu(TTA)₃(phen)), and 2,3,7,8,12,13,17,18-octaethyl-21H,23H-porphinplatinum(II); benzene-based compounds such as distyrylbenzene (DSB) and diaminodistyrylbenzene (DADSB); naphthalene-based compounds such as naphthalene and Nile red; phenanthrene-based compounds such as phenanthrene; chrysene-based compounds such as chrysene and 6-nitrochrysene; perylene-based compounds such as perylene and N,N'-bis(2,5-di-t-butylphenyl)-3,4,9,10-perylene-di-carboxyimide (BPPC); coronene-based compounds such as coronene; anthracene-based compounds such as anthracene, bisstyrylanthracene, and (9,10-bis(4-(9H-carbazol-9-yl)-2,6-dimethylphenyl))-9,10-diboraanthracene (CzDBA) of the below-described formula (27); pyrene-based compounds such as pyrene; pyran-based compounds such as 4-(dicyanomethylene)-2-methyl-6-(para-dimethylaminostyryl)-4H-pyran (DCM); acridine-based compounds such as acridine; stilbene-based compounds such as stilbene; thiophene-based compounds such as 2,5-dibenzoxazolethiophene; benzoxazole-based compounds such as benzoxazole; benzimidazole-based compounds such as benzimidazole; benzothiazole-based compounds such as 2,2'-(para-phenylenedivinylene)-bisbenzothiazole; butadiene-based compounds such as bistyryl(1,4-diphenyl-1,3-butadiene) and tetraphenylbutadiene; naphthalimide-based compounds such as naphthalimide; coumarin-based compounds such as coumarin; perynone-based compounds such as perynone; oxadiazole-based compounds such as oxadiazole; aldazine-based compounds; cyclopentadiene-based compounds such as 1,2,3,4,5-pentaphenyl-1,3-cyclopentadiene (PPCP); quinacridone-based compounds such as quinacridone and quinacridone red; pyridine-based compounds such as pyrrolopyridine and thiadiazolopyridine; triazine-based compounds such as 2,4-diphenyl-6-bis((12-phenylindolo) [2,3a]carbazol-11-yl)-1,3,5-triazine (DIC-TRZ); spiro compounds such as 2,2',7,7'-tetraphenyl-9,9'-spirobifluorene; metallic or non-metallic phthalocyanine-based compounds such as phthalocyanine (H₂Pc) and copper phthalocyanine; and boron compound materials disclosed in JP 2009-155325 A, JP 2011-184430 A, and Japanese Patent Application No. 2011-6458.

Examples of the host material of the emitting layer include a carbazole compound such as 4,4'-bis(9H-carbazol-9-yl)biphenyl (CPB), a silicon compound, a phenanthroline compound, and a triphenylene compound.

The average thickness of the emitting layer 6 is not limited, and is preferably 10 to 150 nm, more preferably 20 to 100 nm.

The average thickness of the emitting layer 6 may be measured with a stylus profiler, or may be measured during formation of the emitting layer 6 with a quartz crystal film thickness monitor.

### "Hole transport layer"

Examples of a hole transport organic material of the hole transport layer 7 include p-type polymer materials (organic polymers) and p-type low-molecular materials. These materials may be used alone or in combination.

Specific examples of the material of the hole transport layer 7 include N,N'-di(1-naphthyl)-N,N'-diphenyl-1,1'-biphenyl-4,4'-diamine (a-NPD), N4,N4'-bis(dibenzo[b,d]thiophen-4-yl)-N4,N4'-diphenylbiphenyl-4,4'-diamine (DBTPB), N3,N3‴-bis(dibenzo[b,d]thiophene-4-yl)-N3,N3'''-diphenyl-[1,1':2',1":2",1'''-quaterphenyl)-3,3‴-diamine (4DBTP3Q), polyarylamine, fluorene-arylamine copolymers, fluorene-bithiophene copolymers, poly(N-vinylcarbazole), polyvinylpyrene, polyvinylanthracene, polythiophene, polyalkylthiophene, polyhexylthiophene, poly(p-phenylenevinylene), polythienylenevinylene, pyrene formaldehyde resin, ethylcarbazole formaldehyde resin, and derivatives thereof. These materials of the hole transport layer 7 may be mixed with other compounds. An example of a mixture containing polythiophene used as the material of the hole transport layer 7 is poly(3,4-ethylenedioxythiophene/styrenesulfonate) (PEDOT/PSS).

As described above, the hole injection layer can relatively easily inject holes into a material used for an emitting layer. Thus, even a device including a hole transport layer made of a material used for the emitting layer can be driven with a low voltage. Thus, the organic EL device of the present invention may not include a hole transport layer made of a hole transport material.

The average thickness of the hole transport layer 7 is not limited, and is preferably 10 to 150 nm, more preferably 20 to 100 nm.

The average thickness of the hole transport layer 7, for example, can be measured with a stylus profiler or a spectroscopic ellipsometer.

### "Hole injection layer"

The hole injection layer 8 may be made of an inorganic material or an organic material. Since an inorganic material is more stable than an organic material, the use of an inorganic material imparts higher resistance to oxygen and water than the use of an organic material.

Non-limiting examples of the inorganic material include metal oxides such as vanadium oxide (V₂O₅), molybdenum oxide (MoO₃), and ruthenium oxide (RuO₂). These may be used alone or in combination of two or more of these.

Examples of the organic material include dipyrazino(2,3-f:2',3'-h]quinoxaline-2,3,6,7,10,11-hexacarbonitrile (HAT-CN), 2,3,5,6-tetrafluoro-7,7,8,8-tetracyano-quinodimethane (F4-TCNQ), fullerene, and a hole injection material "Clevios HIL1.3N" available from Heraeus.

The average thickness of the hole injection layer 8 is not limited, and is preferably 1 to 1000 nm, more preferably 5 to 50 nm.

The average thickness of the hole injection layer 8 can be measured with a quartz crystal thickness monitor or a stylus profiler during film formation.

### "Anode"

Examples of a material of the anode 9 include ITO, IZO, Au, Pt, Ag, Cu, Al, and alloys containing any of these. Preferred among these materials of the anode 9 are ITO, IZO, Au, Ag, and Al.

The average thickness of the anode 9 is not limited, and is preferably 10 to 1000 nm, more preferably 30 to 150 nm. Even when the anode 9 is made of an opaque material, the anode 9 having an average thickness of about 10 to 30 nm can be used as a transparent anode for a top emission organic EL device.

The average thickness of the anode 9 can be measured with a quartz crystal film thickness monitor during formation of the anode 9.

### "Sealing"

The organic EL device 1 illustrated in FIG. 1 may be sealed, as required.

For example, the organic EL device 1 illustrated in FIG. 1 may be sealed in a sealing container (not shown) having a recessed space for containing the organic EL device 1 by bonding the edge of the sealing container and the substrate 2 with an adhesive. Alternatively, the organic EL device 1 may be contained in the sealing container and sealed by filling the container with a sealing material made of an ultraviolet (UV) curable resin, for example. Also, for example, the organic EL device 1 illustrated in FIG. 1 may be sealed using sealing members including a plate member (not shown) placed on the anode 9 and a frame member (not shown) placed along an anode 9-side edge of the plate member, with the plate member being bonded to the frame member and the frame member being bonded to the substrate 2 with an adhesive.

When the organic EL device 1 is sealed using the sealing container or the sealing members, a desiccant to absorb moisture may be placed in the sealing container or in the sealing members. Also, the sealing container or the sealing members may be made of a moisture absorbing material. There may be a space in the sealing container or in the sealing members after sealing.

Examples of a material of the sealing container or the sealing members used to seal the organic EL device 1 illustrated in FIG. 1 include a resin material and a glass material. Examples of the resin material and glass material of the sealing container or sealing members include those mentioned as the material of the substrate 2.

When the organic EL device 1 of the present embodiment includes, as an organic thin film, an electron injection layer containing the first material which is a hexahydropyrimidopyrimidine compound having a structure of the formula (1) and the second material which is the boron-containing compound of the formula (4), the organic EL device 1 has better durability than an organic EL device including an electron injection layer made of an alkali metal, which is unstable in the air. A sealing container or sealing members having a water vapor transmission rate of about 10⁻⁴ to 10⁻³ g/m²/day can sufficiently prevent degradation of the organic EL device 1. Thus, a resin material having a water vapor transmission rate of about 10⁻³ g/m²/day or less can be used as the material of the sealing container or sealing members, and the organic EL device 1 with excellent flexibility can be obtained.

### "Method for producing organic EL device"

Next, a method for producing the organic EL device 1 illustrated in FIG. 1 will be described as an example of the method for producing an organic EL device of the present invention.

In order to produce the organic EL device 1 illustrated in FIG. 1, first, the cathode 3 is formed on the substrate 2.

The cathode 3 may be formed by, for example, a sputtering method, a vacuum evaporation method, a sol-gel method, a spray pyrolysis deposition (SPD) method, an atomic layer deposition (ALD) method, a vapor deposition method, or a liquid phase deposition method. The cathode 3 may be formed by bonding metal foil.

Next, the inorganic oxide layer 4 is formed on the cathode 3.

The oxide layer 4 is formed by, for example, a spray pyrolysis deposition method, a sol-gel method, a sputtering method, or a vacuum evaporation method. The resulting oxide layer 4 sometimes has an irregular surface, not a smooth surface.

Next, the electron injection layer 5 is formed on the oxide layer 4.

The electron injection layer 5 can be formed by the above-described method for producing an organic thin film.

Next, the electron transport layer 10, the emitting layer 6, and the hole transport layer 7 are formed on the electron injection layer 5 in the stated order.

The electron transport layer 10, the emitting layer 6, and the hole transport layer 7 may be formed by any method, and any of known various forming methods can be appropriately used depending on the properties of the materials of the electron transport layer 10, the emitting layer 6, and the hole transport layer 7.

Specific examples of the forming methods of each of the electron transport layer 10, the emitting layer 6, and the hole transport layer 7 include application of a solution of an organic compound that forms the electron transport layer 10, a solution of an organic compound that forms the emitting layer 6, or a solution of an organic compound that forms the hole transport layer 7; vacuum evaporation; and evaporative spray deposition from ultra-dilute solution (ESDUS). In particular, the electron transport layer 10, the emitting layer 6, and the hole transport layer 7 are preferably formed by application. When the organic compound that forms the electron transport layer 10, the organic compound that forms the emitting layer 6, or the organic compound that forms the hole transport layer 7 is less soluble in a solvent, vacuum evaporation or ESDUS is preferably used.

When the electron transport layer 10, the emitting layer 6, and the hole transport layer 7 are formed by application, the solution of an organic compound that forms the electron transport layer 10, the solution of an organic compound that forms the emitting layer 6, and the solution of an organic compound that forms the hole transport layer 7 are prepared respectively by dissolving the organic compound that forms the electron transport layer 10, the organic compound that forms the emitting layer 6, and the organic compound that forms the hole transport layer 7 in respective solvents.

Preferred examples of the solvent used to dissolve the organic compound that forms the electron transport layer 10, the solvent used to dissolve the organic compound that forms the emitting layer 6, and the solvent used to dissolve the organic compound that forms the hole transport layer 7 include aromatic hydrocarbon solvents such as xylene, toluene, cyclohexylbenzene, dihydrobenzofuran, trimethylbenzene, and tetramethylbenzene; aromatic heterocyclic compound solvents such as pyridine, pyrazine, furan, pyrrole, thiophene, and methylpyrrolidone; and aliphatic hydrocarbon solvents such as hexane, pentane, heptane, and cyclohexane. These may be used alone or as a mixture.

Examples of the method for applying the solution of an organic compound that forms the electron transport layer 10, the solution of an organic compound that forms the emitting layer 6, or the solution of an organic compound that forms the hole transport layer 7 include various application methods such as a spin coating method, a casting method, a micro gravure coating method, a gravure coating method, a bar coating method, a roll coating method, a wire bar coating method, a dip coating method, a spray coating method, a screen printing method, a flexographic printing method, an offset printing method, and an inkjet printing method. Preferred among these methods are a spin coating method and a slit coating method because they can easily control the film thickness.

Next, the hole injection layer 8 is formed on the hole transport layer 7, and the anode 9 is formed on the layer 8.

When the hole injection layer 8 is made of an inorganic material, the hole injection layer 8 can be formed in the same manner as for the oxide layer 4, for example.

When the hole transport layer 9 is made of an organic material, the hole injection layer 8 can be formed in the same manner as for the electron transport layer 10, the emitting layer 6, and the hole transport layer 7, for example.

The anode 9 can be formed, for example, in the same manner as for the cathode 3.

The organic EL device 1 illustrated in FIG. 1 is obtained through the above steps.

### "Sealing method"

The organic EL device 1 illustrated in FIG. 1 can be sealed by the method commonly used to seal organic EL devices.

Since the organic EL device 1 of the present embodiment includes the electron injection layer 5 which is an organic thin film that contains the first material which is an organic material having a pKa of 1 or greater and the second material which transports electrons, the first material extracts a proton (H⁺) from the second material, so that a negative charge is generated and an excellent electron injection property is obtained. Thus, electrons are injected and transported from the cathode 3 to the emitting layer 6 at a high speed, and the organic EL device 1 is driven with a low voltage.

In another embodiment of the organic EL device of the present invention, the organic EL device 1 is a device in which the organic thin film containing the first material which is an organic material having a pKa of 1 or greater and the second material which transports electrons is a laminate film, and the layer formed from the second material and the electron injection layer formed from the first material are different layers. In such an embodiment of the organic EL device 1, electrons are injected and transported from the cathode 3 to the emitting layer 6 at a high speed, and the organic EL device 1 is driven with a low voltage.

### "Other examples"

The organic EL device of the present invention is not limited to the organic EL devices described in the above embodiments.

Specifically, in the above embodiments, the device was described with the case where an organic thin film serves as an electron injection layer as an example. The organic EL device of the present invention has only to include the organic thin film between the cathode and the emitting layer. Thus, the organic thin film may not be an electron injection layer, and may be formed as a layer serving as both an electron injection layer and an electron transport layer or may be formed as an electron transport layer.

In the organic EL device 1 illustrated in FIG. 1, the inorganic oxide layer 4, the electron transport layer 10, the hole transport layer 7, and the hole injection layer 8 may be formed as required and may not be formed.

The cathode 3, the oxide layer 4, the electron injection layer 5, the electron transport layer 10, the emitting layer 6, the hole transport layer 7, the hole injection layer 8, and the anode 9 each may be a single layer or may include two or more layers.

The organic EL device 1 illustrated in FIG. 1 may include a different layer between any two of the layers illustrated in FIG. 1. Specifically, for example, in order to enhance the properties of the organic EL device, the device may include an electron blocking layer or other layers, as required.

In the above embodiments, the organic EL device was described with an inverted organic EL device including the cathode 3 between the substrate 2 and the emitting layer 6, as an example. The organic EL device may be a conventional organic EL device including an anode between a substrate and an emitting layer.

### "Display device, lighting system, organic thin film solar cell, photoelectric transducer, thin film transistor"

The organic EL device of the present invention is capable of changing the color of light by suitably selecting a material of an emitting layer or the like, and is also capable of providing a desired color of light by using a color filter or the like together therewith. Thus, the organic EL device of the present invention can be suitably used as an emitting portion of a display device or a lighting system.

The display device of the present invention includes the organic EL device of the present invention that includes an organic thin film between a cathode and an emitting layer and that is highly productive and is driven with a low voltage. Thus, the display device of the present invention is suitable as a display device.

The lighting system of the present invention includes the organic EL device of the present invention that is produced in a high yield and is driven with a low voltage. Thus, the lighting system of the present invention is suitable as a lighting system.

The present invention is not limited to the above-described embodiments, and the organic thin film of the present invention can be used, for example, for devices such as organic thin film solar cells, photoelectric transducers, and thin film transistors.

The organic thin film solar cell or photoelectric transducer of the present invention includes an organic thin film. For example, when the organic thin film is used for an electron injection layer of the organic thin film solar cell or the photoelectric transducer, the first material of the organic thin film extracts a proton (H⁺) from the second material, so that a negative charge is generated. Thus, electrons are transported at a high speed, and high power generation efficiency can be obtained. Thus, the organic thin film solar cell and the photoelectric transducer each including the organic thin film of the present invention are preferred. The thin film transistor of the present invention includes an organic thin film.

For example, when a channel layer of the thin film transistor is formed from the organic thin film, the channel layer has high electron mobility.

When the organic thin film is formed on an electrode, a reduction in contact resistance can be expected.

Thus, the organic thin film of the present invention is suitable as materials of organic thin film solar cells, photoelectric transducers, and thin film transistors. Thus, a hexahydropyrimidopyrimidine compound having a structure of the formula (1) constituting the organic thin film is suitable as the materials of these. The organic thin film solar cell material, photoelectric transducer material, or thin film transistor material, containing a hexahydropyrimidopyrimidine compound having a structure of the formula (1), is also another aspect of the present invention.

### EXAMPLES

The present invention is described in more detail with reference to examples below, but the present invention is not limited to these examples. Herein, "part(s)" means "part(s) by weight", and "%" means "mol%" unless otherwise stated.

### Synthesis Example 1

A compound of the following formula (2-9) was synthesized by the method disclosed in Macromolecules, 45(5), pp. 2249-2256, 2012.

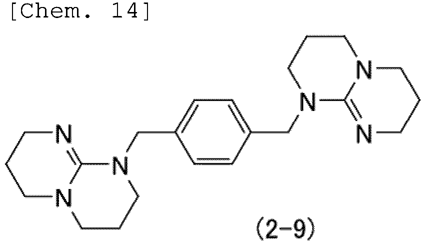

### Synthesis Example 2 (not according to the invention)

A 200-mL three-necked flask was charged with *rac-*BINAP (747 mg) and toluene (67 mL), which were heated in a nitrogen atmosphere at 90°C for dissolution. The mixture was cooled to room temperature, palladium acetate (180 mg) was added thereto, and the contents were stirred at room temperature for one hour. To the mixture were added 2,6-dibromopyridine (4.74 g), 1,3,4,6,7,8-hexahydro-2H-pyrimido[1,2-a]pyrimidine (6.13 g), and KOtBu (6.28 g), and the contents were heated with stirring at 90°C overnight. The mixture was cooled to room temperature, diethyl ether was added thereto to precipitate solids, which were filtered off, and the filtrate was concentrated. To the resulting residue was added acetone to precipitate solids, which were collected by filtration. Thus, a compound of the following formula (2-2) (3.7 g, 52.5%) was obtained.

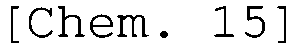

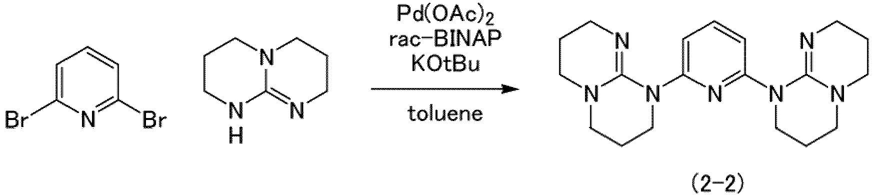

### Synthesis Example 3 (not according to the invention)

A 300-mL three-necked flask containing toluene (100 mL) was charged with *rac*-BINAP (369 mg) under nitrogen stream at room temperature, and the solids were dissolved by heating with stirring in an oil bath at 60°C. The mixture was cooled to room temperature, Pd(OAc)₂ (98 mg) and 6-bromo-2,2'-bipyridine (5.00 g) were added thereto, and the contents were stirred in the oil bath at 60°C again for 20 minutes. The mixture was cooled to room temperature, 1,3,4,6,7,8-hexahydro-2H-pyrimido[1,2-a]pyrimidine (3.26 g) and KOtBu (5.97 g) were added thereto, and the contents were stirred in the oil bath at 90°C for three hours. The mixture was cooled to room temperature, diethyl ether was added thereto, and insoluble matters were filtered off. The filtrate was concentrated under reduced pressure, and ethyl acetate (15 mL) was added to the residue. The mixture was subjected to ultrasonication to obtain solids, which were collected by filtration and dried under reduced pressure overnight. The resulting solids were recrystallized from ethyl acetate, followed by distillation. Thus, a compound of the following formula (2-3) was obtained as a white solid (1.20 g, 19.2%).

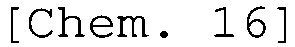

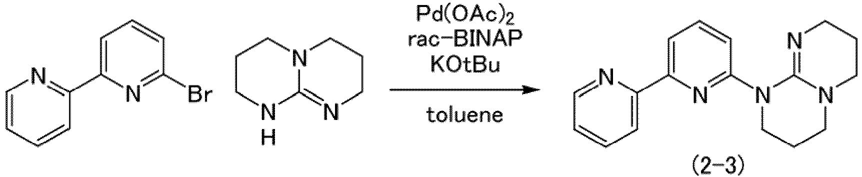

### Synthesis Example 4

A three-necked flask containing toluene (97 mL) was charged with *rac*-BINAP (357 mg) under nitrogen stream at room temperature, and the solids were dissolved by heating with stirring in an oil bath at 60°C. The mixture was cooled to room temperature, palladium acetate (86 mg) and 2-bromo-1,10-phenanthroline (4.40 g) were added thereto, and the contents were stirred in the oil bath at 60°C again for 30 minutes. The mixture was cooled to room temperature, 1,3,4,6,7,8-hexahydro-2H-pyrimido[1,2-a]pyrimidine (2.64 g) and KOtBu (4.72 g) were added thereto, and the contents were stirred in the oil bath at 90°C for four hours. The mixture was cooled to room temperature, diethyl ether (150 mL) was added thereto, and insoluble matters were filtered off. The filtrate was concentrated under reduced pressure, and ethyl acetate (20 mL) was added to the residue. The precipitate was collected by filtration and dried under reduced pressure. To the solids was added acetone (100 mL), and the solids were washed with the acetone with heating and collected by filtration. The resulting solids were purified by sublimation. Thus, the compound of the following formula (2-4) was obtained as a white solid (2.21 g, 25.6%).

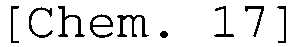

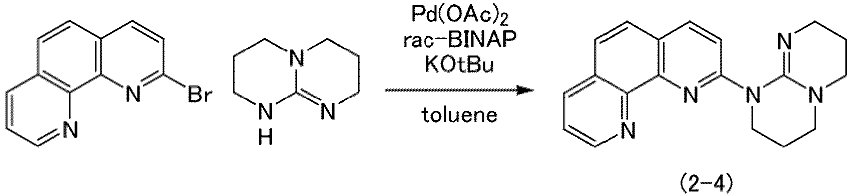

### Synthesis Example 5 (not according to the invention)

A flask containing toluene (80 mL) was charged with *rac*-BINAP (301 mg) under nitrogen stream at room temperature, and the solids were dissolved by heating with stirring in an oil bath at 60°C. The mixture was cooled to room temperature, palladium acetate (78 mg) and 6,6'-dibromo-2,2'-bipyridine (5.00 g) were added thereto, and the contents were stirred in the oil bath at 60°C again for 20 minutes. The mixture was cooled to room temperature, 1,3,4,6,7,8-hexahydro-2H-pyrimido[1,2-a]pyrimidine (4.90 g) and KOtBu (8.95 g) were added thereto, and the contents were stirred in the oil bath at 110°C for six hours. The mixture was cooled to room temperature, diethyl ether was added thereto, and insoluble matters were collected by filtration. The insoluble matters were rinsed with a chloroform-ethyl acetate solvent mixture, and the solvent mixture used for rinse was added to the filtrate. The filtrate was concentrated under reduced pressure. To the residue was added methanol to obtain a precipitate, which was collected by filtration. The resulting solids (3.60 g) were washed with methanol (50 mL) with heating and cooled to room temperature to precipitate solids, which were collected by filtration again. The resulting solids (2.20 g) were purified by sublimation. Thus, a compound of the following formula (2-5) was obtained as a white solid (1.45 g, 21.0%).

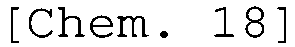

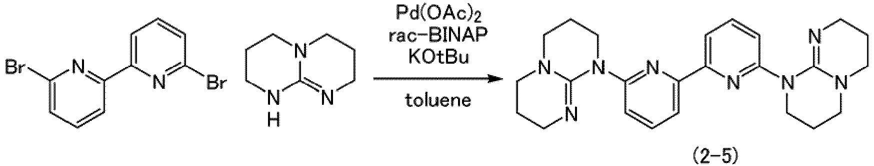

### Synthesis Example 6

A recovery flask containing a mixture of 2-chloro-4,6-diphenyl-1,3,5-triazine (5.00 g) and toluene (95 mL) was charged with 1,3,4,6,7,8-hexahydro-2H-pyrimido[1,2-a]pyrimidine (5.72 g) at room temperature, and the contents were stirred in an oil bath at 100°C for three hours. The mixture was cooled to room temperature, insoluble matters were separated from the mixture by filtration, and the filtrate was concentrated under reduced pressure. To the residue was added chloroform (25 mL), the solution was subjected to ultrasonication to precipitate solids, which were collected by filtration and dried under reduced pressure. Thus, a compound of the following formula (2-6) was obtained as a white solid (5.90 g, 85.2%).

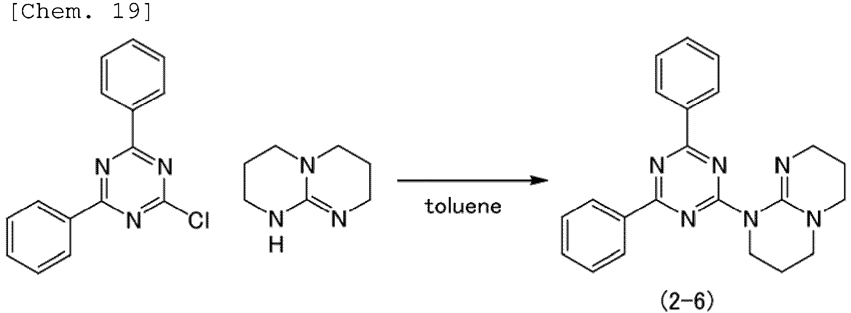

### Synthesis Example 7 (not according to the invention)

A 100-mL reaction vessel was charged with *rac*-BINAP (0.213 g, 0.342 mmol) and toluene (20 mL), which were heated to 70°C for complete dissolution. Thereafter, palladium acetate (51 mg, 0.228 mmol) was added thereto, and the contents were stirred while cooling to room temperature. To the mixture were added 2,4,6-tribromopyridine (1.2 g, 3.8 mmol), KOtBu (1.8 g, 16.0 mmol), and 1,3,4,6,7,8-hexahydro-2H-pyrimido[1,2-a]pyrimidine (1.9 g, 13.7 mmol), and the contents were heated with stirring at 100°C for 14 hours. The reaction solution was cooled to room temperature and filtered with celite, and the filtrate was concentrated. Thus, a compound (2.2 g) of the following formula (2-11) was obtained.

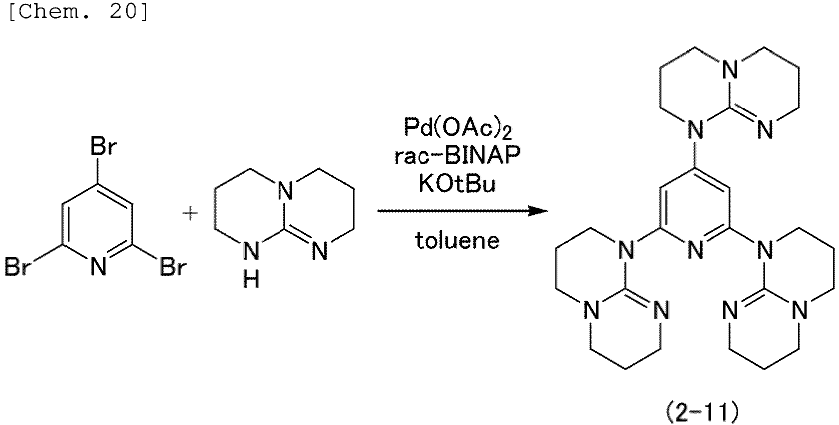

### Synthesis Example 8

A 200-mL recovery flask was charged with *rac*-BINAP (0.238 g, 0.383 mmol) and dehydrated toluene (55 mL), which were heated at 90°C for dissolution. The mixture was cooled to room temperature, palladium acetate (0.054 g, 0.24 mmol) was added thereto, and the reaction vessel was purged with argon. After one hour, 2-bromo-9,9'-spirobi[9H-fluorene] (2.18 g, 5.52 mmol), 1,3,4,6,7,8-hexahydro-2H-pyrimido[1,2-a]pyrimidine (0.957 g, 6.88 mmol), and potassium tert-butoxide (0.936 g, 8.35 mmol) were added thereto, the reaction vessel was purged with argon again, and the contents were heated with stirring in an oil bath at 90°C. After 2.25 hours, the mixture was cooled to room temperature, diethyl ether (220 mL) was added thereto, followed by stirring for a while to precipitate solids, which were collected by suction filtration and washed with diethyl ether. From the filtrate, the diethyl ether was distilled off under reduced pressure, and the remaining toluene layer was washed with water (55 mL). The organic layer#was distilled off under reduced pressure, and the resulting residue was purified by silica gel column chromatography. Thus, 2.13 g of a compound of the following formula (2-30) (4.69 mmol, yield 85%) was obtained.

The resulting compound of the formula (2-30) was combined with a separately synthesized compound of the formula (2-30) to a combined amount of 2.31 g. 2.31 g of the copmpound of the following formula (2-30) were dissolved in chloroform, the solvent was distilled off under reduced pressure, and the residue was suspended in hexane (50 mL), followed by stirring for 30 minutes to obtain solids, which were collected by suction filtration and washed with hexane. Thus, 1.78 g of the compound of the following formula (2-30) was obtained with a purity of 99.2%.

The following describes the result of ¹H-NMR analysis of the compound.
¹H-NMR (500 MHz CDCl₃): δ7.81 (d, 2H, J = 7.5 Hz), 7.74 (t, 2H, J = 8.0 Hz), 7.41 (dd, 1H, J = 1.5, 8.5 Hz), 7.36-7.29 (m, 3H),7.09 (t, 2H, J = 8.0 Hz), 7.03 (t, 1H, J = 7.5 Hz), 6.76 (d, 1H, J = 7.5 Hz), 6.66 (d, 1H, J = 1.5 Hz), 6.44 (d, 1H, J = 1.5 Hz), 3.32 (t, 2H, J = 5.5 Hz), 3.26 (t, 2H, J = 5.5 Hz), 3.14 (t, 2H, J = 6.0 Hz), 3.11 (t, 2H, J = 6.5 Hz), 1.92 (quin, 2H, J = 6.0 Hz), 1.80 (quin, 2H, J = 6.0 Hz).

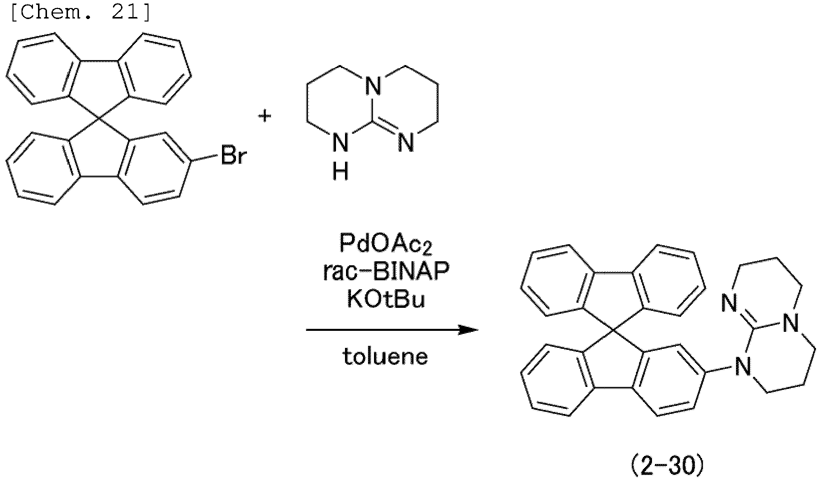

### Synthesis Example 9

A 100-mL recovery flask was charged with *rac*-BINAP (0.138 g, 0.221 mmol) and dehydrated toluene (35 mL), which were heated at 90°C for dissolution. The mixture was cooled to room temperature, palladium acetate (0.0331 g, 0.148 mmol) was added thereto, and the reaction vessel was purged with argon. After one hour, 2,7-dibromo-9,9'-spirobi[9H-fluorene] (0.7 g, 1.48 mmol), 1,3,4,6,7,8-hexahydro-2H-pyrimido[1,2-a]pyrimidine (0.452 g, 3.25 mmol), and potassium tert-butoxide (0.431 g, 3.84 mmol) were added thereto, the reaction vessel was purged with argon again, and the contents were heated with stirring in an oil bath at 90°C. After 3.25 hours, the mixture was cooled to room temperature, diethyl ether (100 mL) was added thereto, followed by stirring for a while to precipitate solids, which were collected by suction filtration and washed with diethyl ether. The filtrate was subjected to back extraction with water (35 mL), and the aqueous layer was distilled off#under reduced pressure to obtain 1.09 g of solids.

The solids were suspended in acetone, the suspension was stirred at room temperature for two hours to obtain solids, which were collected by suction filtration and washed with acetone. This operation was performed twice. Thus, 0.520 g of a compound of the following formula (2-31) was obtained as a pale yellow solid (0.88 mmol, yield 60%).

The following describes the result of ¹H-NMR analysis of the compound.
¹H-NMR (500 MHz CDCl₃): δ7.80 (d, 2H, J = 8.0 Hz), 7.70 (d, 2H, J = 8.0 Hz), 7.44 (d, 2H, J = 6.5 Hz), 7.34 (t, 2H, J = 7.5 Hz), 7.10 (t, 2H, J = 7.5 Hz), 6.80 (d, 2H, J = 7.5 Hz), 6.39 (d, 2H, J = 1.5 Hz), 3.38-3.33 (m, 4H), 3.28-3.25 (m, 4H), 3.23-3.14 (m, 8H), 1.98-1.93 (m, 4H), 1.87-1.81 (m, 4H) .

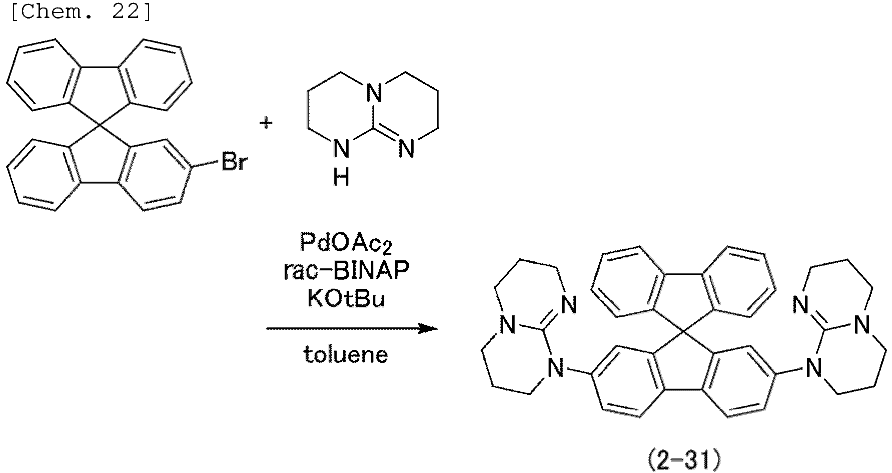

### Synthesis Example 10

A 500-mL recovery flask was charged with *rac*-BINAP (1.21 g, 1.95 mmol) and dehydrated toluene (350 mL), which were heated at 90°C for dissolution. The mixture was cooled to room temperature, palladium acetate (0.345 g, 1.54 mmol) was added thereto, and the reaction vessel was purged with argon. After 55 minutes, 9,10-dibromoanthracene (5.01 g, 14.9 mmol), 1,3,4,6,7,8-hexahydro-2H-pyrimido[1,2-a]pyrimidine (4.45 g, 32.0 mmol), and potassium tert-butoxide (4.26 g, 37.9 mmol) were added thereto, the reaction vessel was purged with argon again, and the contents were heated with stirring in an oil bath at 90°C. After 140 minutes, the mixture was cooled to room temperature, diethyl ether (1 L) was added thereto, followed by stirring for a while to precipitate solids, which were collected by suction filtration and washed with diethyl ether. From the filtrate, the diethyl ether was distilled off under reduced pressure, the toluene layer was subjected to back extraction with water (150 mL), and the aqueous layer was distilled off under reduced pressure to obtain 3.77 g of orange solids.

The solids were suspended in acetone (50 mL), the suspension was stirred at room temperature overnight to obtain solids, which were collected by suction filtration and washed with acetone to obtain 1.0 g of yellow solids.

The yellow solids were dissolved in chloroform (41 mL) at 60°C, and thereafter, hexane (48 mL) was gradually added thereto. After confirmation of the precipitation of solids, the mixture was cooled in a freezer overnight to obtain the solids, which were collected by suction filtration and washed with hexane. Thus, 0.657 g of a compound of the following formula (2-32) was obtained as a yellow solid (1.45 mmol, yield 10%).

The following describes the result of ¹H-NMR analysis of the compound.
¹H-NMR (500 MHz CDCl₃): δ8.03 (q, 4H, J = 3.0 Hz), 7.43 (q, 4H, J = 3.0 Hz), 3.59 (t, 4H, J = 5.5 Hz), 3.48 (t, 4H, J = 5.5 Hz), 3.36 (t, 4H, J = 5.5 Hz), 3.16 (t, 4H, J = 5.5 Hz), 2.29 (quin, 4H, J = 6.0 Hz), 1.86 (quin, 4H, J = 6.0 Hz).

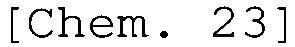

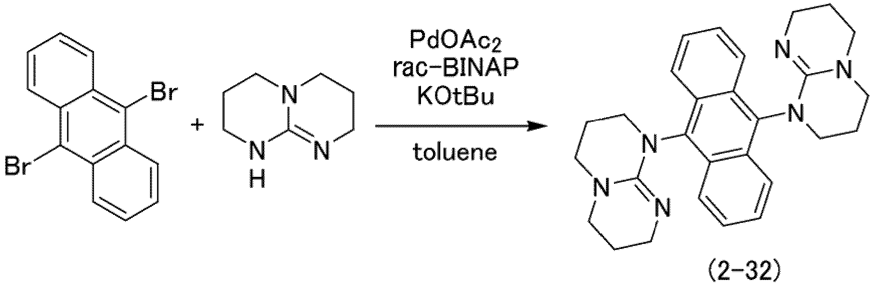

### Synthesis Example 11

A 100-mL recovery flask was charged with *rac*-BINAP (0.407 g, 0.653 mmol) and dehydrated toluene (60 mL), which were heated at 90°C for dissolution. The mixture was cooled to room temperature, palladium acetate (0.124 g, 0.551 mmol) was added thereto, and the reaction vessel was purged with argon. After 30 minutes, 2,2'-dibromo-9,9'-spirobi[9H-fluorene] (2.32 g, 4.90 mmol), 1,3,4,6,7,8-hexahydro-2H-pyrimido[1,2-a]pyrimidine (1.47 g, 10.6 mmol), and potassium tert-butoxide (1.38 g, 12.3 mmol) were added thereto, the reaction vessel was purged with argon again, and the contents were heated with stirring in an oil bath at 90°C. After 21.25 hours, the mixture was cooled to room temperature, diethyl ether (300 mL) was added thereto, followed by stirring for a while to precipitate solids, which were collected by suction filtration and washed with diethyl ether. The filtrate was subjected to back extraction with water (60 mL), the aqueous layer was distilled off under reduced pressure, and the residue was purified by column chromatography. Thus, 1.54 g of a yellow amorphous compound of the following formula (2-33) was obtained (2.61 mmol, yield 53%).

The following describes the result of ¹H-NMR analysis of the compound.
¹H-NMR (500 MHz CDCl₃): δ7.70 (dd, 4H, J = 8.0, 14.0 Hz), 7.37 (dd, 2H, J = 2.0, 8.0 Hz), 7.29 (t, 2H, J = 7.5 Hz), 7.01 (dd, 2H, J = 1.0, 7.5 Hz), 6.69 (d, 2H, J = 8.0 Hz), 6.47 (d, 2H, J = 2.0 Hz), 3.37-3.28 (m, 4H), 3.24 (t, 4H, J = 5.5 Hz), 3.13 (t, 4H, J = 6.0 Hz), 3.09 (t, 4H, J = 6.5 Hz), 1.91 (quin, 4H, J = 6.0 Hz), 1.78 (quin, 4H, J = 6.0 Hz).

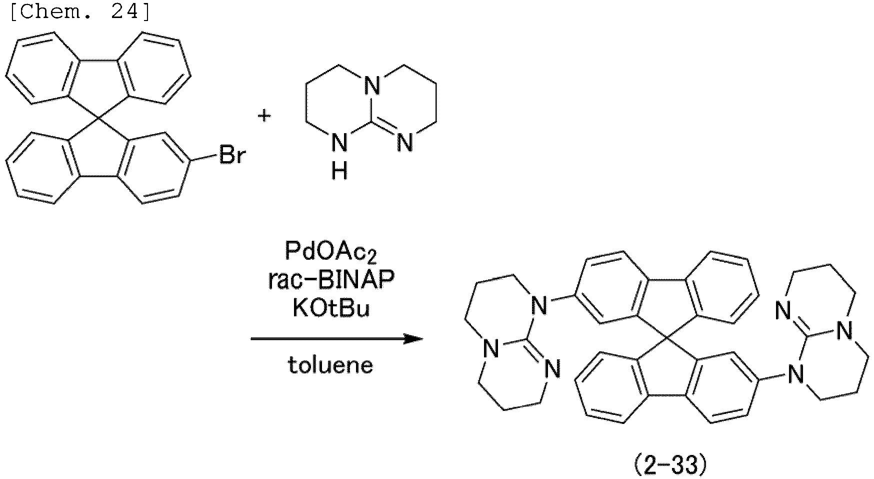

### Synthesis Example 12

A 500-mL recovery flask was charged with *rac*-BINAP (1.60 g, 2.57 mmol) and dehydrated toluene (350 mL), which were heated at 90°C for dissolution. The mixture was cooled to room temperature, palladium acetate (0.395 g, 1.76 mmol) was added thereto, and the reaction vessel was purged with argon. After 30 minutes, 2,2',7,7'-dibromo-9,9'-spirobi[9H-fluorene] (4.43 g, 7.00 mmol), 1,3,4,6,7,8-hexahydro-2H-pyrimido[1,2-a]pyrimidine (4.06 g, 29.1 mmol), and potassium tert-butoxide (4.08 g, 36.3 mmol) were added thereto, the reaction vessel was purged with argon again, and the contents were heated with stirring in an oil bath at 90°C. After 22.75 hours, the mixture was cooled to room temperature, diethyl ether (1.05 L) was added thereto, followed by stirring for a while to precipitate solids, which were collected by suction filtration and washed with diethyl ether. The residue was dissolved in chloroform (100 mL), the solution was subjected to back extraction with water (150 mL), the aqueous layer was washed with chloroform (200 mL) and distilled off under reduced pressure to obtain 3.29 g of a brownish green amorphous substance as a coarse substance.

The substance was suspended in acetone to obtain solids, which were collected by suction filtration and washed with acetone, and the filtrate was subjected to distillation under reduced pressure. Thus, 2.32 g of a compound of the following formula (2-34) was obtained as a yellow amorphous substance (2.68 mmol, yield 38%).

The resulting compound of the formula (2-34) was combined with a separately synthesized compound of the formula (2-34) to a combined amount of 2.45 g. 2.45 g of the compound were suspended in a diethyl ether/toluene/acetone solvent mixture (3/1/1, 25 mL) to obtain solids, which were collected by suction filtration and washed with a diethyl ether/toluene/acetone solvent mixture (3/1/1). Thus, 2.24 g of the compound of the following formula (2-34) was obtained as a pale yellow solid (purity 74.7%).

The following describes the result of ¹H-NMR analysis of the compound.
¹H-NMR (500 MHz CDCl₃): δ7.66-7.55 (m, 4H), 7.32-7.24 (m, 4H), 6.55-6.40 (m, 4H), 3.35 (t, 8H, J = 5.5 Hz), 3.26-3.06 (m, 24H), 1.96-1.88 (m, 8H), 1.88-1.74 (m, 8H).

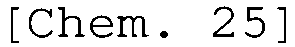

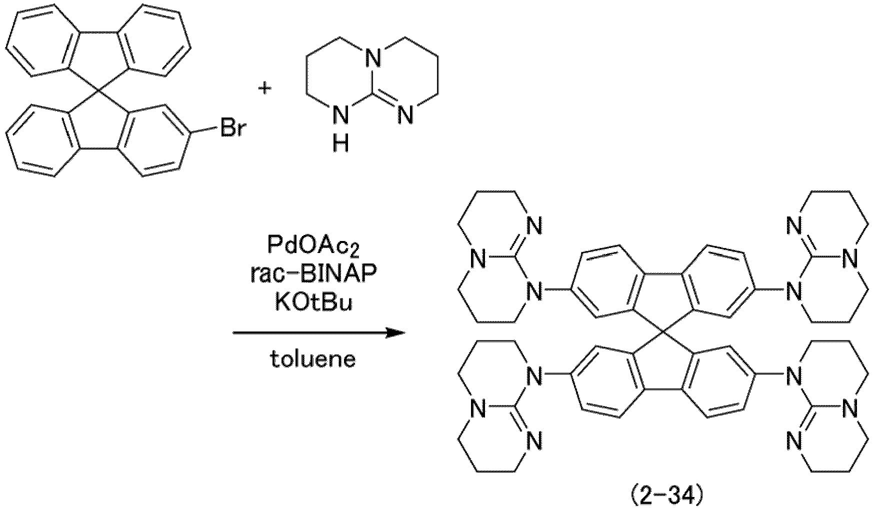

### Synthesis Example 13

A boron-containing compound of the following formula (4) was synthesized as in Synthesis Examples 1 to 3 in WO 2016/181705.

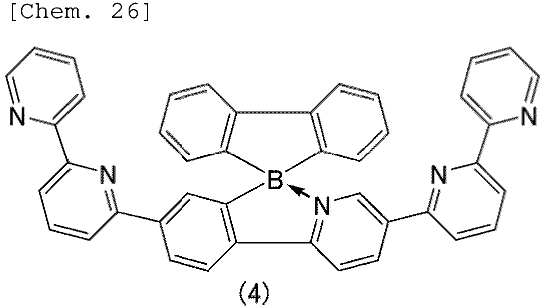

### Synthesis Example 14

### <Synthesis of monomer for synthesizing boron-containing polymer>

A 300-mL reaction vessel was charged with a compound of the following formula (8) (3.96 g), 4-pyridine boronic acid (1.03 g), Pd(PPh₃)₄ (0.24 g), sodium carbonate (2.24 g), toluene (40 mL), distilled water (40 mL), and ethanol (20 mL). The resulting suspension was stirred for 10 minutes under argon bubbling, heated to 95°C with an oil bath, and heated at the same temperature for 18 hours with stirring. To the resulting yellow solution were added water (100 mL) and toluene (100 mL) to prepare a two-layer solution. The organic layer was washed with water, followed by saturated saline, and concentrated. The resulting residue was purified by column chromatography and preparative GPC to obtain colorless solids. The solids were subjected to recrystallization with ethanol/hexane. Thus, a compound of the following formula (9) (0.69 g) was obtained.

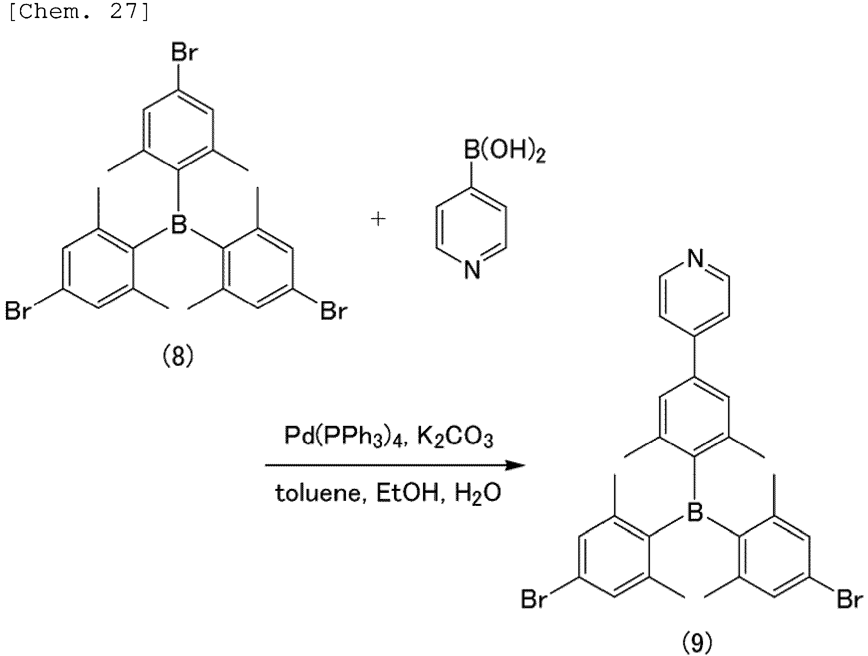

### <Synthesis of boron-containing polymer>

In an argon atmosphere, a 50-mL pressure resistant test tube was charged with the compound of the formula (9) (0.5 g), a compound of the following formula (10) (0.57 g), Pd(PPh₃)₄ (0.1 g), sodium carbonate (0.472 g), Aliquat 336 (0.2 g), toluene (10 mL), and distilled water (10 mL). The suspension was subjected to argon bubbling for about 15 minutes and stirred for 24 hours with heating in an oil bath at 100°C. Iodobenzene (0.181 g) was added thereto, the contents were stirred for 18 hours at the same temperature, phenylboronic acid (0.217 g) was added thereto, and the contents were stirred for 18 hours. The organic layer of the resulting dark brown suspension was filtered with celite, and the filtrate was concentrated. The resulting residue was purified with a column to obtain light brown powder. To the powder were added heptane and ethanol to prepare a dispersion. The dispersion were heated, and cooled to obtain solids, which were collected by filtration and were washed with ethanol. Thus, 0.61 g of a boron-containing polymer (7) was obtained.

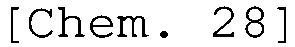

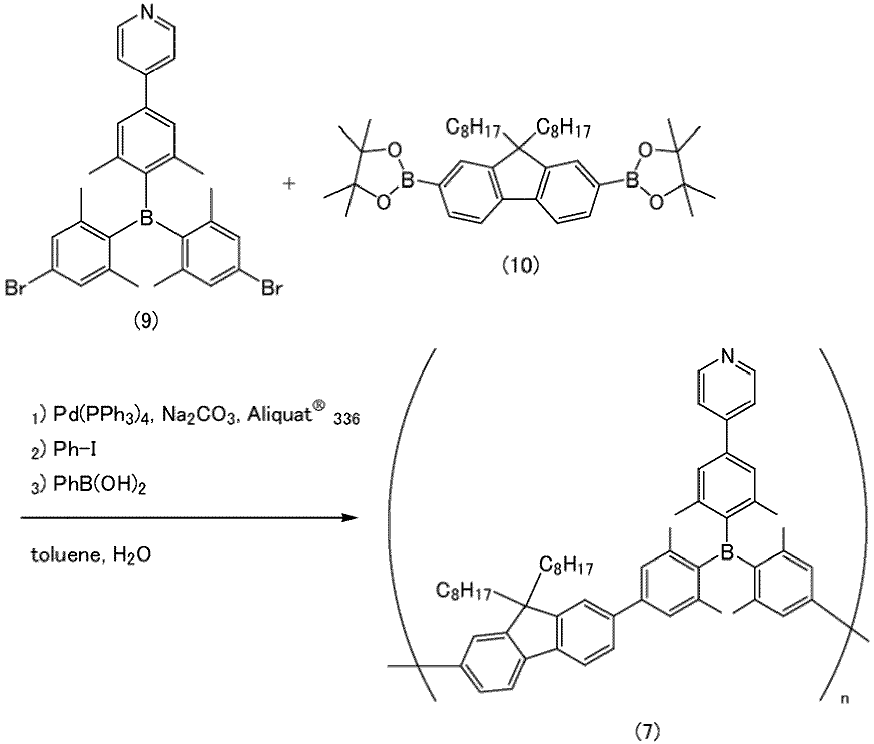

### Example 1 (not according to the invention)

The following describes production of an inverted organic EL device having the laminate structure illustrated in FIG. 1.
(1) A commercially available transparent glass substrate having an average thickness of 0.7 mm with a 150-nm-thick electrode (cathode 3) made of ITO with a pattern of 3 mm width was prepared as a substrate 2. The substrate 2 with the cathode 3 was ultrasonically cleaned in acetone and isopropanol each for 10 minutes and then boiled in isopropanol for five minutes. Thereafter, the substrate 2 with the cathode 3 was taken out from isopropanol, dried by blowing nitrogen, and cleaned with UV ozone for 20 minutes.
(2) The substrate 2 with the cathode 3 cleaned in the step (1) was fixed to a substrate holder of a mirrortron sputtering apparatus having a zinc metal target. The pressure in the chamber of the sputtering apparatus was reduced to about 1 × 10⁻⁴ Pa, and the substrate 2 was subjected to sputtering with argon and oxygen introduced therein to form a zinc oxide layer (oxide layer 4) having a thickness of about 3 nm on the cathode 3 of the substrate 2. Upon the formation of the zinc oxide layer, the zinc oxide film was not formed on part of the ITO electrode (cathode 3) in order to lead out the electrode. The substrate 2 with the oxide layer 4 was annealed in the atmosphere at 400°C for one hour.
(3) Next, an organic thin film containing the first material and the second material was formed as an electron injection layer 5 on the oxide layer 4 in the following way.
   First, the boron-containing compound of the formula (4) and the compound of the formula (2-2) in a weight ratio of 1:0.05 were dissolved in cyclopentanone at a concentration of 0.5% by weight to prepare a coating composition. Next, the substrate 2 with the cathode 3 and the oxide layer 4 produced in the step (2) was placed on a spin coater. Then, the substrate 2 was rotated at 3000 rpm for 30 seconds while the coating composition was dropped on the oxide layer 4 and a coat was formed. Subsequently, the substrate 2 was annealed in a nitrogen atmosphere at 150°C for one hour using an electric griddle to form the electron injection layer 5. The electron injection layer 5 had an average thickness of 15 nm.
(4) Next, the substrate 2 with the electron injection layer 5 and the previous layers was fixed to a substrate holder of a vacuum evaporation apparatus. Bis(2-(2-benzothiazolyl)phenolato) zinc(II) (Zn(BTZ)₂) of the following formula (11), tris[1-phenylisoquinoline]iridium(III) (Ir(piq)₃) of the following formula (12), N,N'-di(1-naphthyl)-N,N'-diphenyl-1,1'-biphenyl-4,4'-diamine (α-NPD) of the following formula (13), 1,4,5,8,9,12-hexaazatriphenylene-2,3,6,7,10,11-hexacarbonitrile (HAT-CN) of the following formula (14), and Al were separately put into alumina crucibles, which were set as evaporation sources.

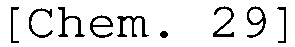

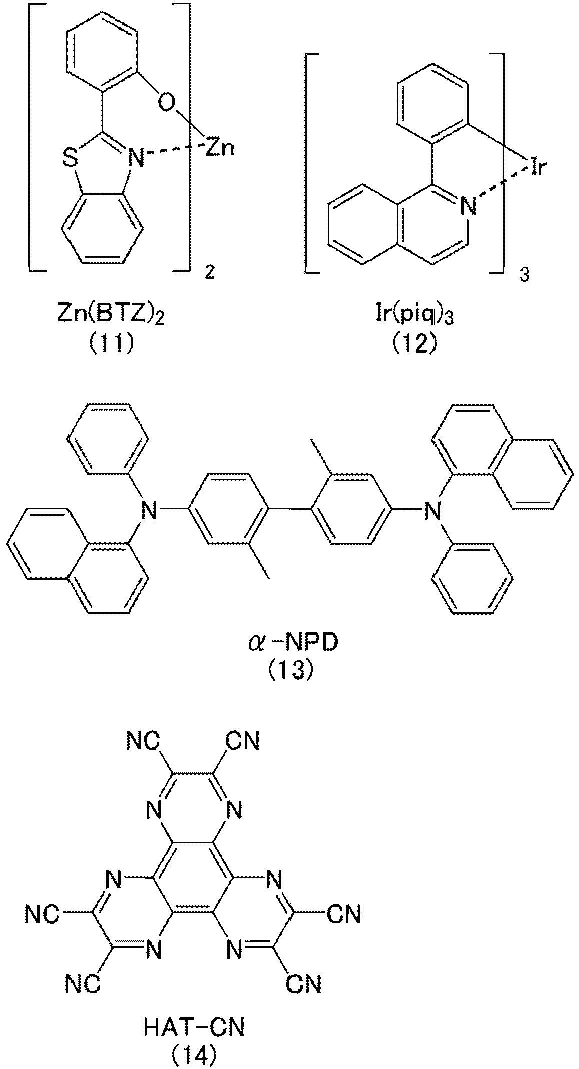

The pressure in the chamber of the vacuum evaporation apparatus was reduced to 1 × 10⁻⁵ Pa, and an electron transport layer 10, an emitting layer 6, a hole transport layer 7, a hole injection layer 8, and an anode 9 were successively formed by a vacuum evaporation method by resistance heating.

First, the electron transport layer 10 with a thickness of 10 nm made of Zn(BTZ)₂ was formed. Subsequently, the emitting layer 6 was formed by co-evaporating Zn(BTZ)₂ as a host and Ir(piq)₃ as a dopant to a thickness of 20 nm. The doping concentration was controlled such that Ir(piq)₃ would be 6% by mass relative to the entire emitting layer 6. Next, the hole transport layer 7 was formed on the substrate 2 with the emitting layer 6 and the previous layers by forming a 50-nm-thick α-NPD film. The hole injection layer 8 was formed by forming a 10-nm-thick HAT-CN film. Next, the anode 9 with a thickness of 100 nm made of aluminum was formed by a vacuum evaporation method on the substrate 2 with the hole injection layer 8 and the previous layers.

The anode 9 was formed using a stainless steel evaporation mask to have a 3-mm-width-band-like evaporation area. The produced organic EL device had an emitting area of 9 mm².

(5) Next, the substrate 2 with the anode 9 and the previous layers was placed in a glass cap (sealing container) with a recessed space, and the container was filled with a sealing material made of an ultraviolet (UV) curable resin to seal the substrate. Thus, an organic EL device of Example 1 was obtained.

### Example 2

An organic EL device of Example 2 was obtained as in Example 1 except that, in the step (3), a compound of the formula (2-4) was used instead of the compound of the formula (2-2).

### Comparative Example 1

An organic EL device of Comparative Example 1 was obtained as in Example 1 except that, in the step (3), a coating composition was used which was prepared by dissolving only the boron-containing compound of the formula (4) in cyclopentanone at a concentration of 0.5% by weight without using the compound of the formula (2-2).

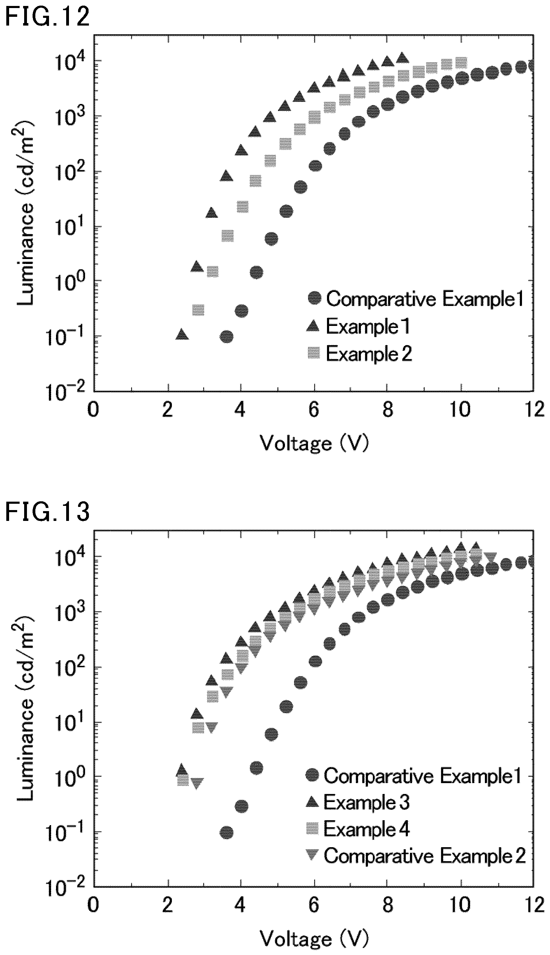

To the organic EL devices produced in Examples 1 and 2 and Comparative Example 1 was applied a voltage using "2400 series SourceMeter" available from Keithley Instruments, and the luminance was measured using "LS-100" available from Konica Minolta, Inc. Thus, the relationship between the applied voltage and the luminance was determined. The results are shown in FIG. 12.

### Example 3 (not according to the invention)

An organic EL device of Example 3 was obtained as in Example 1 except that, in the step (3), a coating composition was used which was prepared by dissolving the boron-containing compound of the formula (4) and the compound of the formula (2-2) in a weight ratio of 1:2 in cyclopentanone at a concentration of 0.5% by weight.

### Example 4

An organic EL device of Example 4 was obtained as in Example 1 except that, in the step (3), a coating composition was used which was prepared by dissolving the boron-containing compound of the formula (4) and the compound of the formula (2-9) in a weight ratio of 1:2 in cyclopentanone at a concentration of 0.5% by weight.

### Comparative Example 2

An organic EL device of Comparative Example 2 was obtained as in Example 1 except that, in the step (3), a coating composition was used which was prepared by dissolving the boron-containing compound of the formula (4) and MTBD of the following formula (15) in a weight ratio of 1:2 in cyclopentanone at a concentration of 0.5% by weight.

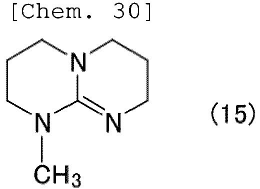

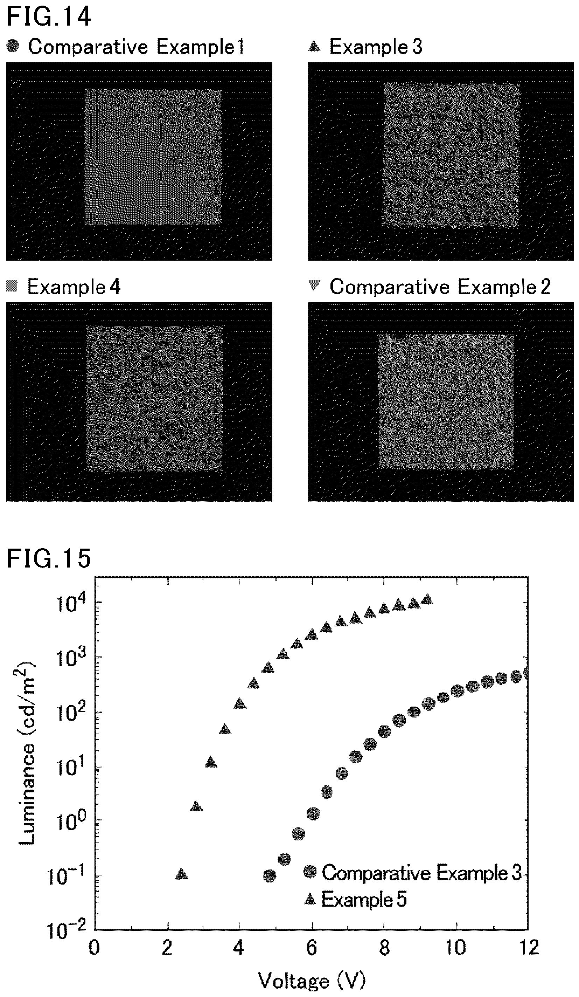

To the organic EL devices produced in Examples 3 and 4 and Comparative Examples 1 and 2 was applied a voltage using "2400 series SourceMeter" available from Keithley Instruments, and the luminance was measured using "LS-100" available from Konica Minolta, Inc. Thus, the relationship between the applied voltage and the luminance was determined. The results are shown in FIG. 13. FIG. 14 illustrates emissions of these devices.

### Example 5 (not according to the invention)

An organic EL device of Example 5 was obtained as in Example 1 except that the step (3) was changed to the following step (3-1).

(3-1) The boron-containing compound of the formula (4) as a host and the compound of the formula (2-2) as a dopant were co-evaporated to a thickness of 10 nm using a vacuum evaporation apparatus to form an electron injection layer 5. The doping concentration was controlled such that the compound of the formula (2-2) would be 5% by mass relative to the entire electron injection layer 5.

### Comparative Example 3

An organic EL device of Comparative Example 3 was obtained as in Example 5 except that, in the step (3-1), only a 10-nm-thinck film of the boron-containing compound of the formula (4) was formed.

To the organic EL devices produced in Example 5 and Comparative Example 3 was applied a voltage using "2400 series SourceMeter" available from Keithley Instruments, and the luminance was measured using "LS-100" available from Konica Minolta, Inc. Thus, the relationship between the applied voltage and the luminance was determined. The results are shown in FIG. 15.

### Example 6 (not according to the invention)

An organic EL device of Example 6 was obtained as in Example 1 except that the step (2) was not performed and the step (3) was changed to the step (3-1).

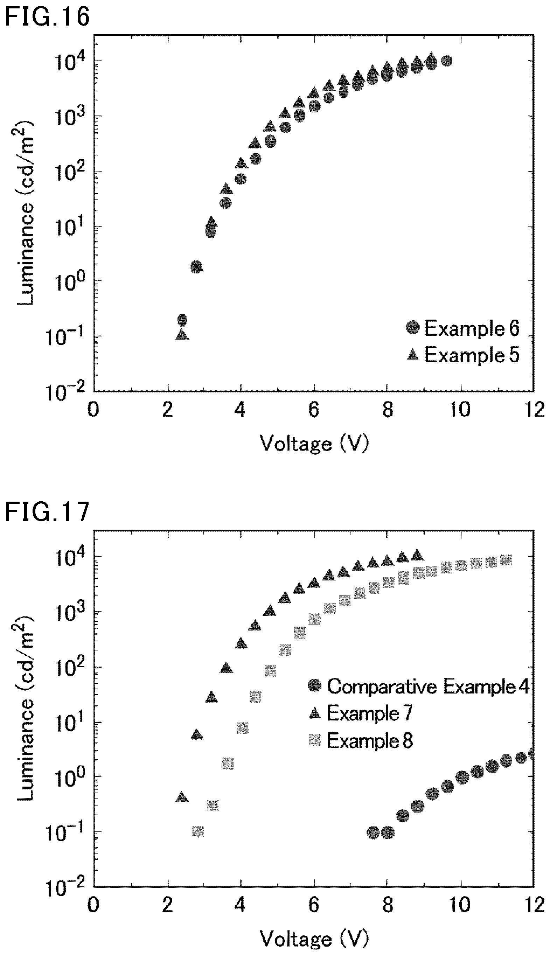

To the organic EL devices produced in Examples 5 and 6 was applied a voltage using "2400 series SourceMeter" available from Keithley Instruments, and the luminance was measured using "LS-100" available from Konica Minolta, Inc. Thus, the relationship between the applied voltage and the luminance was determined. The results are shown in FIG. 16.

### Example 7 (not according to the invention)

An organic EL device of Example 7 was obtained as in Example 5 except that, in the step (3-1), a triazine compound of the following formula (16) disclosed in JP 2018-206889 A was used instead of the compound of the formula (4), and the doping concentration of the compound of the formula (2-2) was 10% by mass.

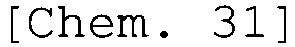

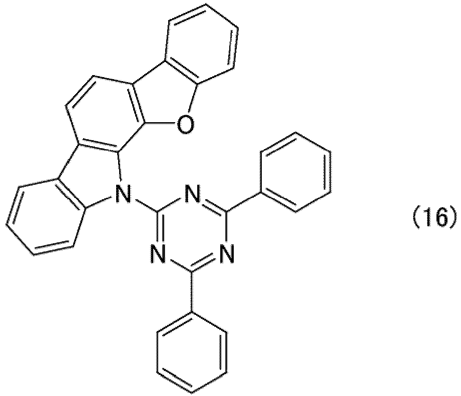

### Example 8 (not according to the invention)

An organic EL device of Example 8 was obtained as in Example 1 except that the step (3) was changed to the following step (3-2).

(3-2) The compound of the formula (2-2) was evaporated to a thickness of 1 nm, and then, the compound of the formula (16) was evaporated to a thickness of 10 nm using a vacuum evaporation apparatus to form a laminate of a layer of the compound of the formula (2-2) and a layer of the compound of the formula (16) as the electron injection layer 5.

### Comparative Example 4

An organic EL device of Comparative Example 4 was obtained as in Example 7 except that, in the step (3-1), only a 10-nm-thick film of the triazine compound of the formula (16) was formed.

To the organic EL devices produced in Examples 7 and 8 and Comparative Example 4 was applied a voltage using "2400 series SourceMeter" available from Keithley Instruments, and the luminance was measured using "LS-100" available from Konica Minolta, Inc. Thus, the relationship between the applied voltage and the luminance was determined. The results are shown in FIG. 17.

### Example 9 (not according to the invention)

An organic EL device of Example 9 was obtained as in Example 5 except that, in the step (3-1), a boron compound of the following formula (17) was used instead of the compound of the formula (4), and the doping concentration of the compound of the formula (2-2) was 5% by mass.

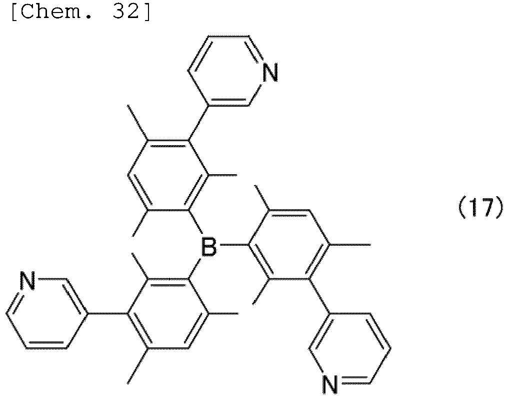

### Example 10 (not according to the invention)

An organic EL device of Example 10 was obtained as in Example 1 except that the step (3) was changed to the following step (3-3).

(3-3) First, a boron-containing polymer of the following formula (7) was dissolved in dimethylacetamide at a concentration of 0.1% by weight to prepare a coating composition. Next, the substrate 2 with the cathode 3 and the oxide layer 4 produced in the step (2) was placed on a spin coater. Then, the substrate 2 was rotated at 3000 rpm for 30 seconds while the coating composition was dropped on the oxide layer 4. Thus, a coat was formed. Subsequently, the substrate 2 was annealed in a nitrogen atmosphere at 120°C for two hours using an electric griddle to form a layer of the second material of the compound of the formula (7). The layer had an average thickness of 15 nm. Next, the substrate 2 was fixed to a substrate holder of a vacuum evaporation apparatus, the boron-containing compound of the formula (16) as a host and the compound of the formula (2-2) as a dopant were co-evaporated to a thickness of 10 nm. Thus, an electron injection layer 5 in which a film of a mixture of the compound of the formula (17) and the compound of the formula (2-2) was laminated on the compound of the formula (7) was formed. In the film of a mixture of the compound of the formula (17) and the compound of the formula (2-2), the amount of the compound of the formula (2-2) was controlled to 5% by mass relative to the boron compound of the formula (17).

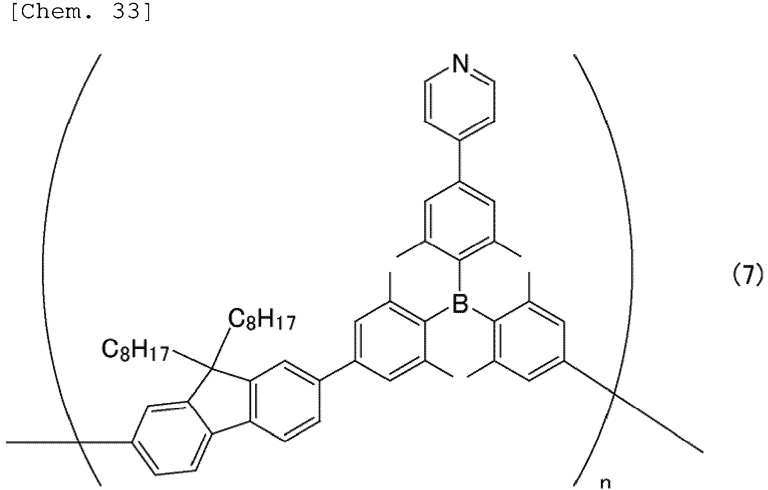

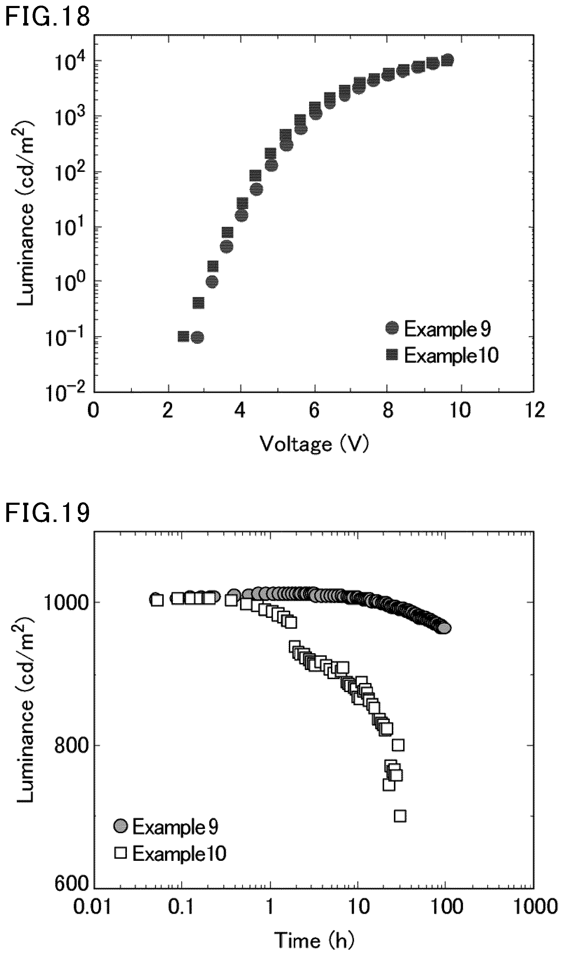

To the organic EL devices produced in Examples 9 and 10 was applied a voltage using "2400 series SourceMeter" available from Keithley Instruments, and the luminance was measured using "LS-100" available from Konica Minolta, Inc. Thus, the relationship between the applied voltage and the luminance and the temporal change of the luminance were determined. The results are shown in FIGs. 18 and 19.

### Example 11 (not according to the invention)

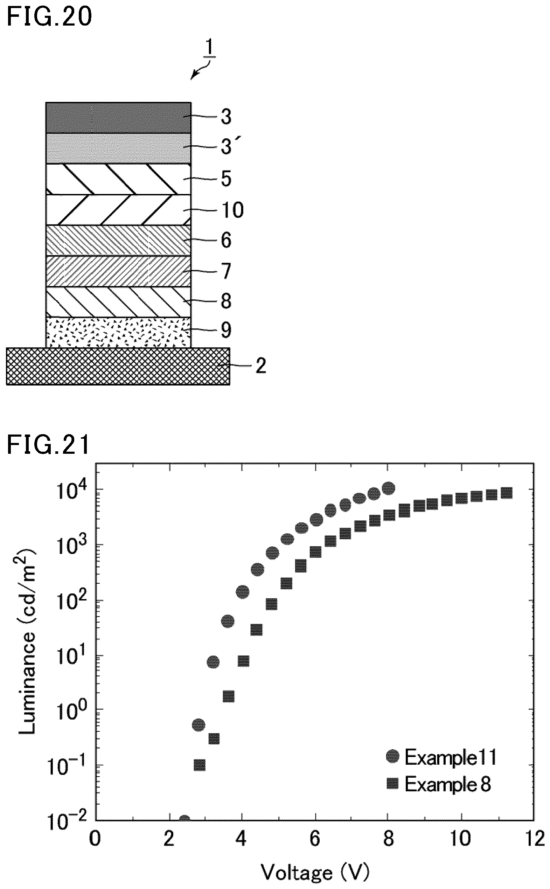

A conventional organic EL device 11 having the laminate structure illustrated in FIG. 20 was produced in the following way.
(1) A commercially available transparent glass substrate 2 (hereinafter, also referred to simply as a substrate) having an average thickness of 0.7 mm with an anode 9 made of a 100-nm-thick ITO film with a pattern of 3 mm width was prepared.
(2) Next, the substrate 2 with the anode 9 was ultrasonically cleaned in acetone and isopropanol each for 10 minutes and then boiled in isopropanol for five minutes. Then, the substrate was taken out from isopropanol, dried by blowing nitrogen, and cleaned with UV ozone for 20 minutes.
(3) Next, a 30-nm-thick hole injection layer 8 made of PEDOT (Clevios HIL1.3N) was formed.
(4) Next, the substrate with the PEDOT layer and the previous layer was fixed to a substrate holder in a chamber of a vacuum evaporation apparatus, and the pressure in the chamber of the vacuum evaporation apparatus was reduced to 1 × 10⁻⁵ Pa. A hole transport layer 7, an emitting layer 6, an electron transport layer 10, an electron injection layer 5, a cathode 1 (cathode 3' illustrated in FIG. 20), and a cathode 2 (cathode 3 illustrated in FIG. 20) were successively formed by a vacuum evaporation method involving resistance heating.

First, a hole transport layer with a thickness of 30 nm made of α-NPD of the formula (13) was formed. Subsequently, an emitting layer was formed by co-evaporating Zn(BTZ)₂ of the formula (11) as a host and Ir(piq)₃ of the formula (12) as a dopant to a thickness of 30 nm. The doping concentration was controlled such that Ir(piq)₃ would be 6% by mass relative to the entire emitting layer. Next, an electron transport layer was formed by forming a 40-nm-thick film made of TmPPyTz of the following formula (18) on the substrate with the emitting layer and the previous layers. An electron injection layer was formed by forming a 1-nm-thick film made of the compound of the formula (2-2). Next, a cathode 1 (cathode 3' illustrated in FIG. 20) with a thickness of 25 nm was formed by co-evaporating silver and magnesium at a mass ratio of 9:1 on the substrate with the electron injection layer and the previous layers, and the cathode 2 (cathode 3 illustrated in FIG. 20) with a thickness of 100 nm consisting of silver was formed thereon.

The cathodes 1 and 2 were each formed using a stainless steel evaporation mask to have a 3-mm-width-band-like evaporation area. The produced organic EL device had an emitting area of 9 mm².

(5) Next, the substrate with the cathode and the previous layers was placed in a glass cap (sealing container) with a recessed space, and the container was filled with a sealing material made of an ultraviolet (UV) curable resin to seal the substrate. Thus, an organic EL device of Example 11 was obtained.

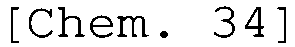

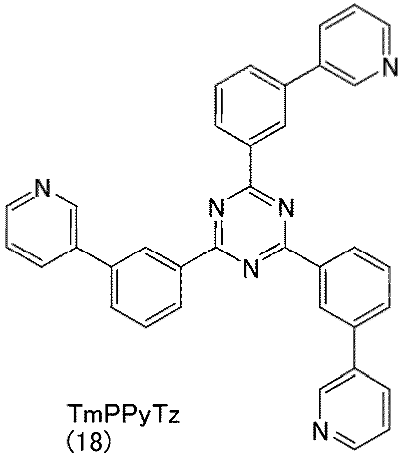

To the organic EL device produced in Example 11 and the organic EL device produced in Example 8 was applied a voltage using "2400 series SourceMeter" available from Keithley Instruments, and the luminance was measured using "LS-100" available from Konica Minolta, Inc. Thus, the relationship between the applied voltage and the luminance was determined. The results are shown in FIG. 21.

### Example 12 (not according to the invention)

A device of Example 12 was produced as in Example 11 except that the step (4) was changed to the following step (4-1).

(4-1) Next, the substrate with the PEDOT layer and the previous layer was fixed to a substrate holder in a chamber of a vacuum evaporation apparatus, and the pressure in the chamber of the vacuum evaporation apparatus was reduced to 1 × 10⁻⁵. A hole transport layer, an emitting layer, an electron transport layer, an electron injection layer, and a cathode were successively formed by a vacuum evaporation method involving resistance heating.

First, a hole transport layer with a thickness of 40 nm made of α-NPD of the formula (13) was formed. Subsequently, an emitting layer was formed by co-evaporating Zn(BTZ)₂ of the formula (11) as a host and Ir(piq)₃ of the formula (12) as a dopant to a thickness of 30 nm. The doping concentration was controlled such that Ir(piq)₃ would be 6% by mass relative to the entire emitting layer. Next, an electron transport layer was formed by forming a 10-nm-thick film of the boron-containing compound of the formula (4) on the substrate with the emitting layer and the previous layers.

Further, an electron injection layer was formed by co-evaporating the boron-containing compound of the formula (4) as a host and the compound of the formula (2-2) as a dopant to a thickness of 35 nm. The doping concentration was controlled such that the compound of the formula (2-2) would be 5% by mass relative to the electron injection layer.

Next, a cathode with a thickness of 100 nm made of aluminum was formed on the substrate with the hole injection layer and the previous layers.

The cathode was formed using a stainless steel evaporation mask to have a 3-mm-width-band-like evaporation area. The produced organic EL device had an emitting area of 9 mm².

### Example 13 (not according to the invention)

A device of Example 13 was produced as in Example 12 except that the doping concentration of the compound (2-2) in the electron injection layer was 20% by mass.

### Comparative Example 5

A device of Comparative Example 5 was produced as in Example 12 except that the electron injection layer consists of the boron-containing compound of the formula (4).

### Comparative Example 6

A device of Comparative Example 6 was produced as in Example 12 except that the thickness of the electron transport layer made of a boron-containing compound (4) was 45 nm and a 0.8-nm-thick film made of lithium fluoride was formed as the electron injection layer.

### Example 14 (not according to the invention)

A device of Example 14 was produced as in Example 12 except that the step (4-1) was changed to the following step (4-2).

(4-2) Next, the substrate with the PEDOT layer and the previous layer was fixed to a substrate holder in a chamber of a vacuum evaporation apparatus, and the pressure in the chamber of the vacuum evaporation apparatus was reduced to 1 × 10⁻⁵ Pa. A hole transport layer, an emitting layer, an electron transport layer, an electron injection layer, and a cathode were successively formed by a vacuum evaporation method involving resistance heating.

First, a hole transport layer with a thickness of 40 nm made of α-NPD of the formula (13) was formed. Subsequently, an emitting layer was formed by co-evaporating Zn(BTZ)₂ of the formula (11) as a host and Ir(piq)₃ of the formula (12) as a dopant to a thickness of 30 nm. The doping concentration was controlled such that Ir(piq)₃ would be 6% by mass relative to the entire emitting layer. Next, an electron transport layer was formed by forming a 10-nm-thick film made of the boron-containing compound of the formula (4) on the substrate with the emitting layer and the previous layers.

The boron-containing compound of the formula (4) as a host and the compound of the formula (2-2) as a dopant were co-evaporated to a thickness of 35 nm. The doping concentration was controlled such that the compound of the formula (2-2) would be 5% by mass relative to the entire electron injection layer 1.

In addition, the boron-containing compound of the formula (4) was evaporated to a thickness of 5 nm, and a laminate film including a film containing the first material and the second material and a film containing only the second material was formed as an electron injection layer.

Next, a cathode with a thickness of 100 nm made of aluminum was formed on the substrate with the electron injection layer and the previous layers.

The cathode was formed using a stainless steel evaporation mask to have a 3-mm-width-band-like evaporation area. The produced organic EL device had an emitting area of 9 mm².

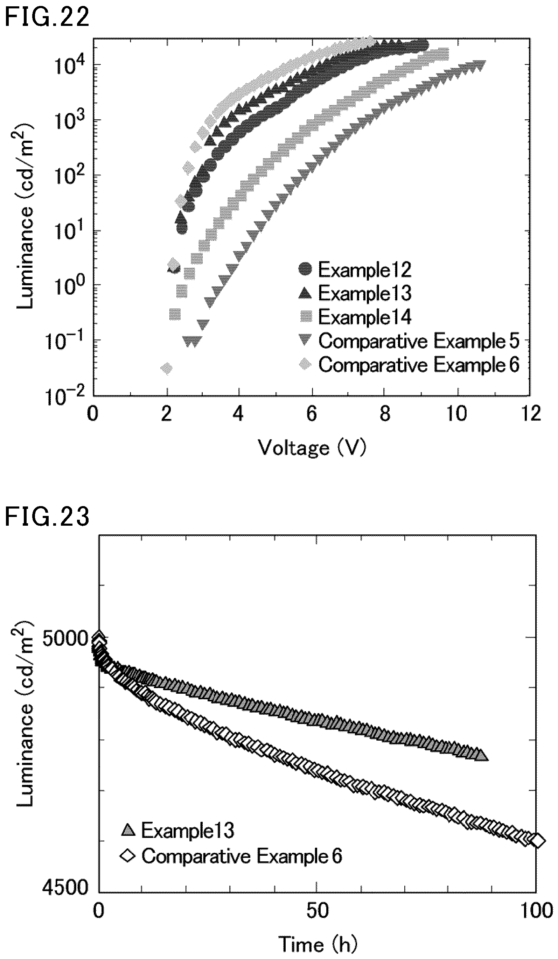

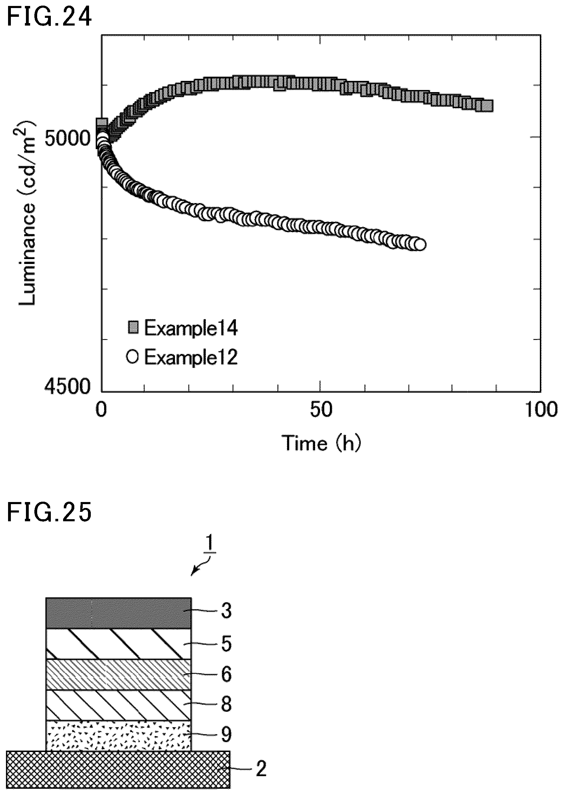

To the organic EL devices produced in Examples 12 to 14 and Comparative Examples 5 and 6 was applied a voltage using "2400 series SourceMeter" available from Keithley Instruments, and the luminance was measured using "LS-100" available from Konica Minolta, Inc. Thus, the relationship between the applied voltage and the luminance was determined. The results are shown in FIG. 22. The temporal change of the luminance of the organic EL devices of Example 13 and Comparative Example 6 and the temporal change of the luminance of the organic EL devices of Examples 12 and 14 were examined. The results are shown in FIGs. 23 and 24.

### Example 15 (not according to the invention)

A conventional organic EL device having the laminate structure illustrated in FIG. 25 was produced in the following way.
(1) A commercially available transparent glass substrate 2 having an average thickness of 0.7 mm with an ITO electrode layer was prepared. The ITO electrode (anode 9) on the substrate had a pattern of 2 mm width. The substrate was ultrasonically cleaned with Clean Ace and pure water, followed by steam cleaning in isopropanol for five minutes. The substrate was dried by blowing nitrogen, and cleaned with UV ozone for 10 minutes.
(2) The substrate was placed on a spin coater, poly(3,4-ethylenedioxythiophene/styrene sulfonic acid) (PEDOT/PSS) (Clevios CH8000) was dropped thereon, and the substrate was rotated at 2000 rpm for 60 seconds, and dried with an electric griddle at 130°C for 10 minutes. Thus, a hole injection layer 8 made of PEDOT/PSS was formed on the anode. The hole injection layer had an average thickness of 50 nm. The average thickness of the hole injection layer was measured with a stylus profiler.
(3) A 2% solution of poly(dioctylfluorene-o-benzothiadiazole) (F8BT) in xylene was prepared. The substrate produced in the step (2) was placed on a spin coater. The F8BT-xylene solution was dropped on the hole injection layer formed in the step (2), and the substrate was rotated at 2,000 rpm for 60 seconds. Thus, an emitting layer 6 made of F8BT was formed. The emitting layer had an average thickness of 20 nm. The average thickness of the emitting layer was measured with a stylus profiler.
(4) The substrate produced in the step (3) was placed on a spin coater. A 1% by weight solution of the compound of the formula (2-2) in 1-propanol was dropped on the emitting layer formed in the step (3), and the substrate was rotated at 2000 rpm for 60 seconds. Thus, an electron injection layer 5 was formed on the emitting layer. The average thickness of the electron injection layer was off the scale of a stylus profiler.
(5) The substrate produced in the step (4) was fixed to a substrate holder of a vacuum evaporation apparatus. Aluminum wire (Al) was put into an alumina crucible, which was set as a evaporation source. The pressure in the vacuum evaporation apparatus was reduced to about 1 × 10⁻⁴ Pa, Al (cathode 3) was evaporated to have an average thickness of 100 nm on the electron injection layer. Thus, an organic electroluminescence device was produced. The average thickness of the cathode was measured with a quartz crystal film thickness monitor during its formation.

### Comparative Example 7

An organic EL device was produced by performing the steps (1) to (3) in Example 15, followed by the following step (4-1).

(4-1) The substrate produced in the step (3) was fixed to a substrate holder of a vacuum evaporation apparatus. Lithium fluoride (LiF) and aluminum wire (Al) were separately put into crucibles, which were set as evaporation sources. The pressure in the vacuum evaporation apparatus was reduced to about 1 × 10⁻⁴ Pa, LiF (electron injection layer 5) was evaporated to have an average thickness of 1 nm, followed by evaporation of Al (cathode 3) to have an average thickness of 100 nm. Thus, an organic electroluminescence device was produced.

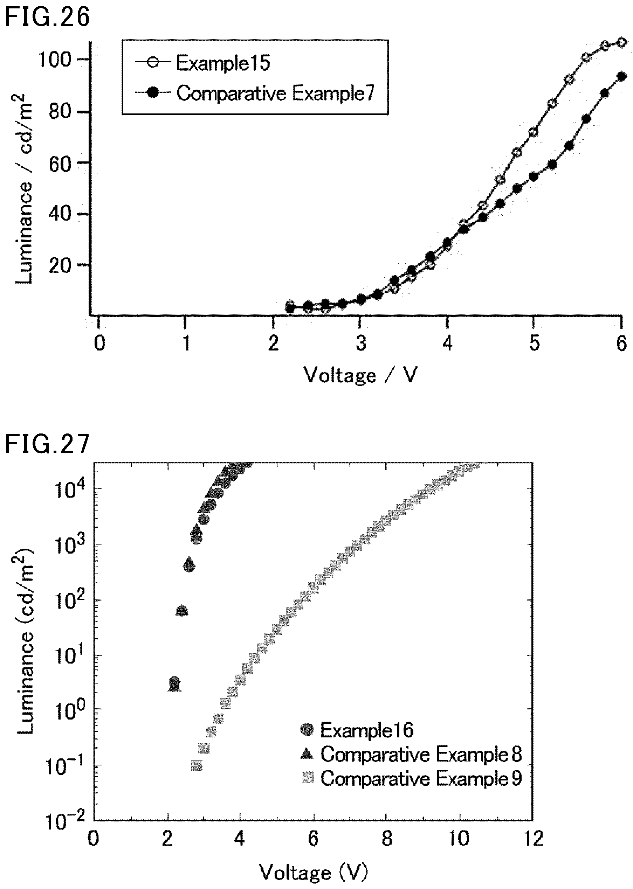

To the organic EL devices of Example 15 and Comparative Example 7 was applied a voltage using "2400 series SourceMeter" available from Keithley Instruments, and the luminance was measured using "LS-100" available from Konica Minolta, Inc. Thus, the relationship between the applied voltage and the luminance was determined. The results are shown in FIG. 26.

### Examples 16 to 20 (not according to the invention) and Comparative Examples 8 to 15

A conventional organic EL device was produced in the following way. The devices of Examples 16 to 20 and Comparative Examples 8, 10, 12, and 14 each had the laminate structure illustrated in FIG. 2, and the devices of Comparative Examples 9, 11, 13, and 15 each had a laminate structure prepared by removing the electron injection layer 5 from the laminate structure illustrated in FIG. 2.
(1) A commercially available transparent glass substrate 2 having an average thickness of 0.7 mm with a 100-nm-thick electrode (anode 9) made of ITO with a pattern of 3 mm width was prepared as a substrate 2.
   The substrate 2 with the anode 9 was ultrasonically cleaned in acetone and isopropanol each for 10 minutes and then boiled in isopropanol for five minutes. Thereafter, the substrate 2 with the anode 9 was taken out from isopropanol, dried by blowing nitrogen, and cleaned with UV ozone for 20 minutes.
(2) The substrate 2 with the anode 9 cleaned in the step (1) was placed on a spin coater, and a 10-nm-thick film made of a hole injection material "Clevios HIL1.3N" available from Heraeus was formed as a hole injection layer 8. Thereafter, the substrate was heated on an electric griddle at 180°C for one hour.
(3) Next, the substrate 2 with the hole injection layer 8 and the previous layers was fixed to a substrate holder of a vacuum evaporation apparatus. 2,4-Diphenyl-6-bis((12-phenylindolo) [2,3-a]carbazol-11-yl)-1,3,5-triazine (DIC-TRZ) of the following formula (19), fac-tris(3-methyl-2-phenylpyridinato-N,C2'-)iridium(III) (Ir(mppy)₃) of the following formula (20), α-NPD of the formula (13), N3,N3‴-bis(dibenzo[b,d]thiophen-4-yl)-N3,N3‴-diphenyl-[1,1':2',1":2",1‴-quaterphenyl)-3,3‴-diamine (4DBTP3Q) of the following formula (21), the compound of the formula (2-2) serving as the first material, any of the following various materials serving as the second material (electron transport material), and Al were separately put into alumina crucibles, which were set as evaporation sources.

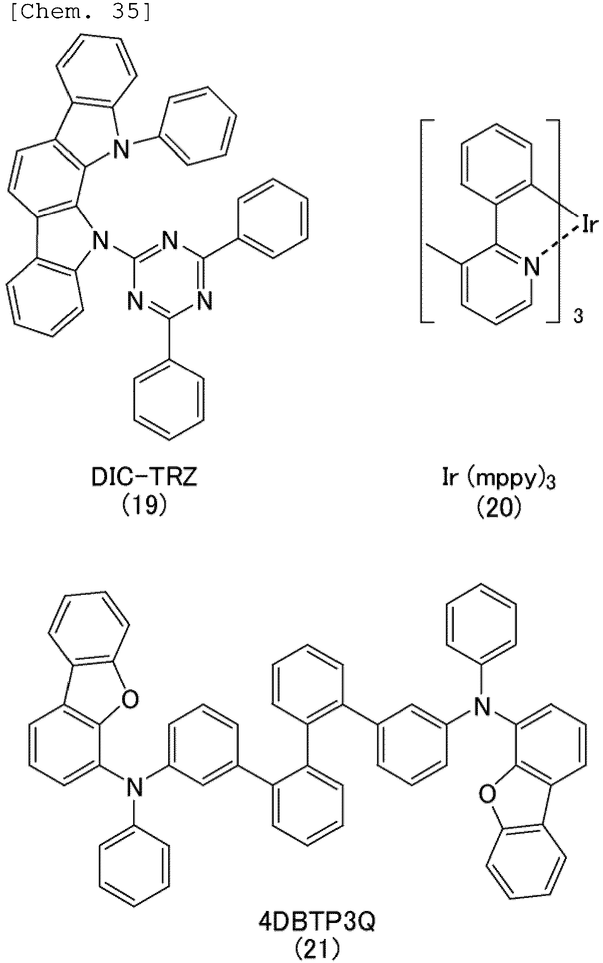

A compound of the following formula (22) as an exemplary pyridine-containing compound, a compound of the following formula (23) and a compound of the following formula (26) as an exemplary triazine derivative, a commercial product of the following formula (24) as a compound containing a carbonyl-containing heterocyclic ring, or a compound of the following formula (25) as an exemplary phenanthroline derivative was used as the second material.

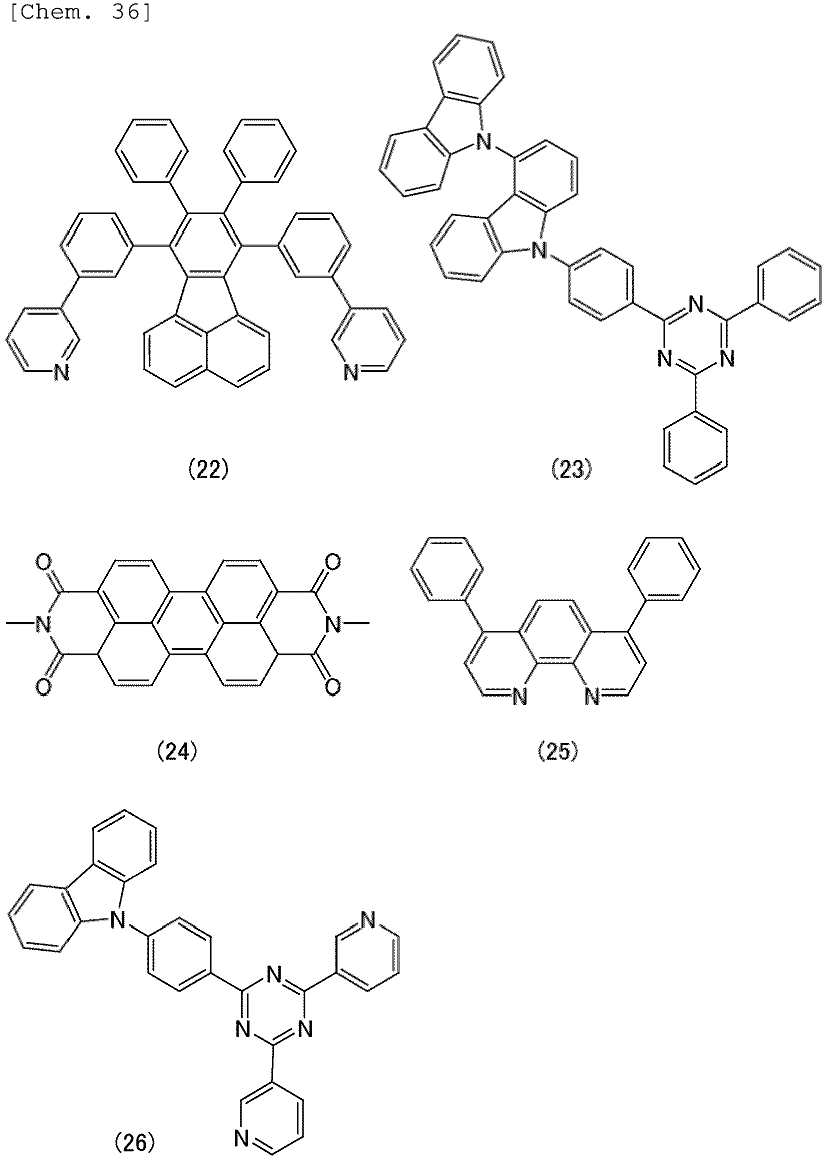

The pressure in the chamber of the vacuum evaporation apparatus was reduced to 1 × 10⁻⁵ Pa. A hole transport layer 7, an emitting layer 6, an electron transport layer 10, an electron injection layer 5, and a cathode 3 were successively formed by a vacuum evaporation method involving resistance heating.

Specifically, first, the hole transport layer 7 with a thickness of 30 nm including 20-nm-thick α-NPD and 10-nm-thick 4DBTP3Q was formed. Subsequently, the emitting layer 6 was formed by co-evaporating DIC-TRZ as a host and Ir(mppy)₃ as a dopant to a thickness of 25 nm. The doping concentration was controlled such that Ir(mppy)₃ would be 3% by mass relative to the entire emitting layer 6. Next, the electron transport layer 10 with a thickness of 40 nm and the electron injection layer 5 with a thickness of 1 nm were formed on the substrate 2 with the emitting layer 6 and the previous layers. Next, the cathode 3 with a thickness of 100 nm made of aluminum was formed by a vacuum evaporation method on the substrate 2 with the electron injection layer 5 and the previous layers. The cathode 3 was formed using a stainless steel evaporation mask to have a 3-mm-width-band-like evaporation area. The produced organic EL device had an emitting area of 9 mm².

The following describes the compounds used as the second material (electron transport layer) and the first material (electron injection layer) in the examples.

Example 16 (not according to the invention) : the second material and the first material were respectively the compound of the formula (22) and the compound of the formula (2-2).

Example 17 (not according to the invention) : the second material and the first material were respectively the compound of the formula (23) and the compound of the formula (2-2).

Example 18 (not according to the invention) : the second material and the first material were respectively the compound of the formula (24) and the compound of the formula (2-2).

Example 19 (not according to the invention) : the second material and the first material were respectively the compound of the formula (25) and the compound of the formula (2-2).

Example 20 (not according to the invention) : the second material and the first material were respectively the compound of the formula (26) and the compound of the formula (2-2).

The following describes the compounds used as the second material (electron transport layer) and the first material (electron injection layer) in Comparative Examples 8, 10, 12, and 14.

Comparative Example 8: the second material and the first material were respectively the compound of the formula (22) and lithium fluoride.

Comparative Example 10: the second material and the first material were respectively the compound of the formula (23) and lithium fluoride.

Comparative Example 12: the second material and the first material were respectively the compound of the formula (24) and lithium fluoride.

Comparative Example 14: the second material and the first material were respectively the compound of the formula (26) and lithium fluoride.

The following describes the compounds used as the second material (electron transport layer) in Comparative Examples 9, 11, 13, and 15. No electron injection layer (first material) was formed before the formation of a cathode.

Comparative Example 9: the second material was the compound of the formula (22).

Comparative Example 11: the second material was the compound of the formula (23).

Comparative Example 13: the second material was the compound of the formula (24).

Comparative Example 15: the second material was the compound of the formula (25).

(4) Next, the substrate 2 with the cathode 3 and the previous layers was placed in a glass cap (sealing container) with a recessed space, and the container was filled with a sealing material made of an ultraviolet (UV) curable resin to seal the substrate. Thus, each organic EL device was obtained.

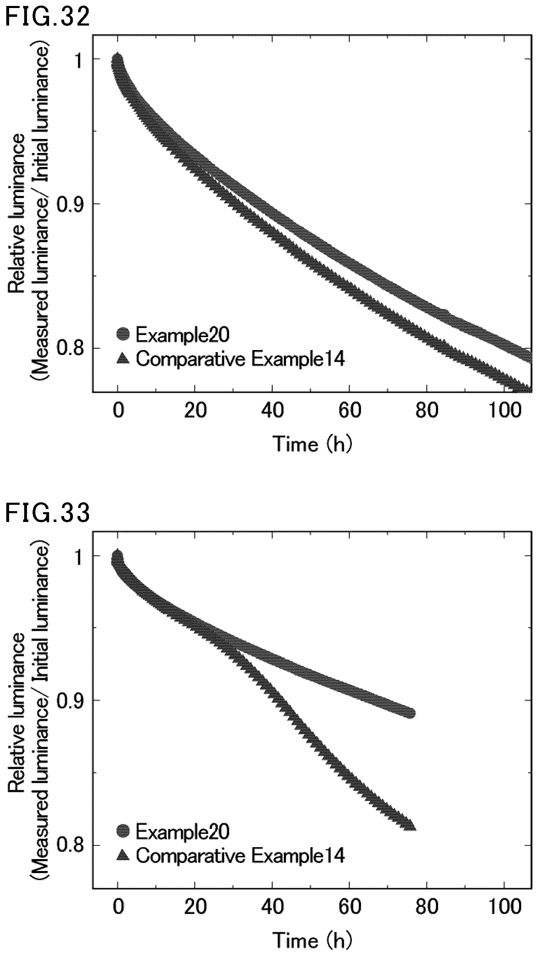

To the organic EL devices produced in Examples 16 to 20 and Comparative Examples 8 to 15 was applied a voltage using "2400 series SourceMeter" available from Keithley Instruments, and the luminance was measured using "LS-100" available from Konica Minolta, Inc. Thus, the relationship between the applied voltage and the luminance was determined. The results are shown in FIGs. 27 to 31. FIG. 32 illustrates the temporal change of the luminance of the organic EL devices of Example 20 and Comparative Example 15 at room temperature, and FIG. 33 illustrates the temporal change of the luminance of the organic EL devices of Example 20 and Comparative Example 15 at 85°C.

### Examples 21 and 22 (not according to the invention) and Comparative Example 16

Conventional organic EL devices were produced and evaluated in the following way. The devices of Example 21 and Comparative Example 16 were organic EL devices each having the laminate structure illustrated in FIG. 3, and the device of Example 22 was an organic EL device having the laminate structure illustrated in FIG. 4.
(1) A commercially available transparent glass substrate 2 having an average thickness of 0.7 mm with a 100-nm-thick electrode (anode 9) made of ITO with a pattern of 3 mm width was prepared as a substrate 2. The substrate 2 with the anode 9 was ultrasonically cleaned in acetone and isopropanol each for 10 minutes and then boiled in isopropanol for five minutes. Thereafter, the substrate 2 with the anode 9 was taken out from isopropanol, dried by blowing nitrogen, and cleaned with UV ozone for 20 minutes.
(2) The substrate 2 with the anode 9 cleaned in the step (1) was placed on a spin coater, and a 10-nm-thick film made of a hole injection material "Clevios HIL1.3N" available from Heraeus was formed as a hole injection layer 8. Thereafter, the substrate 2 was heated on an electric griddle at 180°C for one hour.
(3) Next, the substrate 2 with the hole injection layer 8 and the previous layers was fixed to a substrate holder of a vacuum evaporation apparatus. DIC-TRZ, Ir(mppy)₃, α-NPD, 4DBTP3Q, the compound of the formula (2-2) as the first material, lithium fluoride, and Al were separately put into alumina crucibles, which were set as evaporation sources.

The pressure in the chamber of the vacuum evaporation apparatus was reduced to 1 × 10⁻⁵ Pa. A hole transport layer 7, an emitting layer 6, an electron injection layer 5, and a cathode 3 were successively formed by a vacuum evaporation method involving resistance heating. Specifically, first, the hole transport layer 7 with a thickness of 30 nm including 20-nm-thick α-NPD and 10-nm-thick 4DBTP3Q was formed. Subsequently, the emitting layer 6 was formed by co-evaporating DIC-TRZ as a host and Ir(mppy)₃ as a dopant to a thickness of 25 nm. The doping concentration was controlled such that Ir(mppy)₃ would be 3% by mass relative to the entire emitting layer 6. Next, the electron transport layer 10 with a thickness of 40 nm made of DIC-TRZ and the electron injection layer 5 with a thickness of 1 nm were formed on the substrate 2 with the emitting layer 6 and the previous layers.

In Example 21 (not according to the invention), the electron injection layer 5 was made of the compound of the formula (2-2), and in Comparative Example 16, the electron injection layer 5 was made of lithium fluoride, which is a common electron injection material.

In Example 22 (not according to the invention), the 30-nm-thick hole transport layer 7 was made of DIC-TRZ, and the other layers were made of the same materials as in Example 21.

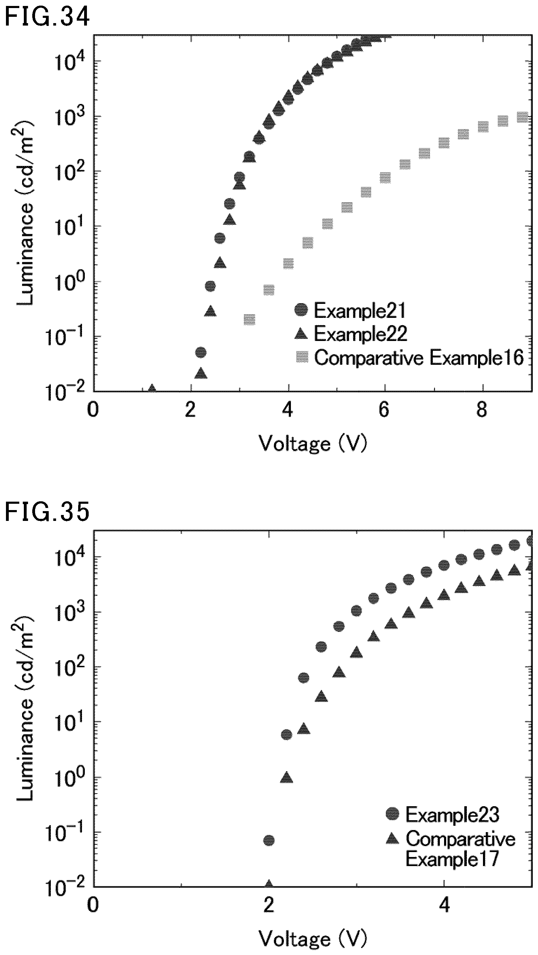

FIG. 34 illustrates the results of the luminance-voltage characteristics of the devices of Examples 21 and 22 and Comparative Example 16 measured by the same method as in the other examples and comparative examples.

### Example 23 and Comparative Example 17

The emitting layer of each of the devices of Examples 21 and 22 contains two materials, a host and a dopant. In order to demonstrate that even an organic EL device including an emitting layer consisting of a single material can achieve a simple structure illustrated in FIG. 4, devices of Example 23 and Comparative Example 17 were produced in the following way.

(1) A commercially available transparent glass substrate 2 having an average thickness of 0.7 mm with a 100-nm-thick electrode (anode 9) made of ITO with a pattern of 3 mm width was prepared as a substrate 2.
   The substrate 2 with the anode 9 was ultrasonically cleaned in acetone and isopropanol each for 10 minutes and then boiled in isopropanol for five minutes. Thereafter, the substrate 2 with the anode 9 was taken out from isopropanol, dried by blowing nitrogen, and cleaned with UV ozone for 20 minutes.
(2) Next, the substrate 2 was fixed to a substrate holder of a vacuum evaporation apparatus.

Fullerene for the hole injection layer 8, (9,10-bis(4-(9Hcarbazol-9-yl)-2,6-dimethylphenyl)-9,10-diboraanthracene (CzDBA) of the following formula (27), the compound of the formula (2-2) as the first material, lithium quinoline, and Al were separately put into alumina crucibles, which were set as evaporation sources. Molybdenum trioxide for the hole injection layer 8 was put into a tungsten boat, which was set.

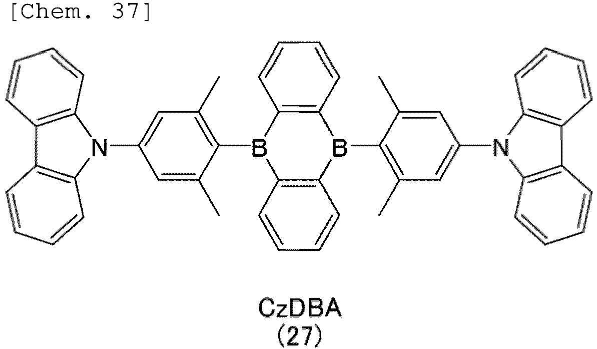

The pressure in the chamber of the vacuum evaporation apparatus was reduced to 1 × 10⁻⁵ Pa, and each layer was evaporated by a vacuum evaporation method involving resistance heating. The hole injection layer 8 was formed by evaporating molybdenum trioxide and fullerene each to a thickness of 5 nm. As a layer of a combination of a hole transport layer 7, an emitting layer 6, and an electron transport layer 10, only CzDBA of a thickness of 75 nm was formed by evaporation, followed by successive formation of a 1-nm-thick electron injection layer 5 and a cathode 3. Thus, the organic EL devices were produced. In Example 23, the electron injection layer 5 was made of the compound of the formula (2-2), and in Comparative Example 17, the electron injection layer 5 was made of lithium quinoline, which is a common electron injection material.

FIG. 35 illustrates the results of the luminance-voltage characteristics of the devices of Example 23 and Comparative Example 17 measured by the same method as in the other examples and comparative examples.

### Example 24 (not according to the invention) and Comparative Example 18

An organic EL device of Example 24 was produced as in Example 21 except that the first material (electron injection layer 5) was the compound of the formula (2-11).

An organic EL device of Comparative Example 18 was produced as in Example 21 except that the first material (electron injection layer 5) was not used.

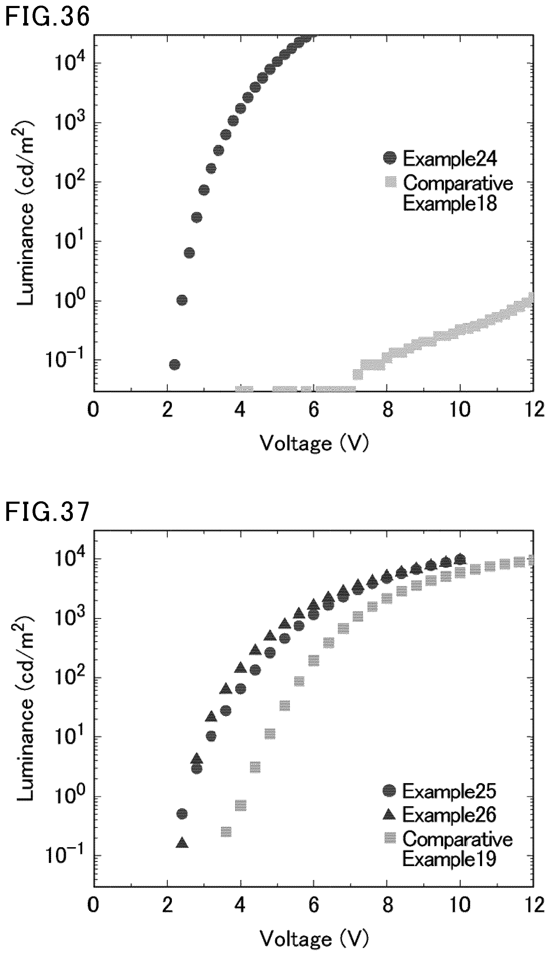

FIG. 36 illustrates the results of the luminance-voltage characteristics of the devices of Example 24 and Comparative Example 18 measured by the same method as in the other examples and comparative examples.

### Example 25

An inverted organic EL device having the laminate structure illustrated in FIG. 1 was produced in the following way.
(1) A commercially available transparent glass substrate 2 having an average thickness of 0.7 mm with a 150-nm-thick electrode (cathode 3) made of ITO with a pattern of 3 mm wide was prepared as a substrate 2. The substrate 2 with the cathode 3 was ultrasonically cleaned in acetone and isopropanol each for 10 minutes and then boiled in isopropanol for five minutes. Thereafter, the substrate 2 with the cathode 3 was taken out from isopropanol, dried by blowing nitrogen, and cleaned with UV ozone for 20 minutes.
(2) The substrate 2 with the cathode 3 cleaned in the step (1) was fixed to a substrate holder of a mirrortron sputtering apparatus having a zinc metal target. The pressure in the chamber of the sputtering apparatus was reduced to about 1 × 10⁻⁴ Pa, and the substrate 2 was subjected to sputtering with argon and oxygen introduced therein to form a zinc oxide layer having a thickness of about 3 nm on the cathode 3 of the substrate 2.
(3) Next, a 1.0% by weight solution of magnesium acetate in ethanol was prepared. The substrate with the zinc oxide layer was placed on a spin coater, the solution of magnesium acetate was dropped on the substrate, and the substrate was rotated at 1300 rpm for 60 seconds.
   Thereafter, the substrate was annealed in the atmosphere at 400°C for one hour to form a 3-nm-thick magnesium oxide film. Through the steps (2) and (3), an oxide layer 4 which was a laminate of a zinc oxide layer and a magnesium oxide layer was formed on the substrate.
(4) Next, an organic thin film containing the first material and the second material was formed as an electron injection layer 5 on the oxide layer 4 in the following way.
   First, the boron-containing compound of the formula (4) and the compound of the formula (2-30) in a weight ratio of 1:0.4 were dissolved in cyclopentanone at a concentration of 1.0% by weight to prepare a coating composition. Next, the substrate 2 with the cathode 3 and the oxide layer 4 produced in the step (2) was placed on a spin coater. Then, the substrate 2 was rotated at 3000 rpm for 30 seconds while the coating composition was dropped on the oxide layer 4. Thus, a coat was formed. Subsequently, the substrate 2 was annealed in a nitrogen atmosphere at 150°C for one hour using an electric griddle to form the electron injection layer 5. The electron injection layer 5 had an average thickness of 20 nm.
(5) Next, the substrate 2 with the electron injection layer 5 and the previous layers was fixed to a substrate holder of a vacuum evaporation apparatus. Bis(2-(2-benzothiazolyl)phenolato)zinc(II) (Zn(BTZ)₂) of the formula (11), tris[1-phenylisoquinoline]iridium(III) (Ir(piq)₃) of the formula (12), N,N'-di(1-naphthyl)-N,N'-diphenyl-1,1'-biphenyl-4,4'-diamine (α-NPD) of the formula (13), 1,4,5,8,9,12-hexaazatriphenylene-2,3,6,7,10,11-hexacarbonitrile (HAT-CN) of the formula (14), and Al were separately put into alumina crucibles, which were set as evaporation sources.

The pressure in the chamber of the vacuum evaporation apparatus was reduced to 1 × 10⁻⁵ Pa, and an electron transport layer 10, an emitting layer 6, a hole transport layer 7, a hole injection layer 8, and an anode 9 were successively formed by a vacuum evaporation method involving resistance heating.

First, the electron transport layer 10 with a thickness of 10 nm made of Zn(BTZ)₂ was formed. Subsequently, the emitting layer 6 was formed by co-evaporating Zn(BTZ)₂ as a host and Ir(piq)₃ as a dopant to a thickness of 20 nm. The doping concentration was controlled such that Ir(piq)₃ would be 6% by mass relative to the entire emitting layer 6. Next, the hole transport layer 7 was formed by forming a 50-nm-thick α-NPD film on the substrate 2 with the emitting layer 6 and the previous layers. The hole injection layer 8 was formed by forming a 10-nm-thick HAT-CN film. Next, the anode 9 with a thickness of 100 nm made of aluminum was formed by a vacuum evaporation method on the substrate 2 with the hole injection layer 8 and the previous layers.

The anode 9 was formed using a stainless steel evaporation mask to have a 3-mm-width-band-like evaporation area. The produced organic EL device had an emitting area of 9 mm².

(6) Next, the substrate 2 with the anode 9 and the previous layers was placed in a glass cap (sealing container) with a recessed space, and the container was filled with a sealing material made of an ultraviolet (UV) curable resin to seal the substrate. Thus, an organic EL device of Example 25 was obtained.

### Example 26

An organic EL device of Example 26 was obtained as in Example 25 except that, in the step (4), the compound of the formula (2-31) was used instead of the compound of the formula (2-30).

### Comparative Example 19

An organic EL device of Comparative Example 19 was obtained as in Example 25 except that, in the step (4), a coating composition was used which was prepared by dissolving only the boron-containing compound of the formula (4) in cyclopentanone at a concentration of 1.0% by weight without using the compound of the formula (2-30).

FIG. 37 illustrates the results of the luminance-voltage characteristics of the devices of Examples 25 and 26 and Comparative Example 19 measured by the same method as in the other examples and comparative examples.

As shown in FIG. 12, the devices of Examples 1 and 2 using the compound of the formula (2-2) or (2-4) emit light at a lower voltage than the device of Comparative Example 1 using only the boron-containing compound of the formula (4) as a material of the electron injection layer. As shown in FIG. 13, the devices of Examples 3 and 4 using the compound of the formula (2-2) or (2-4) emit light at a lower voltage than the device using the boron-containing compound of the formula (4) and MTBD. These results demonstrate that use of a hexahydropyrimidopyrimidine compound of the formula (1) in the present invention in combination with an electron transport material provides a device that can be driven with a lower voltage.

As shown in FIG. 14, use of a hexahydropyrimidopyrimidine compound of the formula (1) in the present invention in combination with an electron transport material can provide a perfect light emitting surface having no crack as seen in a device using MTBD.

The results shown in FIG. 15 demonstrate that a device in which a hexahydropyrimidopyrimidine compound of the formula (1) in the present invention is used in combination with an electron transport material and a layer is formed by evaporation can be driven with a low voltage. A material containing the hexahydropyrimidopyrimidine compound in combination with the electron transport material can be used without any restriction on the production process and can provide a device exhibiting excellent characteristics regardless of whether the film is formed by application or evaporation.

The results shown in FIG. 16 demonstrate that a device in which a hexahydropyrimidopyrimidine compound of the formula (1) in the present invention is used in combination with an electron transport material, but in which a metal oxide layer is not present between the electron injection layer made of this material and the cathode, has characteristics equivalent to those of a device including a metal oxide layer between the electron injection layer and the cathode. Thereby, the structure of the device can be simplified.

The results shown in FIG. 17 demonstrate that Example 7 and Comparative Example 4 show achievement of the effects of doping a hexahydropyrimidopyrimidine compound in the case of using compounds other than the compound of the formula (4). Also, Example 8 shows achievement of the effects not only in the case where the electron injection layer is a film of a mixture of the first material and the second material, but also in the case where the electron injection layer is a laminate film.

The results shown in FIG. 18 demonstrate that the device of Example 9 prepared by doping a hexahydropyrimidopyrimidine compound into the compound of the formula (17) exhibits good characteristics equivalent to those of the device of Example 5. The device of Example 10 including a film of a mixture of the first material and the second material as an electron injection layer, the metal oxide layer 4, and a different material (e.g., a second x material) therebetween exhibits good characteristics similarly to the device of Example 9. FIG. 19 demonstrates that such a structure can provide a device with a long life time. This may be presumably because the first material is prevented from deteriorating due to interaction with the metal oxide layer.

The results shown in FIG. 21 demonstrate that the conventional organic EL device of Example 11 in which the electron injection layer is made of only the compound of the formula (2-2) has good characteristics. Also, the device of Example 11 in which the electron injection layer is a single layer of the compound of the formula (2-2) has characteristics equivalent to those of the device of Example 8 which is an inverted organic EL device including the same hole transport material and emitting material as those in the device of Example 11. Thus, the electron injection layer in the present invention can function well even in a conventional organic EL device.

The results shown in FIG. 22 demonstrate that the devices of Examples 12 and 13 emit light at a lower voltage than the device of Comparative Example 5 including an electron injection layer consisting of a boron compound (4). Also, the effects of using a hexahydropyrimidopyrimidine compound in combination with an electron transport material are confirmed in a conventional organic EL device.

The results shown in FIG. 23 demonstrate that the device of Example 13 not only has device characteristics equivalent to those of the device of Comparative Example 6 including an electron injection layer made of lithium fluoride, which is commonly used in a conventional organic EL device, but also has a long life time. Alkali metals such as lithium fluoride are excellent in electron injection property, but they are known to be diffused in devices as, for example, Li ions, decreasing the stability of the organic EL devices. It is understood from the above that the organic thin film and the organic EL device material of the present invention are free of such a problem.

The results shown in FIG. 24 demonstrate that the device of Example 14 includes the second material between a film of a mixture of the first material and the second material and a metal electrode like the device of Example 10, and has a longer life time than the device of Example 12 not including the second material. It is considered that this is because even the conventional device is prevented from deterioration due to the interaction between the first material and the inorganic material.

The results shown in FIG. 26 demonstrate that even in the conventional organic EL device including an emitting layer made of a polymer emitting material, the compound of the formula (2-2) exhibits characteristics equivalent to or higher than those exhibited by LiF, which is commonly used as a material of an electron injection layer.

As for Example 16 in which the compound of the formula (22) is used, which exemplifies the case of using a pyridine-containing compound as the second material, FIG. 27 shows that the device of Example 16 emits light at a lower voltage than the device of Comparative Example 9 in which the compound of the formula (2-2) is not used and has luminance-voltage characteristics equivalent to those of the device of Comparative Example 8 using lithium fluoride. The results demonstrate that a device which can be driven with a low voltage can be provided by using a hexahydropyrimidopyrimidine compound of the formula (1) in the present invention even when the second material is a pyridine-containing compound. Thus, an organic EL device which can be driven with a low voltage can be achieved without using an alkali metal.

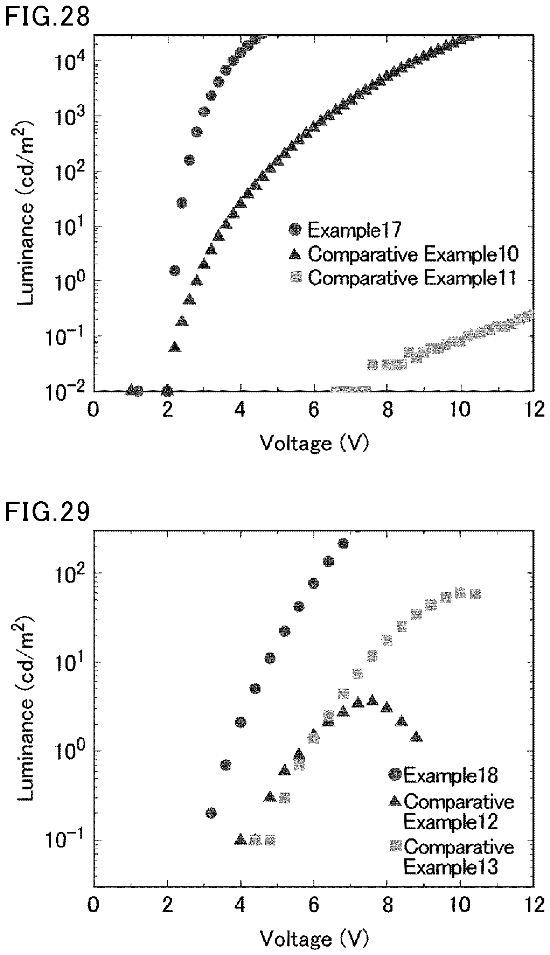

As for Example 17 in which the compound of the formula (23) is used, which exemplifies the case of using a triazine derivative as the second material, FIG. 28 shows that the device of Example 17 emits light at a lower voltage than the device of Comparative Example 10 in which lithium fluoride is used and the device of Comparative Example 11 in which the compound of the formula (2-2) is not used. The results demonstrate that a device which can be driven with a low voltage can be provided by using a hexahydropyrimidopyrimidine compound of the formula (1) in the present invention even when the second material is a triazine derivative. The results also demonstrate that a hexahydropyrimidopyrimidine compound has a better electron injection property than lithium fluoride.

As for Example 18 in which the compound of the formula (24) is used, which exemplifies the case of using a compound containing a carbonyl-containing heterocyclic ring as the second material, FIG. 29 shows that the device of Example 18 emits light at a lower voltage than the device of Comparative Example 12 in which lithium fluoride is used and the device of Comparative Example 13 in which the compound of the formula (2-2) is not used. The results demonstrate that a device which can be driven with a low voltage can be provided by using a hexahydropyrimidopyrimidine compound of the formula (1) in the present invention even when the second material is a compound containing a carbonyl-containing heterocyclic ring. The results also demonstrate that a hexahydropyrimidopyrimidine compound has a better electron injection property than lithium fluoride.

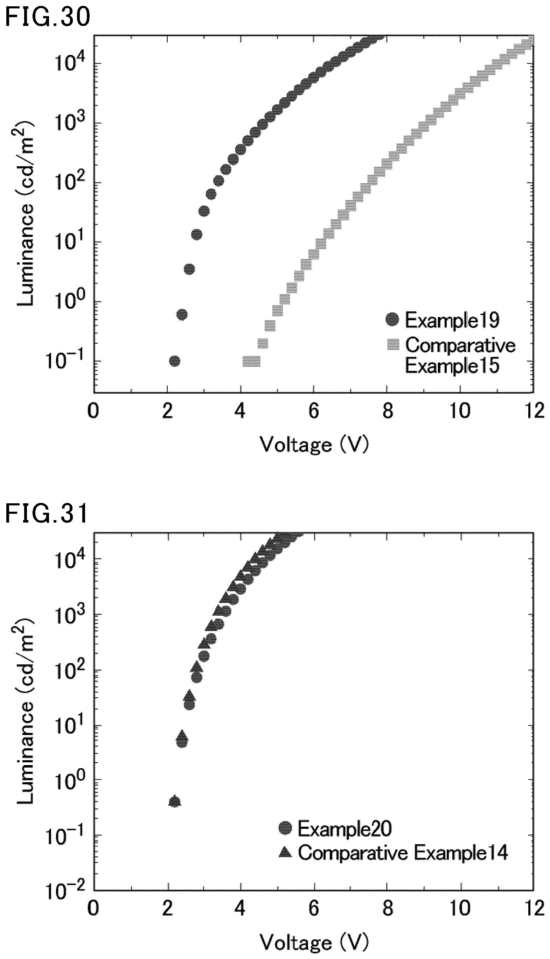

As for Example 19 in which the compound of the formula (25) is used, which exemplifies the case of using a phenanthroline derivative as the second material, FIG. 30 shows that the device of Example 19 emits light at a lower voltage than the device of Comparative Example 15 in which the compound of the formula (2-2) is not used. The results demonstrate that a device which can be driven with a low voltage can be provided by using a hexahydropyrimidopyrimidine compound of the formula (1) in the present invention even when the second material is a phenanthroline derivative.

As for Example 20 in which the second material is the compound of the formula (26), FIG. 31 and FIG. 32 show that the device of Example 20 not only has device properties equivalent to those of the device of Comparative Example 14 including an electron injection layer made of lithium fluoride, which is commonly used in a conventional organic EL device, but also has a long life time. The results of FIG. 33 demonstrate that the life times of the devices are significantly different from each other at a high temperature (85°C). The results demonstrate that alkali metals such as lithium fluoride are excellent in electron injection property, but they are diffused in the device as, for example, Li ions, decreasing the stability of the organic EL device. It is understood from the above that the organic thin film and the organic EL device material of the present invention are free of such a problem. The results also demonstrate that Li ions may be highly diffused at a high temperature, and the organic thin film and the organic EL device material of the present invention are highly resistant to high temperatures.

FIG. 34 showing the measured luminance-voltage characteristics of the devices of Examples 21 and 22 and Comparative Example 16 demonstrates that the device of Comparative Example 16 is driven with a high voltage because electrons are hardly injected from lithium fluoride to DIC-TRZ, whereas the device of Example 21 can be driven with a low voltage because electrons can be injected efficiently. FIG. 34 also demonstrates that the device of Example 22 including a hole transport layer made of DIC-TRZ which is the host of the emitting layer is driven with a voltage equivalent to that for the device of Example 21. Use of a material excellent in electron injection property enables simplification of the structure of the organic EL device and reduction of the number of materials to be used.

FIG. 35 showing the measured luminance-voltage characteristics of the organic EL devices of Example 23 and Comparative Example 17 each including an emitting layer made of a single material demonstrates that the device of Comparative Example 17 is driven with a high voltage because electrons are hardly injected from lithium quinoline to CzDBA, whereas the device of Example 23 can be driven with a low voltage because electrons can be injected efficiently. Use of a material excellent in electron injection property enables simplification of the structure of the organic EL device and reduction of the number of materials to be used.

In FIG. 36 showing the measured luminance-voltage characteristics of the organic EL device of Example 24 in which the first material (electron injection layer 5) is the compound of the formula (2-11) and the organic EL device of Comparative Example 18 in which the first material (electron injection layer 5) is not used, the device of Example 24 in which the material (first material) of the electron injection layer is the compound of the formula (2-11) emits light at a lower voltage than the device of Comparative Example 18 in which the first material is not used. The results demonstrate that use of the compound of the formula (2-11) provides a device that can be driven with a lower voltage.

In FIG. 37 showing the measured luminance-voltage characteristics of the devices of Examples 25 and 26 and Comparative Example 19, the devices of Examples 25 and 26 in which the material (first material) of the electron injection layer is the compound of the formula (2-30) or the compound of the formula (2-31) emit light at a lower voltage than the device of Comparative Example 19 in which only the boron-containing compound of the formula (4) is used. The results demonstrate that use of the compound of the formula (2-30) or the compound of the formula (2-31) provides a device that can be driven with a lower voltage.

### REFERENCE SIGNS LIST

1: organic EL device, 2: substrate, 3: cathode (cathode 2), 3': cathode 1, 4: oxide layer, 5: electron injection layer, 6: emitting layer, 7: hole transport layer, 8: hole injection layer, 9: anode, 10: electron transport layer.

## Claims

1. An organic thin film, which is
a single film containing a first material which is a hexahydropyrimidopyrimidine compound having a structure of the following formula (1) and a second material which transports electrons or
a laminate film including a film containing the first material and a film containing the second material, 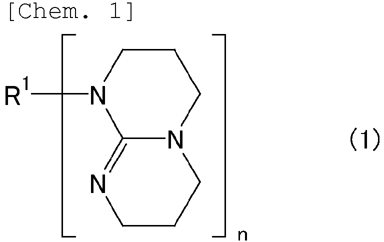
wherein R¹ is an optionally substituted aromatic hydrocarbon group, an optionally substituted aromatic heterocyclic group, an optionally substituted arylalkylene group, an optionally substituted divalent to tetravalent acyclic or cyclic hydrocarbon group,
wherein the aromatic heterocyclic group is a compound consisting of one aromatic heterocyclic ring selected from thiophene, furan, pyrrole, oxazole, oxadiazole, thiazole, thiadiazole, imidazole, pyrimidine, pyrazine, or triazine; a compound in which any two or more of the compounds each consisting of one aromatic heterocyclic ring are directly bound to each other via a carbon-carbon bond, and bipyridine; and a group prepared by removing 1 to 4 hydrogen atoms from any of the aromatic heterocyclic rings of a condensed cyclic heteroaromatic hydrocarbon compound, selected from quinoline, quinoxaline, benzothiophene, benzothiazole, benzimidazole, benzoxazole, indole, carbazole, dibenzofuran, dibenzothiophene, acridine, or phenanthroline,
a group of a combination of two or more of an optionally substituted aromatic hydrocarbon group, an optionally substituted aromatic heterocyclic group, an optionally substituted arylalkylene group, an optionally substituted divalent to tetravalent acyclic or cyclic hydrocarbon groups, or
a group of a combination of one or more of an optionally substituted aromatic hydrocarbon group, an optionally substituted aromatic heterocyclic group, an optionally substituted arylalkylene group, an optionally substituted divalent to tetravalent acyclic or cyclic hydrocarbon groups, and a nitrogen atom; and
n is an integer of 1 to 4.

2. The organic thin film according to claim 1,
wherein the first material is a hexahydropyrimidopyrimidine compound of the formula (1) in which n is 2 or 3.

3. A laminate film comprising:
an oxide layer; and
a layer of the organic thin film according to claim 1 or 2 formed on the oxide layer.

4. An organic electroluminescence device comprising:
a cathode;
an anode;
an emitting layer between the cathode and the anode; and
the organic thin film according to claim 1 or 2 or the laminate film according to claim 3 between the cathode and the emitting layer.

5. The organic electroluminescence device according to claim 4, further comprising:
an inorganic oxide layer between the cathode and the organic thin film.

6. The organic electroluminescence device according to claim 4 or 5, comprising:
a laminate film including the film containing the first material and the second material and the film containing the second material between the cathode and the emitting layer.

7. The organic electroluminescence device according to claim 6,
wherein the layer containing the second material is present between the emitting layer and the film containing the first material and the second material.

8. The organic electroluminescence device according to claim 6,
wherein the layer containing the second material is present between the cathode and the film containing the first material and the second material.

9. The organic electroluminescence device according to any one of claims 4 to 8,
wherein the layer containing the second material is present between the anode and the emitting layer.

10. The organic electroluminescence device according to any one of claims 4 to 9,
wherein the emitting layer contains the second material.

11. An organic thin film solar cell, comprising:
the organic thin film according to claim 1 or 2; or
the laminate film according to claim 3.

12. A photoelectric transducer, comprising:
the organic thin film according to claim 1 or 2; or
the laminate film according to claim 3.

13. A thin film transistor, comprising:
the organic thin film according to claim 1 or 2; or
the laminate film according to claim 3.

14. A method for producing an organic thin film, comprising:
forming a single film containing a first material which is a hexahydropyrimidopyrimidine compound having a structure of the following formula (1) and a second material which transports electrons on a surface on which the organic thin film is to be formed; or
forming successively a film containing the first material and a film containing the second material on the surface on which the organic thin film is to be formed, 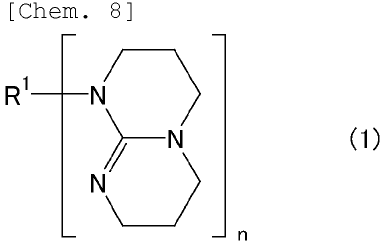
wherein R¹ is an optionally substituted aromatic hydrocarbon group, an optionally substituted aromatic heterocyclic group, an optionally substituted arylalkylene group, an optionally substituted divalent to tetravalent acyclic or cyclic hydrocarbon group,
wherein the aromatic heterocyclic group is a compound consisting of one aromatic heterocyclic ring selected from thiophene, furan, pyrrole, oxazole, oxadiazole, thiazole, thiadiazole, imidazole, pyrimidine, pyrazine, or triazine; a compound in which any two or more of the compounds each consisting of one aromatic heterocyclic ring are directly bound to each other via a carbon-carbon bond, and bipyridine; and a group prepared by removing 1 to 4 hydrogen atoms from any of the aromatic heterocyclic rings of a condensed cyclic heteroaromatic hydrocarbon compound, selected from quinoline, quinoxaline, benzothiophene, benzothiazole, benzimidazole, benzoxazole, indole, carbazole, dibenzofuran, dibenzothiophene, acridine, or phenanthroline,
a group of a combination of two or more of an optionally substituted aromatic hydrocarbon group, an optionally substituted aromatic heterocyclic group, an optionally substituted arylalkylene group, an optionally substituted divalent to tetravalent acyclic or cyclic hydrocarbon groups, or
a group of a combination of one or more of an optionally substituted aromatic hydrocarbon group, an optionally substituted aromatic heterocyclic group, an optionally substituted arylalkylene group, an optionally substituted divalent to tetravalent acyclic or cyclic hydrocarbon groups, and a nitrogen atom; and
n is an integer of 1 to 4.

## Patentansprüche

1. Organische Dünnschicht, die ist:
eine einzelne Schicht, die ein erstes Material, das eine Hexahydropyrimidopyrimidinverbindung ist, die eine Struktur der folgenden Formel (1) aufweist, und ein zweites Material enthält, das Elektronen transportiert, oder
eine Laminatschicht, die eine Schicht, die das erste Material enthält, und eine Schicht, die das zweite Material enthält, aufweist, 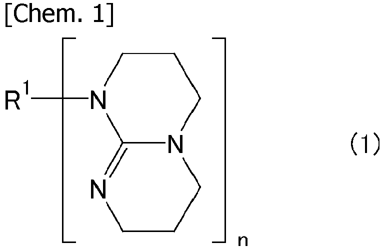
wobei R¹ eine optional substituierte aromatische Kohlenwasserstoffgruppe, eine optional substituierte aromatische heterocyclische Gruppe, eine optional substituierte Arylalkylengruppe, eine optional substituierte zweiwertige bis vierwertige acyclische oder cyclische Kohlenwasserstoffgruppe ist,
wobei die aromatische heterocyclische Gruppe eine Verbindung ist, die aus einem aromatischen heterocyclischen Ring besteht, ausgewählt aus Thiophen, Furan, Pyrrol, Oxazol, Oxadiazol, Thiazol, Thiadiazol, Imidazol, Pyrimidin, Pyrazin oder Triazin; eine Verbindung ist, wobei beliebige zwei oder mehr der Verbindungen, die jeweils aus einem aromatischen heterocyclischen Ring bestehen, durch eine Kohlenstoff-Kohlenstoff-Bindung direkt aneinandergebunden sind, und Bipyridin; und eine Gruppe ist, die durch Entfernen von 1 bis 4 Wasserstoffatomen aus einem beliebigen der aromatischen heterocyclischen Ringe einer kondensierten, cyclischen, heteroaromatischen Kohlenwasserstoffverbindung hergestellt ist, ausgewählt aus Chinolin, Chinoxalin, Benzothiophen, Benzothiazol, Benzimidazol, Benzoxazol, Indol, Carbazol, Dibenzofuran, Dibenzothiophen, Acridin oder Phenanthrolin,
eine Gruppe aus einer Kombination von zwei oder mehr einer optional substituierten aromatischen Kohlenwasserstoffgruppe, einer optional substituierten aromatischen heterocyclischen Gruppe, einer optional substituierten Arylalkylengruppe, einer optional substituierten, zweiwertigen bis vierwertigen acyclischen oder cyclischen Kohlenwasserstoffgruppe, oder eine Gruppe aus einer Kombination aus einer oder mehreren einer optional substituierten aromatischen Kohlenwasserstoffgruppe, einer optional substituierten aromatischen heterocyclischen Gruppe, einer optional substituierten Arylalkylengruppe, einer optional substituierten zweiwertigen bis vierwertigen acyclischen oder cyclischen Kohlenwasserstoffgruppe und einem Stickstoffatom; und
n eine ganze Zahl von 1 bis 4 ist.

2. Organische Dünnschicht nach Anspruch 1,
wobei das erste Material eine Hexahydropyrimidopyrimidinverbindung der Formel (1) ist, wobei n 2 oder 3 ist.

3. Laminatschicht, die umfasst:
eine Oxidlage; und
eine Lage aus der organischen Dünnschicht nach Anspruch 1 oder 2, die auf der Oxidlage gebildet ist.

4. Organische Elektrolumineszenzvorrichtung, die umfasst:
eine Kathode;
eine Anode;
eine emittierende Lage zwischen der Kathode und der Anode; und
die organische Dünnschicht nach Anspruch 1 oder 2 oder die Laminatschicht nach Anspruch 3 zwischen der Kathode und der emittierenden Lage.

5. Organische Elektrolumineszenzvorrichtung nach Anspruch 4, die ferner umfasst:
eine Lage aus anorganischem Oxid zwischen der Kathode und der organischen Dünnschicht.

6. Organische Elektrolumineszenzvorrichtung nach Anspruch 4 oder 5, die umfasst:
eine Laminatschicht, die die Schicht, die das erste Material und das zweite Material enthält, und die Schicht, die das zweite Material enthält, zwischen der Kathode und der emittierenden Lage aufweist.

7. Organische Elektrolumineszenzvorrichtung nach Anspruch 6,
wobei die Lage, die das zweite Material enthält, zwischen der emittierenden Lage und der Schicht, die das erste Material und das zweite Material enthält, vorhanden ist.

8. Organische Elektrolumineszenzvorrichtung nach Anspruch 6,
wobei die Lage, die das zweite Material enthält, zwischen der Kathode und der Schicht, die das erste Material und das zweite Material enthält, vorhanden ist.

9. Organische Elektrolumineszenzvorrichtung nach einem der Ansprüche 4 bis 8,
wobei die Lage, die das zweite Material enthält, zwischen der Anode und der emittierenden Lage vorhanden ist.

10. Organische Elektrolumineszenzvorrichtung nach einem der Ansprüche 4 bis 9,
wobei die emittierende Lage das zweite Material enthält.

11. Solarzelle aus organischer Dünnschicht, die umfasst:
die organische Dünnschicht nach Anspruch 1 oder 2; oder
die Laminatschicht nach Anspruch 3.

12. Photoelektrischer Wandler, der umfasst:
die organische Dünnschicht nach Anspruch 1 oder 2; oder
die Laminatschicht nach Anspruch 3.

13. Dünnschichttransistor, der umfasst:
die organische Dünnschicht nach Anspruch 1 oder 2; oder
die Laminatschicht nach Anspruch 3.

14. Verfahren zum Herstellen einer organischen Dünnschicht, das umfasst:
Bilden einer einzelnen Schicht, die ein erstes Material, das eine Hexahydropyrimidopyrimidinverbindung ist, die eine Struktur der folgenden Formel (1) aufweist, und ein zweites Material enthält, das Elektronen transportiert, auf einer Oberfläche, auf der die organische Dünnschicht zu bilden ist; oder
Bilden einer Schicht, die das erste Material enthält, und einer Schicht, die das zweite Material enthält, nacheinander auf der Oberfläche, auf der die organische Dünnschicht zu bilden ist, 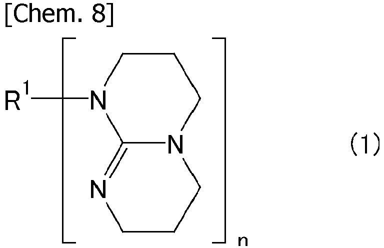
wobei R¹ eine optional substituierte aromatische Kohlenwasserstoffgruppe, eine optional substituierte aromatische heterocyclische Gruppe, eine optional substituierte Arylalkylengruppe, eine optional substituierte zweiwertige bis vierwertige acyclische oder cyclische Kohlenwasserstoffgruppe ist,
wobei die aromatische heterocyclische Gruppe eine Verbindung ist, die aus einem aromatischen heterocyclischen Ring besteht, ausgewählt aus Thiophen, Furan, Pyrrol, Oxazol, Oxadiazol, Thiazol, Thiadiazol, Imidazol, Pyrimidin, Pyrazin oder Triazin; eine Verbindung ist, wobei beliebige zwei oder mehr der Verbindungen, die jeweils aus einem aromatischen heterocyclischen Ring bestehen, durch eine Kohlenstoff-Kohlenstoff-Bindung direkt aneinandergebunden sind, und Bipyridin; und eine Gruppe ist, die durch Entfernen von 1 bis 4 Wasserstoffatomen aus einem beliebigen der aromatischen heterocyclischen Ringe einer kondensierten, cyclischen, heteroaromatischen Kohlenwasserstoffverbindung hergestellt ist, ausgewählt aus Chinolin, Chinoxalin, Benzothiophen, Benzothiazol, Benzimidazol, Benzoxazol, Indol, Carbazol, Dibenzofuran, Dibenzothiophen, Acridin oder Phenanthrolin,
eine Gruppe aus einer Kombination von zwei oder mehr einer optional substituierten aromatischen Kohlenwasserstoffgruppe, einer optional substituierten aromatischen heterocyclischen Gruppe, einer optional substituierten Arylalkylengruppe, einer optional substituierten, zweiwertigen bis vierwertigen acyclischen oder cyclischen Kohlenwasserstoffgruppe, oder eine Gruppe aus einer Kombination aus einer oder mehreren einer optional substituierten aromatischen Kohlenwasserstoffgruppe, einer optional substituierten aromatischen heterocyclischen Gruppe, einer optional substituierten Arylalkylengruppe, einer optional substituierten zweiwertigen bis vierwertigen acyclischen oder cyclischen Kohlenwasserstoffgruppe und einem Stickstoffatom; und
n eine ganze Zahl von 1 bis 4 ist.

## Revendications

1. Film mince organique, qui est
un film unique contenant un premier matériau qui est un composé hexahydropyrimidopyrimidine ayant une structure de formule (1) suivante et un deuxième matériau qui transporte des électrons, ou
un film stratifié comprenant un film contenant le premier matériau et un film contenant le deuxième matériau, 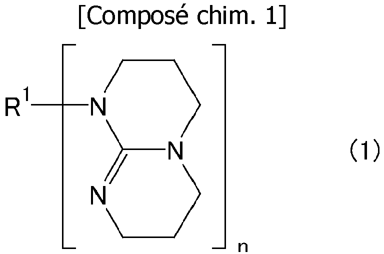
dans lequel R¹ est un groupe hydrocarboné aromatique éventuellement substitué, un groupe hétérocyclique aromatique éventuellement substitué, un groupe arylalkylène éventuellement substitué, un groupe hydrocarboné acyclique ou cyclique divalent à tétravalent éventuellement substitué,
dans lequel le groupe hétérocyclique aromatique est un composé se composant d'un cycle hétérocyclique aromatique choisi parmi le thiophène, le furane, le pyrrole, l'oxazole, l'oxadiazole, le thiazole, le thiadiazole, l'imidazole, la pyrimidine, la pyrazine ou la triazine ; un composé dans lequel deux composés quelconques ou plus se composant chacun d'un cycle hétérocyclique aromatique sont directement liés l'un à l'autre par une liaison carbone-carbone, et la bipyridine ; et un groupe préparé en éliminant 1 à 4 atomes d'hydrogène de l'un quelconque des cycles hétérocycliques aromatiques d'un composé hydrocarboné hétéroaromatique cyclique condensé, choisi parmi la quinoléine, la quinoxaline, le benzothiophène, le benzothiazole, le benzimidazole, le benzoxazole, l'indole, le carbazole, le dibenzofurane, le dibenzothiophène, l'acridine ou la phénanthroline,
un groupe d'une combinaison de deux groupes ou plus parmi un groupe hydrocarboné aromatique éventuellement substitué, un groupe hétérocyclique aromatique éventuellement substitué, un groupe arylalkylène éventuellement substitué, un groupe hydrocarboné acyclique ou cyclique divalent à tétravalent éventuellement substitué, ou
un groupe d'une combinaison d'un ou plusieurs groupes parmi un groupe hydrocarboné aromatique éventuellement substitué, un groupe hétérocyclique aromatique éventuellement substitué, un groupe arylalkylène éventuellement substitué, un groupe hydrocarboné acyclique ou cyclique divalent à tétravalent éventuellement substitué, et un atome d'azote ; et
n est un nombre entier de 1 à 4.

2. Film mince organique selon la revendication 1, dans lequel le premier matériau est un composé hexahydropyrimidopyrimidine de formule (1) dans laquelle n est égal à 2 ou 3.

3. Film stratifié comprenant :
une couche d'oxyde ; et
une couche du film mince organique selon la revendication 1 ou 2 formée sur la couche d'oxyde.

4. Dispositif d'électroluminescence organique comprenant :
une cathode ;
une anode ;
une couche émettrice entre la cathode et l'anode ; et
le film mince organique selon la revendication 1 ou 2 ou le film stratifié selon la revendication 3 entre la cathode et la couche émettrice.

5. Dispositif d'électroluminescence organique selon la revendication 4, comprenant en outre :
une couche d'oxyde inorganique entre la cathode et le film mince organique.

6. Dispositif d'électroluminescence organique selon la revendication 4 ou 5, comprenant :
un film stratifié comprenant le film contenant le premier matériau et le deuxième matériau, et le film contenant le deuxième matériau entre la cathode et la couche émettrice.

7. Dispositif d'électroluminescence organique selon la revendication 6,
dans lequel la couche contenant le deuxième matériau est présente entre la couche émettrice et le film contenant le premier matériau et le deuxième matériau.

8. Dispositif d'électroluminescence organique selon la revendication 6,
dans lequel la couche contenant le deuxième matériau est présente entre la cathode et le film contenant le premier matériau et le deuxième matériau.

9. Dispositif d'électroluminescence organique selon l'une quelconque des revendications 4 à 8,
dans lequel la couche contenant le deuxième matériau est présente entre l'anode et la couche émettrice.

10. Dispositif d'électroluminescence organique selon l'une quelconque des revendications 4 à 9,
dans lequel la couche émettrice contient le deuxième matériau.

11. Cellule solaire à film mince organique, comprenant :
le film mince organique selon la revendication 1 ou 2 ; ou
le film stratifié selon la revendication 3.

12. Transducteur photoélectrique, comprenant :
le film mince organique selon la revendication 1 ou 2 ; ou
le film stratifié selon la revendication 3.

13. Transistor à film mince, comprenant :
le film mince organique selon la revendication 1 ou 2 ; ou
le film stratifié selon la revendication 3.

14. Procédé de production d'un film mince organique, comprenant :
la formation d'un film unique contenant un premier matériau qui est un composé hexahydropyrimidopyrimidine ayant une structure de formule (1) suivante et un deuxième matériau qui transporte des électrons sur une surface sur laquelle le film mince organique doit être formé ; ou
la formation de manière successive d'un film contenant le premier matériau et d'un film contenant le deuxième matériau sur la surface sur laquelle le film mince organique doit être formé, 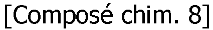 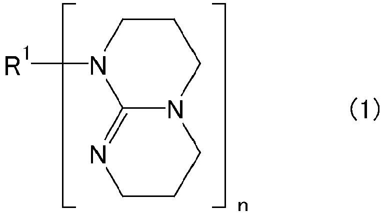
dans lequel R¹ est un groupe hydrocarboné aromatique éventuellement substitué, un groupe hétérocyclique aromatique éventuellement substitué, un groupe arylalkylène éventuellement substitué, un groupe hydrocarboné acyclique ou cyclique divalent à tétravalent éventuellement substitué,
dans lequel le groupe hétérocyclique aromatique est un composé se composant d'un cycle hétérocyclique aromatique choisi parmi le thiophène, le furane, le pyrrole, l'oxazole, l'oxadiazole, le thiazole, le thiadiazole, l'imidazole, la pyrimidine, la pyrazine ou la triazine ; un composé dans lequel deux composés quelconques ou plus se composant chacun d'un cycle hétérocyclique aromatique sont directement liés l'un à l'autre par une liaison carbone-carbone, et la bipyridine ; et un groupe préparé en éliminant 1 à 4 atomes d'hydrogène de l'un quelconque des cycles hétérocycliques aromatiques d'un composé hydrocarboné hétéroaromatique cyclique condensé, choisi parmi la quinoléine, la quinoxaline, le benzothiophène, le benzothiazole, le benzimidazole, le benzoxazole, l'indole, le carbazole, le dibenzofurane, le dibenzothiophène, l'acridine ou la phénanthroline,
un groupe d'une combinaison de deux groupes ou plus parmi un groupe hydrocarboné aromatique éventuellement substitué, un groupe hétérocyclique aromatique éventuellement substitué, un groupe arylalkylène éventuellement substitué, un groupe hydrocarboné acyclique ou cyclique divalent à tétravalent éventuellement substitué, ou
un groupe d'une combinaison d'un ou plusieurs groupes parmi un groupe hydrocarboné aromatique éventuellement substitué, un groupe hétérocyclique aromatique éventuellement substitué, un groupe arylalkylène éventuellement substitué, un groupe hydrocarboné acyclique ou cyclique divalent à tétravalent éventuellement substitué, et un atome d'azote ; et
n est un nombre entier de 1 à 4.
